(19) Europäisches Patentamt / European Patent Office / Office européen des brevets

(11) EP 4 257 978 A1

(12) **EUROPEAN PATENT APPLICATION**
published in accordance with Art. 153(4) EPC

(43) Date of publication:
**11.10.2023 Bulletin 2023/41**

(21) Application number: **21911029.3**

(22) Date of filing: **24.12.2021**

(51) International Patent Classification (IPC):
***G01N 33/537*** (2006.01) ***C12N 15/12*** (2006.01)
***C12N 15/53*** (2006.01) ***C12Q 1/6869*** (2018.01)
***G01N 33/15*** (2006.01) ***G01N 33/50*** (2006.01)
***G01N 33/68*** (2006.01)

(52) Cooperative Patent Classification (CPC):
**C07K 14/435; C12N 9/0004; C12Q 1/6869; G01N 33/15; G01N 33/50; G01N 33/537; G01N 33/68**

(86) International application number:
**PCT/JP2021/048097**

(87) International publication number:
**WO 2022/138892 (30.06.2022 Gazette 2022/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.12.2020 JP 2020216004**

(71) Applicant: **CHUGAI SEIYAKU KABUSHIKI KAISHA**
**Tokyo 115-8543 (JP)**

(72) Inventors:
- **KOJIMA, Tetsuo**
**Kamakura-shi, Kanagawa 247-8530 (JP)**
- **NISHIMURA, Kaori**
**Kamakura-shi, Kanagawa 247-8530 (JP)**
- **KURIKI, Yugo**
**Kamakura-shi, Kanagawa 247-8530 (JP)**

(74) Representative: **Vossius & Partner Patentanwälte Rechtsanwälte mbB**
**Siebertstraße 3**
**81675 München (DE)**

Remarks:
The complete document including Reference Table(s) and the Sequence Listing(s) can be downloaded from the EPO website

# (54) METHOD FOR SCREENING FOR CANDIDATE MOLECULE CAPABLE OF FORMING COMPLEX IN CONJUNCTION WITH PLURALITY OF TARGET MOLECULES

(57) A method for screening for a molecule capable of forming a complex with a plurality of target molecules has been found by applying an affinity-based method for recovering a candidate molecule to techniques represented by Split GFP techniques. The method includes allowing a first target molecule linked to a first moiety of a protein, a second target molecule linked to a second moiety of the protein, and a library containing a plurality of test molecules to coexist, and recovering a test molecule capable of forming a complex with the first target molecule linked to the first moiety and the second target molecule linked to the second moiety by an affinity technique.

EP 4 257 978 A1

## Description

### Technical Field

The present disclosure relates to a method for screening for a candidate molecule capable of forming a complex with a plurality of target molecules.

### Background Art

As the molecule that forms a complex with a plurality of target molecules, a bispecific molecule such as a bispecific antibody or proteolysis targeting chimera (PROTAC) and a molecule that binds to a target molecule through binding to a FK506 binding protein (FKBP) such as rapamycin or FK506, are known. A known method for producing the former molecule includes separately obtaining molecules that can bind to different targets, then linking the molecules (Non Patent Literature 1). A method for obtaining the latter molecule includes mixing a test compound with FKBP to form a complex of two components, then mixing the complex with a target protein and detecting a formation of a complex of three components by mass spectrometry (Patent Literature 1).

Split GFP techniques is techniques that utilize the phenomenon of GFP protein that has been divided into fragments so as not to emit luminescence by a fragment alone, but emits luminescence when the fragments are in proximity or association. When different proteins are bound to each GFP fragment, the interaction between the proteins can be detected using the luminescence of GFP as an indicator. Specifically, it has been reported that, when GFP is divided into three of GFP 1-9, GFP 10, and GFP 11 in beta-strand units, and GFP 10 and GFP 11 are bound to different proteins, respectively, the interaction of these proteins can be detected when GFP1-9 is added based on their luminescence (Non Patent Literature 2). It has also been reported that the activation state of GTPase was detected with cells expressing split GFP, using the luminescence caused by the interaction between GTPase and GTPase-binding domain in the cells as an indicator (Patent Literature 2 and Non Patent Literature 3).

### Citation List

#### Patent Literature

Patent Literature 1: US 9428845 B
Patent Literature 2: US 2018-0164324 A

#### Non Patent Literature

Non Patent Literature 1: Ulrich, H. W. et al. CANCER GENOMICS & PROTEOMICS 10: 1-18 (2013)
Non Patent Literature 2: Cabantous, S. et al. Sci Rep. 2013; 3: 2854. doi: 10.1038/srep02854
Non Patent Literature 3: Koraichi, F. et al. Journal of Cell Science 2018; 131: jcs210419 doi: 10.1242/jcs.210419

### Summary of Invention

#### Technical Problem

When it is sought to obtain a molecule that forms a complex with a plurality of target molecules, possible methods include a method (i) of screening binders for each target molecule and then linking the obtained binders, and a method (ii) of obtaining a binder for one target molecule, and then contacting a complex of the target molecule and the binder with another target molecule.

Increasing oral absorption and targeting an intracellular target requires reducing the molecular weight of candidate molecule to some extent. Thus, when aiming to obtain a candidate molecule for such purposes, the method (i) of obtaining binders for each target molecule and then linking the obtained binders will impose a significant limitation on the molecular weights of the individual binders. Contrarily, when the molecular weight is too small, limitations are imposed on the structure of the candidate molecule and the binding site of the target molecule that can be used for binding, thus the target molecules that can be applied are limited. Meanwhile, the method (ii) is a method for screening a library for a molecule having a desired property, rather than linking two molecules, and thus the first step is to select a single target

molecule by mono-specificity. In this step, molecules having a predominant binding mode to the target (first target) molecule are enriched, and thus, in the next step, molecules having the desired properties are selected from a population of molecules that are predominated in the binding mode to the first target. The predominant binding mode to the first target molecule does not necessarily match the binding mode to form a complex with the plurality of target molecules, thus the diversity of the compounds to be screened is limited. Thus, it is desired to have a screening method capable of directly selecting a molecule having a desired property from a library allowing a variety of binding modes to each target molecule.

In addition, as more diversity of properties of the candidate molecule having not only the binding activity to a single target molecule is required, the need for screening a library containing a variety of test molecules increases. Furthermore, to form a complex with a plurality of target molecules, the candidate molecule is required to provide a complicated binding and reaction mode, which makes it difficult to predict the optimal molecular structure. Thus, a high throughput screening method that is applicable to libraries as diverse as possible and is easy to select and identify candidate molecules is required.

Split GFP techniques is utilized for detection of protein-protein interactions (PPIs) (Non Patent Literature 2). However, since the emission of GFP is used as an indicator, the throughput capacity when applied for screening is not high. That is, in the case of fluorescence detection, a target molecule and a single candidate molecule are required to provide in each closed space such as a well, which is laborious and costly, and limits the number of test molecules that can be handled. Even if a library containing a plurality of test molecules in each confined space is prepared and screened for efficiency purposes, multiple assays are required step by step to select and identify a particular candidate molecule until fluorescence of only a single molecule can be ascertained. Thus, the throughput capacity is not high either. From these viewpoints, new methods to detect and recover divided proteins in proximity or association, instead of the method that uses the luminescence of the GFP proteins as an indicator, are required.

The present invention has been made considering such circumstances, and one object of the present invention is to provide a method for screening for a molecule capable of forming a complex with a plurality of target molecules.

Solution to Problem

The present inventors have considered that performing a screening of test molecules contained in a library and a plurality of target molecules which are allowed to coexist in the same system is the most effective way to produce diverse reaction modes with the target molecules. To this end, the present inventors have applied an affinity-based method for recovering candidate molecules to the techniques represented by Split GFP techniques, and have found a method for screening for a molecule capable of forming a complex with a plurality of target molecules. It has been found in this method that a candidate molecule capable of forming a complex with a plurality of target molecules can be recovered while suppressing the recovery rate of a test molecule that forms a complex with only one target molecule.

The present invention is based on such findings, and, in one specific, non-limiting aspect, encompasses the following.

[1] A method for screening for a candidate molecule capable of forming a complex with a first target molecule and a second target molecule, the method comprising steps (1) and (2) below:

(1) mixing the first target molecule linked to a first moiety, the second target molecule linked to a second moiety, and a library containing a plurality of test molecules, wherein the first moiety and the second moiety constitute a part or all of a protein; and
(2) recovering, after step (1), a complex containing the first target molecule, the second target molecule, and the candidate molecule by an affinity-based method using a third molecule capable of detecting that the first moiety and the second moiety are in proximity or association. [1.1] The method according to [1], wherein the first target molecule and the second target molecule in step (2) is the first target molecule linked to a first moiety and the second target molecule linked to the second moiety, respectively.

[2] The method according to [1] or [1.1], further comprising, after step (2), a step of identifying the candidate molecule contained in the recovered complex (step (3)).
[3] The method according to [1] or [2], wherein the third molecule is a molecule that reacts with the first moiety and/or the second moiety when the first moiety and the second moiety are in proximity or association.
[4] The method according to any one of [1] to [3], wherein the third molecule is a molecule that reacts with the first moiety and/or the second moiety when a complex containing the first target molecule, the second target molecule, and the candidate molecule is formed.
[5] The method according to any one of [1] to [4], wherein the third molecule is a molecule that does not substantially react with the first moiety and/or the second moiety when the first moiety and the second moiety are neither in

proximity nor in association.

[6] The method according to any one of [1] to [5], wherein the third molecule is a molecule that reacts with the first moiety and/or the second moiety in less than one-tenth when the first moiety and the second moiety are neither in proximity nor in association, compared to when the first moiety and the second moiety are in proximity or association.

[7] The method according to any one of [1] to [6], wherein the third molecule is a molecule that reacts with the first moiety and/or the second moiety in less than one-twentieth when the first moiety and the second moiety are neither in proximity nor in association, compared to when the first moiety and the second moiety are in proximity or association.

[8] The method according to any one of [1] to [7], wherein the third molecule is a molecule that reacts with the first moiety and/or the second moiety in less than one-fortieth when the first moiety and the second moiety are neither in proximity nor in association, compared to when the first moiety and the second moiety are in proximity or association.

[9] The method according to any one of [1] to [8], wherein the third molecule is a molecule that does not substantially react with the first moiety and/or the second moiety when a complex containing the first target molecule, the second target molecule, and the candidate molecule is not formed.

[10] The method according to any one of [1] to [9], wherein the third molecule is a molecule that reacts with the first moiety and/or the second moiety in less than one-tenth when a complex containing the first target molecule, the second target molecule, and the candidate molecule is not formed, compared to when the complex is formed.

[11] The method according to any one of [1] to [10], wherein the third molecule is a molecule that reacts with the first moiety and/or the second moiety in less than one-twentieth when a complex containing the first target molecule, the second target molecule, and the candidate molecule is not formed, compared to when the complex is formed.

[12] The method according to any one of [1] to [11], wherein the third molecule is a molecule that reacts with the first moiety and/or the second moiety in less than one-fortieth when a complex containing the first target molecule, the second target molecule, and the candidate molecule is not formed, compared to when the complex is formed.

The method according to any one of [1] to [12], wherein the complex containing the first target molecule, the second target molecule, and the candidate molecule is a complex containing the first target molecule linked to the first moiety, the second target molecule linked to the second moiety, and the candidate molecule.

[13] The method according to any one of [1] to [12.1], wherein the third molecule is a molecule capable of detecting a complex containing the first moiety and the second moiety.

[14] The method according to any one of [1] to [13], wherein the third molecule is a molecule that can form a complex containing the first moiety and the second moiety when the first moiety and the second moiety are in proximity or association.

[15] The method according to any one of [1] to [14], wherein the third molecule is a molecule that binds to the first moiety and/or the second moiety when the first moiety and the second moiety are in proximity or association.

[16] The method according to any one of [2] to [15], further comprising, between steps (2) and (3), a step of eluting the candidate molecule from a complex containing the first target molecule, the second target molecule, and the candidate molecule (step (2A)).

The method according to [16], wherein step (2A) is a step of eluting a complex containing the first target molecule, the second target molecule, and the candidate molecule.

[17] The method according to [16] or [16.1], further comprising a step of amplifying, between steps (2A) and (3), a polynucleotide encoding the candidate molecule eluted in step (2A) (step (2B)).

[18] The method according to [16] or [17], wherein step (3) comprises determining a sequence of a polynucleotide encoding the candidate molecule eluted in step (2A) or a sequence of a polynucleotide amplified in step (2B).

[19] The method according to any one of [1] to [18], wherein step (1) comprises mixing the first target molecule, the second target molecule, a library containing the plurality of test molecules, and the third molecule.

The method according to [19], wherein the first target molecule and the second target mixed in step (1) is the first target molecule linked to the first moiety and the second target molecule linked to the second moiety, respectively.

[20] The method according to any one of [1] to [18], further comprising, after step (1), a step (step (1A)) of mixing the first target molecule, the second target molecule, a library containing the plurality of test molecules, and the third molecule. [20.1] The method according to [20], wherein the first target molecule and the second target mixed in step (1A) is the first target molecule linked to the first moiety and the second target molecule linked to the second moiety, respectively.

[21] The method according to any one of [16] to [20.1], comprising a step of repeating steps (1), (2), and (2B), or steps (1), (1A), (2), and (2B) a plurality of times.

[22] The method according to any one of [16] to [21], comprising a step of repeating steps (1), (2), (2A), and (2B), or steps (1), (1A), (2), (2A), and (2B) a plurality of times.

[23] The method according to any one of [1] to [22], wherein the method is performed in vitro.

[24] The method according to any one of [1] to [23], wherein the test molecule is a molecule encoded by a polynucleotide.
[25] The method according to any one of [1] to [23], wherein the test molecule is a peptide.
[26] The method according to any one of [1] to [23], wherein the test molecule is a peptide linked to a polynucleotide.
[27] The method according to any one of [1] to [26], wherein a molecular weight of the test molecule is 3,000 or less.
[28] The method according to any one of [1] to [27], wherein a molecular weight of the test molecule is 2,000 or less.
[29] The method according to any one of [1] to [28], wherein the test molecule is a molecule known to bind to either one of the first target molecule or the second target molecule.
[30] The method according to any one of [1] to [29], wherein the library is a display library or a DNA encoding library.
[31] The method according to [30], wherein the display library is an mRNA display library.
[32] The method according to any one of [1] to [31], wherein the library is a library having a diversity of $1 \times 10^4$ or more.
[33] The method according to any one of [1] to [32], wherein the library is a library having a diversity of $1 \times 10^5$ or more.
[34] The method according to any one of [1] to [33], wherein the library is a library having a diversity of $1 \times 10^6$ or more.
[35] The method according to any one of [1] to [34], wherein the library is a library having a diversity of $1 \times 10^8$ or more.
[36] The method according to any one of [1] to [35], wherein the library is a library having a diversity of $1 \times 10^{10}$ or more.
[37] The method according to any one of [1] to [36], wherein the library is a library having a diversity of $1 \times 10^{11}$ or more.
[38] The method according to any one of [1] to [37], wherein the library is a library having a diversity of $1 \times 10^{12}$ or more.
[39] The method according to any one of [1] to [38], wherein the candidate molecule is a peptide.
[40] The method according to any one of items [1] to [39], wherein the candidate molecule is a cyclic peptide.
[41] The method according to any one of [1] to [40], wherein the candidate molecule is a molecule capable of binding to the first target molecule and the second target molecule simultaneously.
[42] The method according to any one of [1] to [41], wherein the candidate molecule when bound to the first target molecule has 2 times or more binding affinity for the second target molecule compared to when the candidate molecule is alone.
[43] The method according to any one of [1] to [42], wherein the candidate molecule when bound to the first target molecule has 5 times or more binding affinity for the second target molecule compared to when the candidate molecule is alone.
[44] The method according to any one of [1] to [43], wherein the candidate molecule when bound to the first target molecule has 10 times or more binding affinity for the second target molecule compared to when the candidate molecule is alone.
[45] The method according to any one of [1] to [44], wherein the candidate molecule when bound to the first target molecule has 20 times or more binding affinity for the second target molecule compared to when the candidate molecule is alone.
[46] The method according to any one of [1] to [45], wherein the candidate molecule is a molecule having any one of the following properties (i) to (iii):

(i) a candidate molecule capable of binding to the first target molecule, wherein the binding affinity of the candidate molecule for the second target molecule is higher than before the binding, due to the binding;
(ii) a candidate molecule capable of binding to the first target molecule, wherein the candidate molecule is capable of binding to the second target molecule as a complex of the candidate molecule and the first target molecule, due to the binding; and
(iii) a candidate molecule capable of binding to the first target molecule, wherein the binding affinity of the first target molecule for the second target molecule is higher than before binding, due to the binding.

[47] The method according to any one of [1] to [46], wherein the complex containing the first target molecule, the second target molecule, and the candidate molecule consists of the first target molecule, the second target molecule, and the candidate molecule.
[48] The method according to any one of [1] to [47], wherein the complex which the candidate molecule is capable of forming with the first target molecule and the second target molecule is a complex of three components.
[49] The method according to any one of [1] to [48], wherein step (1) comprises the following steps of:

(i) mixing the first target molecule linked to the first moiety and a test molecule; and
(ii) further mixing the mixture obtained in step (i) and the second target molecule linked to the second moiety.

[50] The method according to any one of [1] to [48], wherein step (1) comprises the following steps of:

(i) mixing the second target molecule linked to the second moiety and a test molecule; and
(ii) further mixing the mixture obtained in step (i) and the first target molecule linked to the first moiety.

[51] The method according to any one of [1] to [50], wherein the first moiety and the first target molecule and the second moiety and the second target molecule are each linked by a linker.

[52] The method according to any one of [1] to [51], wherein the first moiety and the second moiety are in proximity or association when a complex containing the first target molecule, the second target molecule, and the candidate molecule is formed.

[53] The method according to [52], wherein the third molecule reacts with or binds to the first moiety and/or the second moiety when the first moiety and the second moiety are in proximity or association.

[54] The method according to any one of [1] to [53], wherein a complex containing the first target molecule, the second target molecule and the candidate molecule is not formed when the test molecule does not bind to either the first target molecule or the second target molecule.

[55] The method according to [54], wherein the first moiety and the second moiety are in proximity or association when a complex containing the first target molecule, the second target molecule, and the candidate molecule is formed.

[56] The method according to [55], wherein the third molecule does not substantially react with or bind to the first moiety and/or the second moiety when the first moiety and the second moiety are neither in proximity nor in association.

The method according to any one of [1] to [56], wherein the complex containing the first target molecule, the second target molecule, and the candidate molecule is a complex containing the first target molecule linked to the first moiety, the second target molecule linked to the second moiety, and the candidate molecule.

[57] The method according to any one of [1] to [56.1], wherein the third molecule is determined to be a molecule that reacts with or binds to the first moiety and/or the second moiety when the first moiety and the second moiety are in proximity nor in association, when a recovery rate in which a molecule linking FKBP to the first moiety, a molecule linking FRB to the second moiety, and rapamycin linked to the nucleic acid are mixed, and the rapamycin linked to the nucleic acid is recovered using an affinity-based method using a third molecule is greater than or equal to 5 times the recovery rate in which either a molecule linking FKBP to the first moiety or a molecule linking FRB to the second moiety and rapamycin linked to the nucleic acid are mixed, and the rapamycin linked to the nucleic acid is recovered using an affinity-based method using the third molecule.

[58] The method according to any one of [1] to [57], wherein the third molecule is determined to be a molecule that does not substantially react with or bind to the first moiety and/or the second moiety when the first moiety and the second moiety are in proximity nor in association, when a recovery rate in which a molecule linking FKBP to the first moiety, a molecule linking FRB to the second moiety, and rapamycin linked to the nucleic acid are mixed, and the rapamycin linked to the nucleic acid is recovered using an affinity-based method using a third molecule is less than 5 times the recovery rate in which either a molecule linking FKBP to the first moiety or a molecule linking FRB to the second moiety and rapamycin linked to the nucleic acid are mixed, and the rapamycin linked to the nucleic acid is recovered using an affinity-based method using the third molecule.

[59] The method according to any one of [13] to [58], wherein the complex containing the first moiety and the second moiety contains a part or all of the protein.

[60] The method according to any one of [1] to [59], wherein the protein is GFP, luciferase, ubiquitin, or a SNAP tag.

[61] The method according to any one of [1] to [60], wherein the first moiety and the second moiety are GFP 10 and GFP11, respectively, or GFP11 and GFP 10, respectively, and the third molecule is a protein containing GFP1-9.

[62] The method according to any one of [1] to [60], wherein the first moiety and the second moiety are an N-terminal fragment of a SNAP tag and a C-terminal fragment of a SNAP tag, respectively, or a C-terminal fragment of a SNAP tag and an N-terminal fragment of a SNAP tag, respectively, and the third molecule is a SNAP biotin.

[63] The method according to any one of [1] to [60], wherein the first moiety and the second moiety are an N-terminal fragment of ubiquitin and a C-terminal fragment of ubiquitin, respectively, or a C-terminal fragment of ubiquitin and an N-terminal fragment of ubiquitin, respectively, and the third molecule is an anti-ubiquitin antibody.

[64] The method according to any one of [1] to [60], wherein the first moiety and the second moiety are Nanoluc-9 and Nanoluc-10, respectively, or Nanoluc-10 and Nanoluc-9, respectively, and the third molecule is a protein containing Nanoluc1-8.

[65] The method according to any one of [1] to [61], wherein GFP10 contains an amino acid sequence selected from the group consisting of the following (a) to (c):

(a) an amino acid sequence represented by SEQ ID NO: 73;
(b) the amino acid sequence represented by SEQ ID NO: 73 in which one or more amino acids are deleted, inserted, substituted, and/or added; and
(c) an amino acid sequence having 80% or more, 85% or more, 90% or more, 95% or more, or 98% or more sequence identity to the amino acid sequence represented by SEQ ID NO: 73.

[66] The method according to any one of [1] to [61] and [65], wherein GFP11 contains an amino acid sequence selected from the group consisting of the following (a) to (c):

(a) an amino acid sequence represented by SEQ ID NO: 74;
(b) the amino acid sequence represented by SEQ ID NO: 74 in which one or more amino acids are deleted, inserted, substituted, and/or added; and
(c) an amino acid sequence having 80% or more, 85% or more, 90% or more, 95% or more, or 98% or more sequence identity to the amino acid sequence represented by SEQ ID NO: 74.

[67] The method according to any one of [1] to [61], [65], and [66], wherein GFP1-9 contains an amino acid sequence selected from the group consisting of the following (a) to (c):

(a) an amino acid sequence represented by SEQ ID NO: 72;
(b) the amino acid sequence represented by SEQ ID NO: 72 in which one or more amino acids are deleted, inserted, substituted, and/or added; and
(c) an amino acid sequence having 80% or more, 85% or more, 90% or more, 95% or more, or 98% or more sequence identity to the amino acid sequence represented by SEQ ID NO: 72.

[68] The method according to any one of [1] to [60] and [62], wherein the N-terminal fragment of the SNAP tag contains an amino acid sequence selected from the group consisting of the following (a) to (c):

(a) an amino acid sequence represented by SEQ ID NO: 83;
(b) the amino acid sequence represented by SEQ ID NO: 83 in which one or more amino acids are deleted, inserted, substituted, and/or added; and
(c) an amino acid sequence having 80% or more, 85% or more, 90% or more, 95% or more, or 98% or more sequence identity to the amino acid sequence represented by SEQ ID NO: 83.

[69] The method according to any one of [1] to [60], [62], and [68], wherein the C-terminal fragment of the SNAP tag contains an amino acid sequence selected from the group consisting of the following (a) to (c):

(a) an amino acid sequence represented by SEQ ID NO: 84;
(b) the amino acid sequence represented by SEQ ID NO: 84 in which one or more amino acids are deleted, inserted, substituted, and/or added; and
(c) an amino acid sequence having 80% or more, 85% or more, 90% or more, 95% or more, or 98% or more sequence identity to the amino acid sequence represented by SEQ ID NO: 84.

[70] The method according to any one of [1] to [60] and [63], wherein the N-terminal fragment of ubiquitin contains an amino acid sequence selected from the group consisting of the following (a) to (c):

(a) an amino acid sequence represented by SEQ ID NO: 76;
(b) the amino acid sequence represented by SEQ ID NO: 76 in which one or more amino acids are deleted, inserted, substituted, and/or added; and
(c) an amino acid sequence having 80% or more, 85% or more, 90% or more, 95% or more, or 98% or more sequence identity to the amino acid sequence represented by SEQ ID NO: 76.

[71] The method according to any one of [1] to [60], [63] and [70], wherein the C-terminal fragment of ubiquitin contains an amino acid sequence selected from the group consisting of the following (a) to (c):

(a) an amino acid sequence represented by SEQ ID NO: 77;
(b) the amino acid sequence represented by SEQ ID NO: 77 in which one or more amino acids are deleted, inserted, substituted, and/or added; and
(c) an amino acid sequence having 80% or more, 85% or more, 90% or more, 95% or more, or 98% or more sequence identity to the amino acid sequence represented by SEQ ID NO: 77.

[72] The method according to any one of [1] to [60] and [64], wherein the Nanoluc-9 contains an amino acid sequence selected from the group consisting of the following (a) to (c):

(a) an amino acid sequence represented by SEQ ID NO: 80;

(b) the amino acid sequence represented by SEQ ID NO: 80 in which one or more amino acids are deleted, inserted, substituted, and/or added; and
(c) an amino acid sequence having 80% or more, 85% or more, 90% or more, 95% or more, or 98% or more sequence identity to the amino acid sequence represented by SEQ ID NO: 80.

[73] The method according to any one of [1] to [60], [64] and [72], wherein the Nanoluc-10 contains an amino acid sequence selected from the group consisting of the following (a) to (c):

(a) an amino acid sequence represented by SEQ ID NO: 81;
(b) an amino acid sequence in which one or more amino acids are deleted, inserted, substituted, and/or added in the amino acid sequence represented by SEQ ID NO: 81; and
(c) an amino acid sequence having 80% or more sequence identity to the amino acid sequence represented by SEQ ID NO: 81.

[74] The method according to any one of [1] to [60], [64], [72] and [73], wherein the Nanoluc1-8 contains an amino acid sequence selected from the group consisting of the following (a) to (c):

(a) an amino acid sequence represented by SEQ ID NO: 79;
(b) the amino acid sequence represented by SEQ ID NO: 79 in which one or more amino acids are deleted, inserted, substituted, and/or added; and
(c) an amino acid sequence having 80% or more, 85% or more, 90% or more, 95% or more, or 98% or more sequence identity to the amino acid sequence represented by SEQ ID NO: 79.

[75] The method according to any one of [1] to [74], wherein the first target molecule is a protein belonging to an immunophilin family.
[76] The method according to any one of [1] to [74], wherein the first target molecule is selected from the group consisting of a FK506 binding protein (FKBP), cyclophilin, FRB, calcineurin, and Ras proteins such as Kras, Nras, and Hras.
[77] The method according to any one of [1] to [76], wherein the affinity-based method is a pull-down method.
[78] The method according to any one of [1] to [77], wherein the affinity-based method is a method based on an affinity between biotin and a biotin-binding protein or an affinity between an antigen and an antibody.
[79] The method according to any one of [16] to [78], wherein the eluting in step (2A) is performed with an enzyme or by heat.
[80] The method according to any one of [16] to [79], wherein the eluting in step (2A) is performed by cleaving a first linker located between the first moiety of the protein and the first target molecule and a second linker located between the second moiety of the protein and the second target molecule.
[81] The method according to [80], wherein the first and second linkers contain sequences that are cleaved by an enzyme.
[82] The method according to [81], wherein the enzyme is a TEV protease.
[83] The method according to any one of [1] to [82], wherein the protein providing the first moiety and the second moiety contains an amino acid sequence selected from the group consisting of the following (a) to (c);

(a) an amino acid sequence represented by SEQ ID NO: 71, 75, 78, or 82;
(b) the amino acid sequence represented by SEQ ID NO: 71, 75, 78, or 82 in which one or more amino acids are deleted, inserted, substituted, and/or added; and
(c) an amino acid sequence having 80% or more sequence identity to the amino acid sequence represented by SEQ ID NO: 71, 75, 78, or 82.

[84] The method according to any one of [1] to [83], wherein the protein providing the first moiety and the second moiety contains an amino acid sequence selected from the group consisting of the following (a) to (c):

(a) an amino acid sequence represented by SEQ ID NO: 71;
(b) the amino acid sequence represented by SEQ ID NO: 71 in which one or more amino acids are deleted, inserted, substituted, and/or added; and
(c) an amino acid sequence having 80% or more, 85% or more, 90% or more, 95% or more, or 98% or more sequence identity to the amino acid sequence represented by SEQ ID NO: 71.

[85] The method according to any one of [1] to [83], wherein the protein providing the first moiety and the second

moiety contains an amino acid sequence selected from the group consisting of the following (a) to (c):

(a) an amino acid sequence represented by SEQ ID NO: 75;
(b) the amino acid sequence represented by SEQ ID NO: 75 in which one or more amino acids are deleted, inserted, substituted, and/or added; and
(c) an amino acid sequence having 80% or more, 85% or more, 90% or more, 95% or more, or 98% or more sequence identity to the amino acid sequence represented by SEQ ID NO: 75.

[86] The method according to any one of [1] to [83], wherein the protein providing the first moiety and the second moiety contains an amino acid sequence selected from the group consisting of the following (a) to (c):

(a) an amino acid sequence represented by SEQ ID NO: 78;
(b) the amino acid sequence represented by SEQ ID NO: 78 in which one or more amino acids are deleted, inserted, substituted, and/or added; and
(c) an amino acid sequence having 80% or more, 85% or more, 90% or more, 95% or more, or 98% or more sequence identity to the amino acid sequence represented by SEQ ID NO: 78.

[87] The method according to any one of [1] to [83], wherein the protein providing the first moiety and the second moiety contains an amino acid sequence selected from the group consisting of the following (a) to (c):

(a) an amino acid sequence represented by SEQ ID NO: 82;
(b) the amino acid sequence represented by SEQ ID NO: 82 in which one or more amino acids are deleted, inserted, substituted, and/or added; and
(c) an amino acid sequence having 80% or more, 85% or more, 90% or more, 95% or more, or 98% or more sequence identity to the amino acid sequence represented by SEQ ID NO: 82.

[88] The method according to any one of [1] to [87], wherein the third molecule is derived from the protein that provides the first moiety and the second moiety.
[89] The method according to any one of [1] to [88], further comprising the following steps of:

(4) mixing the first target molecule fixed on a solid phase support and a library containing a plurality of test molecules, and recovering a test molecule capable of binding to the first target molecule; and/or
(5) mixing the second target molecule fixed on a solid phase support and a library containing a plurality of test molecules, and recovering a test molecule capable of binding to the second target molecule.

[90] The method according to [89], wherein in step (4), the first target molecule fixed on a solid phase support, a free second target molecule, and a library containing a plurality of test molecules is mixed.
[91] The method according to [89] or [90], wherein in step (5), the second target molecule fixed on a solid phase support, a free first target molecule, and a library containing a plurality of test molecules is mixed.
[92] The method according to any one of [89] to [91], wherein steps (4) and (5) are performed before step (1).
[93] The method according to any one of [89] to [92], wherein step (5) is performed after step (4), or step (4) is performed after step (5).

Advantageous Effect of Invention

The present disclosure provides a method for screening for a molecule capable of forming a complex with a plurality of target molecules. The use of the method of the present disclosure enables a candidate molecule that forms a complex with a plurality of target molecules can be selected from a system in which a plurality of target molecules and a test molecule coexist while excluding a test molecule that forms a complex with only one target molecule.

Brief Description of Drawings

[Fig. 1] Fig. 1 is a diagram of gel shift utilizing the interaction between biotinylated GFP1-9 and streptavidin evaluated by SDS-PAGE. The detection was performed by CBB staining. (Lane 1: Molecular weight marker; Lane 2: Mixture of biotinylated GFP1-9 and Streptavidin; Lane 3: Biotinylated GFP1-9; Lane 4: Biotinylated GFP1-9 (1/10 amount); Lane 5: Streptavidin)

[Fig. 2] Fig. 2 shows the results of SEC analysis of biotinylated GFP1-9 (upper figure) and the results of molecular weight markers (lower figure) flowed in the same column. (Marker molecular weight: $290.00 \times 10^3$, $142.00 \times 10^3$, $67.00 \times 10^3$, $32.00 \times 10^3$, $12.40 \times 10^3$ from the peak on the left)

[Fig. 3] Fig. 3 is a diagram of gel shift utilizing the interaction between biotinylated splitNanoluc and streptavidin evaluated by SDS-PAGE. The detection was performed by CBB staining. (Lane 1: Molecular weight marker; Lane 2: Mixture of biotinylated splitNanoluc and Streptavidin; Lane 3: Biotinylated splitNanoluc; Lane 4: Biotinylated splitNanoluc (1/10 amount); Lane 5: Streptavidin)

[Fig. 4] Fig. 4 shows the results of SEC analysis of biotinylated splitNanoluc (upper figure) and the results of molecular weight markers (lower figure) flowed in the same column. (Marker molecular weight: $290.00 \times 10^3$, $142.00 \times 10^3$, $67.00 \times 10^3$, $32.00 \times 10^3$, $12.40 \times 10^3$ from the peak on the left)

Description of Embodiments

The present disclosure relates to a method for screening for a candidate molecule capable of forming a complex with a first target molecule and a second target molecule. In an aspect, the screening method according to the present disclosure comprises the following steps (1) and (2):

(1) mixing the first target molecule linked to a first moiety, the second target molecule linked to a second moiety, and a library containing a plurality of test molecules, wherein the first moiety and the second moiety constitute a part or all of a protein; and
(2) recovering, after step (1), a complex containing the first target molecule, the second target molecule, and the candidate molecule by an affinity-based method using a third molecule capable of detecting that the first moiety and the second moiety are in proximity or association.

Step (1)

The screening method according to the present disclosure comprises a step of mixing the first target molecule linked to a first moiety, the second target molecule linked to a second moiety, and a library containing a plurality of test molecules (step (1)). In the present disclosure, the first target molecule linked to the first moiety, the second target molecule linked to the second moiety, and a library containing a plurality of test molecules may be mixed at once. Also in the present disclosure, the first target molecule linked to the first moiety, the second target molecule linked to the second moiety, and a library containing a plurality of test molecules may be mixed in multiple portions. Specifically, the first target molecule linked to the first moiety and the second target molecule linked to the second moiety may be mixed first, and then the mixture of these may be mixed with a library containing the test molecules. In another aspect, the first target molecule linked to the first moiety and a library containing the test molecules may be mixed first, and then the mixture of these may be mixed with the second target molecule linked to the second moiety. In yet another aspect, the second target molecule linked to the second moiety and a library containing the test molecules may be mixed first, and then the mixture of these may be mixed with the first target molecule linked to the first moiety.

That is, step (1) in the present disclosure, in one aspect, can comprise the following steps of:

(i) mixing the first target molecule linked to a first moiety and the second target molecule linked to a second moiety; and
(ii) mixing the mixture obtained in (i) with one or more test molecules or a library containing the one or more test molecules.

Alternatively, step (1) in the present disclosure, in one aspect, can comprise the following steps of:

(i) mixing the first target molecule linked to a first moiety and one or more test molecules or a library containing the one or more test molecules; and
(ii) mixing the mixture obtained in (i) with the second target molecule linked to a second moiety.

Alternatively, step (1) in the present disclosure, in one aspect, can comprise the following steps of:

(i) mixing the second target molecule linked to a second moiety and one or more test molecules or a library containing the one or more test molecules; and
(ii) mixing the mixture obtained in (i) with the first target molecule linked to a first moiety.

Alternatively, step (1) in the present disclosure, in one aspect, can comprise a step of: mixing a complex formed with

(A) the first target molecule linked to a first moiety,
(B) the second target molecule linked to a second moiety, and
(C) a test molecule

with a third molecule.

Alternatively, the screening method according to the present disclosure may comprise, after step (1), a step (step (1A)) of mixing the mixture obtained in step (1) with a third molecule capable of detecting that the first moiety and the second moiety are in proximity or association.

In a further aspect, the screening method according to the present disclosure may comprise a step of mixing the first target molecule linked to a first moiety, the second target molecule linked to a second moiety, a third molecule, and a library containing a plurality of test molecules as the step (1), instead of comprising step (1A) after step (1). In this case, the first target molecule linked to the first moiety, the second target molecule linked to the second moiety, the third molecule, and the library containing a plurality of test molecules may be mixed at once or in multiple portions. Also, when mixed in multiple portions, the four components may be mixed one by one; or a mixture containing two of these four components (e.g., the first target molecule linked to the first moiety and the second target molecule linked to the second moiety) may be prepared in advance and mixed with the other components; or a mixture containing three of these four components (e.g., the first target molecule linked to the first moiety, the second target molecule linked to the second moiety, and a library containing a plurality of test molecules) may be prepared in advance and mixed with the other component.

In another non-limiting aspect, the present disclosure may be a method of screening for a candidate molecule capable of forming a complex with the first target molecule and a second target molecule, comprising the following steps (1) and (2):

(1) mixing a complex formed with

(A) the first target molecule linked to a first moiety,
(B) the second target molecule linked to a second moiety, and
(C) a test molecule,

with
a third molecule capable of detecting that the first moiety and the second moiety are in proximity or association,

in which, the first moiety and the second moiety constitute a part or all of a protein, and
the third molecule binds to the first moiety and/or the second moiety when the first moiety and the second moiety are in proximity; and

(2) recovering the complex by an affinity-based method using the third molecule, after step (1).

In the screening method according to the present disclosure, the molecules identified in each step coexist in a screening system by mixing those molecules. Thus, as an example, step (1) in the present disclosure can be expressed as follows:

(1) allowing the first target molecule linked to a first moiety, the second target molecule linked to a second moiety, and a library containing a plurality of test molecules to coexist, in which the first moiety and the second moiety constitute a part or all of a protein.

In the present disclosure, the molecules identified in each step may be contacted with each other by mixing those molecules. That is, as an example, step (1) in the present disclosure can be expressed as follows:

(1) contacting the first target molecule linked to a first moiety, the second target molecule linked to a second moiety, and a library containing a plurality of test molecules with each other, in which the first moiety and the second moiety constitute a part or all of a protein.

As is described later, the complex containing the first target molecule linked to the first moiety, the second target molecule linked to the second moiety, and the candidate molecule in the present disclosure also includes aspects in which the candidate molecule binds to the first (or second) target molecule and the first (or second) target molecule binds to the second (or first) target molecule (where the candidate molecule does not directly bind to the second (or first) target molecule). Thus, in step (1) in the present disclosure, the test molecule is not required to contact both the first

target molecule and the second target molecule. Step (1) in the present disclosure encompasses aspects in which the test molecule is contacted only with either one of the first target molecule or the second target molecule as long as the first target molecule linked to the first moiety, the second target molecule linked to the second moiety, and a library containing a plurality of test molecules are mixed or coexist in the same system.

Step (2)

The screening method according to the present disclosure comprises a step (step (2)) of recovering a complex containing the first target molecule, the second target molecule, and the candidate molecule formed in step (1) by an affinity-based method using a third molecule capable of detecting that the first moiety and the second moiety are in proximity or association. In the screening method according to the present disclosure, when a complex containing the first target molecule linked to the first moiety, the second target molecule linked to the second moiety, and the candidate molecule is formed, the first moiety and the second moiety are in proximity or association in the complex. According to or in conjunction with this, a complex containing the first moiety and the second moiety is formed. Thus, in the present disclosure, the state where the first moiety and the second moiety are in proximity or association can also be described as the state where a complex containing the first moiety and the second moiety is formed. In the screening method according to the present disclosure, the third molecule can then react with or bind to the first moiety and/or the second moiety when the first moiety and the second moiety are in proximity or association. In the screening method according to the present disclosure, a complex including the first target molecule, the second target molecule, the candidate molecule, and the third molecule thus formed can be selected, and the candidate molecule can be recovered from the complex. In one aspect, a complex consisting of the first target molecule, the second target molecule, and the candidate molecule can be obtained by cleaving the first moiety and the second moiety from the first target molecule and the second target molecule, respectively. In addition, the candidate molecule can be recovered from the complex. The third molecule in the present disclosure is a molecule capable of detecting at least one of "a complex containing the first target molecule, the second target molecule, and a candidate molecule", "a complex containing the first target molecule linked to a first moiety, the second target molecule linked to a second moiety, and a candidate molecule", "a complex containing a first moiety and a second moiety", and "that the first moiety and the second moiety are in proximity or association" described above.

Meanwhile, in the screening method according to the present disclosure, when the test molecule does not bind to either the first target molecule or the second target molecule, a complex containing the first target molecule, the second target molecule, and the candidate molecule is not formed. And when such a complex is not formed, the first moiety and the second moiety are neither in proximity nor in association. In other words, a complex containing the first moiety and the second moiety is not formed when the candidate molecule is not present. In this case, the third molecule does not substantially react with or bind to the first moiety and/or to the second moiety. In this case, the test molecule is not recovered by the affinity-based method using the third molecule. Thus, in this case, a complex containing the first target molecule linked to the first moiety, the second target molecule linked to the second moiety, the candidate molecule, and the third molecule is not formed, and the probability of such a test molecule being isolated can be reduced using the screening method according to the present disclosure, even if there is a molecule capable of forming a complex only with the first target molecule or a molecule capable of forming a complex only with the second target molecule among the test molecules. The screening method of the present disclosure can prevent the decreasing of detection accuracy due to the presence of such candidate molecules.

In one aspect, the complex containing "the first target molecule, the second target molecule, and the candidate molecule" referred to in step (2) can refer to a complex containing "the first target molecule linked to the first moiety, the second target molecule linked to the second moiety, and the candidate molecule". The complex may further contain a third molecule depending on the context.

Step (2A)

In one aspect, the screening method according to the present disclosure can further comprise, after recovering the complex containing the first target molecule, the second target molecule, and the candidate molecule, a step of eluting the candidate molecule from the complex (step (2A)). In one aspect, the elution can be performed by an enzymatic reaction or thermal reaction. For elution by the enzyme reaction, TEV proteases described below can be used.

Step (2B)

In one aspect, the screening method according to the present disclosure can comprise a step (step (2B)) of amplifying the polynucleotide when the candidate molecule contains or is linked to the polynucleotide, when the candidate molecule is encoded by the polynucleotide, or when the candidate molecule is the polynucleotide or the like. Amplification

of the polynucleotide can be performed by the methods well known to those skilled in the art, such as polymerase chain reaction (PCR), loop-mediated isothermal amplification (LAMP), transcription-mediated amplification (TMA), transcription-reverse transcription-related reaction (TRC), ligase chain reaction (LCR), standard displacement amplification (SDA), or nucleic acid sequence-based amplification (NASBA).

In one aspect, an mRNA library may be obtained by transcribing the polynucleotide amplified above. Using the mRNA library as a template, a library of peptides may be produced, and the library of peptides may be used for the screening method according to the present disclosure. Since such libraries are enriched for peptides capable of forming a complex with the first target molecule and the second target molecule, panning again using such libraries can further enrich the peptides capable of forming a complex with the first target molecule and the second target molecule. In the screening method according to the present disclosure, steps (1) and (2), steps (1), (2) and (2B), or steps (1), (1A), (2) and (2B) can be repeated multiple times (e.g., two or more times, three or more times, or four or more times) to enrich the candidate molecule of interest. In the screening method according to the present disclosure, the candidate molecule of interest can also be enriched by repeating steps (1), (2), (2A) and (2B), or steps (1), (1A), (2), (2A) and (2B) multiple times (e.g., two or more times, three or more times, or four or more times). In one aspect, when the candidate molecule is a peptide linked to a polynucleotide, an amino acid sequence can be identified based on the nucleotide sequence information contained in the peptide thus enriched, and a peptide capable of forming a complex with the first target molecule and the second target molecule can be produced. In one aspect, the screening method according to the present disclosure can be performed in vitro.

<u>Step (3)</u>

In one aspect, the screening method according to the present disclosure can further comprise a step of identifying the candidate molecule contained in a recovered complex containing the first target molecule, the second target molecule, and the candidate molecule. Upon the complex containing the first target molecule, the second target molecule, and the candidate molecule is obtained, the candidate molecule can be isolated and obtained therefrom by methods well known to those skilled in the art. In one aspect, the identification of the candidate molecule can be performed after isolating the candidate molecule from the complex containing the first target molecule, the second target molecule, and the candidate molecule. The identification of the candidate molecule can be performed by methods well known to those skilled in the art. For example, when the candidate molecule is a peptide or compound linked to a polynucleotide, the peptide or compound can be identified by sequencing a nucleotide sequence of the polynucleotide encoding the candidate molecule. Specifically, an amino acid sequence of a peptide or compound linked to a polynucleotide can be identified, for example, by amplifying the polynucleotide by PCR and analyzing the nucleotide sequence. When the polynucleotide is an mRNA, cDNA synthesized from the mRNA may be used.

When the candidate molecule is not tagged, the candidate molecule can be identified, for example, by Affinity Selection Mass Spectrometry (ASMS).

<u>Steps (4) and (5)</u>

In one aspect, the screening method according to the present disclosure can comprise a step of mixing the first target molecule fixed on a solid phase support and a library containing a plurality of test molecules, and recovering a test molecule capable of binding to the first target molecule (step (4)); and/or a step of mixing the second target molecule fixed on a solid phase support and a library containing a plurality of test molecules, and recovering a test molecule capable of binding to the second target molecule (step (5)). In step (4), the first target molecule fixed on a solid phase support, the free second target molecule, and the library containing a plurality of test molecules may be mixed. In step (5), the second target molecule fixed on a solid phase support, the free first target molecule, and the library containing a plurality of test molecules may be mixed. In one aspect, step (5) may be performed after step (4), or step (4) may be performed after step (5). Steps (4) and (5) may be combined with steps (1) to (3). For example, steps (4) and (5) may be performed, for example, before step (1). The number of steps (4) and (5) has no limit. For example, steps (4) or (5) may be repeated one or more times (e.g., two or more times, three or more times, or four or more times) independently. Further, steps (4) and (5) as a set may be repeated one or more times (e.g., two or more times, three or more times, or four or more times).

In an aspect, each of steps (4) and (5) can be followed by a step of eluting a test molecule or candidate molecule, similar to step (2A) described above. When the candidate molecule contains or is linked to a polynucleotide, the method can comprise a step of amplifying the polynucleotide after each of steps (4) and (5) in the same manner as in step (2B) described above. In an aspect, the identification of the candidate molecule can be performed according to step (3) described above.

Without being bound by a particular theory, it is believed that, when one target molecule (e.g., a first target molecule) is fixed to a solid phase support and the other target molecule (e.g., a second target molecule) is in a free

state upon mixing with a library containing a test molecule, the group of candidate molecules to be recovered may include candidate molecules capable of forming a complex with both target molecules. It is then believed that mixing the previously obtained group of candidate molecules with the target molecules in a state where the target molecule fixed to the solid phase support and the free target molecule are replaced with each other will enrich the candidate molecules capable of forming a complex with both target molecules. It is believed that performing a panning in this manner while alternating the fixed/free state of the target molecule enables a candidate molecule capable of forming a complex with a plurality of target molecules to be selected from a library. In one aspect, steps (4) and (5) may be performed before step (1), and it is believed that, by panning in this order, the candidate molecule capable of specifically binding to the first target molecule and/or the second target molecule can be efficiently enriched. Specifically, it is believed that the residual rate of a non-specific test molecule having no binding affinity for the target molecule can be reduced at step (1) or thereafter.

In one aspect, the screening method according to the present disclosure is a method of screening a candidate molecule capable of forming a complex with a first target molecule and a second target molecule, comprising the following steps of:

(4) mixing the first target molecule fixed on a solid phase support and a library containing a plurality of test molecules, and recovering a test molecule capable of binding to the first target molecule; and
(5) mixing the second target molecule fixed on a solid phase support and a library containing a plurality of test molecules, and recovering a test molecule capable of binding to the second target molecule.

Third molecule

As described above, in the screening method according to the present disclosure, when a complex is formed among the first target molecule linked to the first moiety, the second target molecule linked to the second moiety, and the candidate molecule, the first moiety and the second moiety are in proximity or association in the complex. In the present disclosure, the third molecule is a molecule capable of detecting that the first moiety and the second moiety in the present disclosure are in proximity or association. In another aspect, the third molecule in the present disclosure is a molecule capable of causing a complex containing the first moiety and the second moiety to form when the first moiety and the second moiety are in proximity or association. In one aspect, the third molecule in the present disclosure can be a molecule that is derived from a protein that provides the first moiety and the second moiety. In one aspect, the amino acid sequence of the third molecule may be the same as or different from the wild-type amino acid sequence present in the protein that provides the first moiety and the second moiety. In one aspect, the third molecule in the present disclosure, together with the first moiety and the second moiety, can constitute a part or all of a protein, e.g., can constitute the entirety of GFP or luciferase. Even in this case, the third molecule can contain a tag and/or a linker sequence or the like that is not contained in the protein that provides the first moiety and the second moiety. Even in this case, the third molecule, together with the first moiety and the second moiety, can constitute a part or all of the protein. In one aspect, the third molecule in the present disclosure also includes one having a modification in the amino acid sequence. For example, the amino acid sequence may have amino acid modifications (deletion, insertion, substitution and/or addition of one or more amino acids) suitable for the preparation, stability, or the like of the third molecule.

When screening a test molecule using affinity instead of a conventional method using luminescence as an indicator, it is required that the third molecule be a molecule that reacts with or binds to the first moiety and/or the second moiety only when the first moiety and the second moiety are in proximity or association. The present inventors have thus verified whether GFP1-9 can associate with GFP11 (or GFP10) in the absence of GFP10 (or GFP11), or whether GFP1-9 requires the presence of both GFP10 and GFP11 to associate with GFP10 and GFP11. As a result, the present inventors have found that GFP 1-9 does not associate with GFP 11 (or GFP10) in the absence of GFP10 (or GFP11) and that GFP10 and GFP11 must be in proximity, association, or interaction so that GFP1-9 associates with GFP10 and GFP11.

Furthermore, the present inventors have conducted similar validation to that for GFP described above, using Nanoluc (registered trademark), a type of luciferase. That is, verification was performed to determine whether Nanoluc1-8 can associate with Nanoluc-10 (or Nanoluc-9) in the absence of Nanoluc-9 (or Nanoluc-10), or whether Nanoluc1-8 requires the presence of both Nanoluc-9 and Nanoluc-10 to associate with Nanoluc-9 and Nanoluc-10. As a result, the present inventors have found that Nanoluc1-8 does not associate with Nanoluc-10 (or Nanoluc-9) in the absence of Nanoluc-9 (or Nanoluc-10) and that Nanoluc-9 and Nanoluc-10 must be in proximity, association, or interaction so that Nanoluc1-8 associates with Nanoluc-9 and Nanoluc-10.

These findings have been important in establishing a system that can screen a large library having diversity for candidate molecules by applying an affinity-based method of recovering candidate molecules to the split-protein system represented by Split GFP techniques and Nanoluc techniques, while excluding a test molecule that forms a complex with only one target molecule.

Without being bound by a particular theory, the present inventors consider the following. When fluorescence

is used as an indicator to screen libraries for binders for target molecules, it may take some time for the fluorophores to mature, thus converging over time into a binder population having certain or similar properties (e.g., high binding activity), which may make cause problems to obtain candidate molecules with diverse properties. To increase the probability of obtaining candidate molecules that provide complex binding/reaction modes, it is desired that a screening method can obtain candidate molecules having as diverse properties as possible, rather than a population of molecules having a specific or similar property, regardless of the low binding activity. Also from this viewpoint, it is important for screening for candidate molecules in the present disclosure to establish a method of screening for candidate molecules utilizing affinity as an alternative to the method using luminescence as an indicator.

Furthermore, the present inventors have found that molecules capable of forming a complex with a plurality of proteins can be screened for by using ubiquitin and SNAP tags as proteins, similarly to the manner using GFP or Nanoluc. That is, the present inventors have conceived and revealed that similar screening can be performed using split ubiquitin, based on the use of anti-ubiquitin antibodies that can bind to full-length ubiquitin, but not to the fragment of split ubiquitin. The present inventors have also conceived and revealed that similar screening can be performed using split SNAP, based on the properties that the full-length SNAP tag can react with the substrate SNAP-biotin, whereas the fragment split SNAP cannot react with SNAP-biotin.

Thus, in one aspect, the third molecule in the present disclosure is a molecule that can react with or bind to the first moiety and/or the second moiety when the first moiety and the second moiety are in proximity or association. In another aspect, the third molecule in the present disclosure is a molecule that can react with or bind to the first moiety and/or the second moiety when a complex is formed among the first target molecule linked to the first moiety, the second target molecule linked to the second moiety, and the candidate molecule.

In one aspect in the present disclosure, the first moiety and the second moiety can be said to be in proximity or association when a complex is formed among the first target molecule linked to the first moiety, the second target molecule linked to the second moiety, and the candidate molecule. In another aspect, the first moiety and the second moiety can be said to be in proximity or association when a complex containing the first moiety and the second moiety is formed.

The proximity of the first moiety and the second moiety and the association of the first moiety and the second moiety in the present disclosure are of any degree and any mode as long as they are due to the formation of a complex among the first target molecule linked to the first moiety, the second target molecule linked to the second moiety, and the candidate molecule. For example, a state in which the first moiety and the second moiety are in proximity or association in the present disclosure includes both a state in which the first moiety and the second moiety are bound by an intermolecular force or the like and a state in which they are not bound.

As described above, in the screening method according to the present disclosure, when a complex is not formed among the first target molecule linked to the first moiety, the second target molecule linked to the second moiety, and the candidate molecule, the first moiety and the second moiety are neither in proximity nor in association. In an aspect, the third molecule in the present disclosure is a molecule that does not substantially react with or bind to the first moiety and/or the second moiety when the first moiety and the second moiety are neither in proximity nor in association. In another aspect, the third molecule in the present disclosure is a molecule that is unable to react with or bind to the first moiety and/or the second moiety when a complex is not formed among the first target molecule linked to the first moiety, the second target molecule linked to the second moiety, and the candidate molecule.

In yet another aspect, the third molecule in the present disclosure is a molecule that reacts with or binds to the first moiety and/or the second moiety in less than one-tenth, less than one-twentieth, less than one-thirtieth, or less than one-fortieth when the first moiety and the second moiety are neither in proximity or nor in association, as compared to when the first moiety and the second moiety are in proximity or association. In yet another aspect, the third molecule in the present disclosure is a molecule that reacts with or binds to the first moiety and/or the second moiety in less than one-tenth, less than one-twentieth, less than one-thirtieth, or less than one-fortieth when a complex containing the first target molecule linked to the first moiety, the second target molecule linked to the second moiety, and the candidate molecule is not formed, as compared to when the complex is formed.

Whether or not the third molecule reacts with or binds to the first moiety and/or the second moiety can be determined, for example, by performing the screening method according to the present disclosure using FK506 binding protein/FK506 binding protein (FKBP) as the first target molecule, FKBP-rapamycin binding domain of FKBP12-rapamycin associated protein/FKBP-rapamycin binding domain of rapamycin binding protein (FRB) as the second target molecule, and rapamycin as the test molecule, and trying to recover a complex containing a molecule linking the first moiety with FKBP, a molecule linking the second moiety with FRB, rapamycin, and the third molecule; or a complex containing a molecule linked to the first moiety with FKBP, a molecule linked to the second moiety with FRB, and a molecule of rapamycin to determine whether the complex is recovered or not. In the present disclosure, when the complex is recovered, the third molecule can be determined to be a molecule that reacts with or binds to the first moiety and/or the second moiety. In another aspect, when the complex is not recovered, the third molecule can be determined to be a molecule that did not substantially react with or bind to the first moiety and/or the second moiety. Also in the present disclosure,

a molecule can be determined to be a molecule that did not substantially react with or bind to the first moiety and/or the second moiety when the extent of the reaction or binding of the molecule to the first moiety and/or the second moiety is less than one-tenth, less than one-twentieth, less than one-thirtieth, or less than one-fortieth as compared to that of the third molecule capable of reacting with or binding to the first moiety and/or the second moiety when the first moiety and the second moiety are in proximity or association. Whether or not the complex containing a molecule linked the first moiety with FKBP, a molecule linking the second moiety with FRB, rapamycin, and the third molecule; or the complex containing a molecule linked to the first moiety with FKBP, a molecule linked to the second moiety with FRB, and rapamycin, and the third molecule can be recovered or a recovery rate thereof can be calculated, for example, using the amount of nucleic acid (number of molecules) linked to rapamycin as an indicator.

In the present disclosure, the third molecule can be determined to be a molecule that reacts with or binds to the first moiety and/or the second moiety when the first moiety and the second moiety are in proximity or association, when (a) the recovery rate of rapamycin obtained when the screening method according to the present disclosure is performed using FKBP as the first target molecule, FRB as the second target molecule, and rapamycin as the test molecule is compared to (b) the recovery rate of rapamycin obtained when the screening method according to the present disclosure is performed using FKBP as the first target molecule and rapamycin as the test molecule without the second target molecule, or (c) the recovery rate of rapamycin obtained when the screening method according to the present disclosure is performed using FRB as the second target molecule and rapamycin as the test molecule without the first target molecule, and then the recovery rate in (a) is 5 times or more, 10 times or more, 15 times or more, 20 times or more, 30 times or more, or 40 times or more higher than the recovery rate in (b) or (c). In other words, in this case, the third molecule can be determined to be a molecule that does not significantly react with or bind to a complex containing either only the first target molecule or the second target molecule, but significantly reacts with or binds to only a complex containing the test molecule, the first target molecule linked to the first moiety and the second target molecule linked to the second moiety. In one aspect, the third molecule can be determined to be a molecule that does not significantly react with or bind to the first moiety and/or the second moiety when the first moiety and the second moiety are in proximity or association, in case that, when the (a) above and the (b) or (c) above are compared, the recovery rate in (a) is 5 times or more, 10 times or more, 15 times or more, 20 times or more, 30 times or more, or 40 times or more lower than the recovery rate in (b) or (c). Such evaluations can be performed, for example, based on the descriptions in Examples.

As described above, one characteristic of the screening method according to the present disclosure is that an affinity-based method for recovering the candidate molecule is applied to the Split protein system represented by Split GFP techniques. Thus, the screening method according to the present disclosure comprises a step of recovering a complex containing the first target molecule linked to the first moiety, the second target molecule linked to the second moiety, the candidate molecule, and a third molecule, or a complex containing the first target molecule linked to the first moiety, the second target molecule linked to the second moiety, and the candidate molecule, by an affinity-based method using the third molecule. Accordingly, the third molecule in the present disclosure may have a tag for an affinity-based recovery of the complex. In one aspect, the third molecule may have a tag sequence for biotinylation. The third molecule may also contain a tag, a linker, and/or another sequence for purification.

<u>First moiety and second moiety</u>

In one aspect of the present disclosure, the first moiety and the second moiety can be moieties or fragments that form a part of a protein. In one aspect, the moiety or fragment does not have an original function or activity of the protein. The first and second moieties in the present disclosure can be moieties (fragments) of the protein obtained by dividing the protein into two, three, or more moieties. Alternatively, the first moiety and the second moiety in one aspect of the present disclosure can be moieties that constitute all or a part of the original protein when they are in proximity or association. Each moiety of the divided protein may have a partially overlapping amino acid sequence. In one aspect, such overlapping amino acid sequences can be present in the terminal portion of each moiety of the divided protein. In aspects where all of the original protein is constituted when the first moiety and the second moiety are associated, the protein in which the first moiety and the second moiety are in association may have the structure, function, or activity that the protein had before it was divided. Also, in aspects where a part of the original protein is constituted when the first moiety and the second moiety are associated, the protein in which the first moiety and the second moiety are in proximity or association may have the structure, function, or activity that the protein had before it was divided, by further associating with another moiety that constitutes the protein, e.g., the third molecule. Examples of the former protein include a SNAP tag and ubiquitin, and examples of the latter protein include GFP and luciferase.

In the present disclosure, a protein from which the first moiety and the second moiety are derived may be referred to as a "protein that provides the first moiety and the second moiety".

In the present disclosure, the amino acid sequence of the first moiety and/or the second moiety may be the same as or different from the wild-type amino acid sequence of the protein that provides the first moiety and the second moiety. For example, the amino acid sequence may have amino acid modifications (deletion, insertion, substitution,

and/or addition of one or more amino acids) suitable for the preparation, stability, or the like of the first moiety and the second moiety.

Complex containing first target molecule, second target molecule, and candidate molecule

In the screening method according to the present disclosure, when the candidate molecule is a molecule capable of forming a complex with the first target molecule and the second target molecule, a complex containing the first target molecule linked to the first moiety, the second target molecule linked to the second moiety, and the candidate molecule is formed. In one aspect, removing the first moiety and the second moiety from the complex can result in a complex containing the first target molecule, the second target molecule, and the candidate molecule. In the present disclosure, the complex containing the first target molecule, the second target molecule, and the candidate molecule is preferably a complex composed of the first target molecule, the second target molecule, and the candidate molecule or a complex composed of the first target molecule, the second target molecule, the candidate molecule, and the third molecule. In an aspect, the complex according to the present disclosure may be a complex of four, five, or six or more components, including other molecules, as long as it contains the three or four components described above, but preferably is a complex of three components.

The complex in the present disclosure is not limited to any aspects as long as the first target molecule, the second target molecule, and the candidate molecule form a complex. That is, the complex in the present disclosure includes aspects in which a complex is formed by binding the candidate molecule to both the first target molecule and the second target molecule. Such aspects include those in which the first target molecule and the second target molecule are bound to each other via the candidate molecule, or in which any of the candidate molecule, the first target molecule, and the second target molecule are bound to both of the other two. In one aspect, a complex containing the first target molecule, the second target molecule, and the candidate molecule in the present disclosure also encompasses an aspect in which the candidate molecule binds to the first (or second) target molecule and the first (or second) target molecule binds to the second (or first) target molecule thereby forming a complex of three components. In such aspects, the candidate molecule is not directly bound to the second (or first) target molecule. In another aspect, the complex of the present disclosure also encompasses aspects in which a binding of the candidate molecule to the first (or second) target molecule alters the structure of the first (or second) target molecule and improves the binding affinity of the first (or second) target molecule to the second (or first) target molecule, thereby promoting the formation of a complex of three components. In such aspects, the candidate molecule is not directly bound to the second (or first) target molecule.

As described above, the first moiety and the second moiety in the present disclosure are fragments of a protein, specifically a protein that provides the first moiety and the second moiety. Thus, the "complex containing a first target molecule linked to a first moiety and a second target molecule linked to a second moiety" in the present disclosure includes all or a part of a protein that provides the first moiety and the second moiety in the present disclosure. Specifically, in an aspect where the protein that provides the first moiety and the second moiety is GFP or luciferase as described below, the complex includes a part of GFP or luciferase. In another aspect, for example, where the protein that provides the first moiety and the second moiety is a SNAP tag or ubiquitin, the complex includes the entire of the SNAP tag or ubiquitin.

First target linked to first moiety and second target linked to second moiety

In the present disclosure, the first moiety, the first target molecule, the second moiety, and the second target molecule may be linked via a linker. In the present disclosure, a linker between the first moiety and the first target molecule can be referred to as a first linker, and a linker between the second moiety and the second target molecule can be referred to as a second linker. As a linker, any peptide linker that can be introduced by genetic engineering or a linker that can be introduced by chemical synthesis (see, for example, Protein Engineering, 9(3), 299-305, 1996) can be used, but in the present invention, a peptide linker is preferred. The length of the peptide linker is not particularly limited and can be appropriately selected by those skilled in the art according to the purpose. The length is preferably 8 or more amino acids (the upper limit is not particularly limited and is usually 30 or less amino acids, preferably 20 or less amino acids), particularly preferably 12 amino acids.

Examples of the peptide linker can include

Ser,
Gly-Ser,
Gly-Gly-Ser,
Ser-Gly-Gly,
Gly-Gly-Ser-Ser (SEQ ID NO: 88)
Gly-Gly-Gly-Ser (SEQ ID NO: 89)

Ser-Gly-Gly-Gly (SEQ ID NO: 90)
Gly-Ser-Gly-Ser (SEQ ID NO: 91)
Gly-Gly-Gly-Gly-Ser (SEQ ID NO: 92)
Ser-Gly-Gly-Gly-Gly (SEQ ID NO: 93)
Gly-Gly-Gly-Gly-Gly-Ser (SEQ ID NO: 94)
Ser-Gly-Gly-Gly-Gly-Gly (SEQ ID NO: 95)
Gly-Gly-Gly-Gly-Gly-Gly-Ser (SEQ ID NO: 96)
Ser-Gly-Gly-Gly-Gly-Gly-Gly (SEQ ID NO: 97)
(Gly-Gly-Gly-Gly-Ser)n
(Ser-Gly-Gly-Gly-Gly)n
Gly-Gly-Gly-Gly-Ser-Gly-Gly-Gly-Ser Gly-Gly-Ser (SEQ ID NO: 124)
wherein n is an integer of 1 or larger. However, the length or sequence of the peptide linker can be appropriately selected by those skilled in the art according to the purpose.

In another aspect, as described below, a linker in the present disclosure can include a linker cleavable by an enzyme. Examples of such enzymes include Tobacco Etch Virus Protease (TEV protease), and in the present disclosure, a TEV protease to which a modification to improve stability, activity, specificity, and the like is added may be employed. Such TEV proteases can be purchased from reagent companies. Examples of the linker that is cleaved by a TEV protease include:
Glu-Asn-Leu-Tyr-Phe-Gln-Gly (SEQ ID NO: 2).

Test molecule and library

Examples of the test molecule in the present disclosure include, without particular limitation, a single compound such as a natural substance, an organic compound, an inorganic compound, a protein, a peptide, or an amino acid, as well as a compound library, an expression product of a gene library, a cell extract, a cell culture supernatant, a fermented microorganism product, a marine organism extract, a plant extract, a prokaryotic cell extract, a eukaryotic single-cell extract, or an animal cell extract. These may be purified products or crude purified products such as extracts of plants, animals or microorganisms. The method for producing the test molecule is not particularly limited, and the test molecule may be isolated from a natural substance, chemically or biochemically synthesized, or genetically engineered.
In an aspect, the test molecule in the present disclosure can be a molecule encoded by a polynucleotide. Such molecules include peptides. The test molecule in the present disclosure may also be a peptide linked to a polynucleotide that is a tag. The test molecule in the present disclosure may have a molecular weight of 3,000 or less, or 2,000 or less. When the test molecule includes a polynucleotide as a tag or a linker for linking to it, the molecular weight here refers to a molecular weight of the moiety excluding the polynucleotide or the linker.
In the present disclosure, the peptide refers to a peptide in which two or more amino acids are bound by an amide bond and/or an ester bond. Without limitation, the peptide in the present disclosure include a linear peptide and a cyclic peptide. The peptide in the present disclosure also includes a peptide, a complex of peptide and polynucleotide (peptide-polynucleotide complex), a complex of peptide and ribosome and polynucleotide, and the like. The "polynucleotide" in the present disclosure includes DNA, mRNA, and tRNA. The "peptide" in the present disclosure may also include a pharmaceutically acceptable salt thereof.
In one aspect, the peptide in the present disclosure has 2 to 100, 3 to 50, 4 to 30, or 5 to 20 amino acids bound by an amide bond and/or an ester bond. For example, in one aspect, when the peptide obtained by the screening method according to the present disclosure is used as a medicament, in order, the number of amino acids constituting the peptide may be preferably 20 or less, more preferably 18 or less, 16 or less, 15 or less, or 14 or less, particularly preferably 13 or less, and specifically, 9, 10, 11, 12, 13 to obtain high membrane permeability. To obtain high metabolic stability, the number of amino acids constituting the peptide is preferably 8 or more, more preferably 9 or more, further preferably 10 or more, and particularly preferably 11 or more. Considering having both membrane permeability and metabolic stability, the number of amino acids constituting the peptide is preferably 5 to 20 or 7 to 20, more preferably 7 to 17, 8 to 16, 9 to 16, or 10 to 16, further preferably 8 to 13, 10 to 15, 11 to 15, 10 to 14, 10 to 13, or 11 to 14, and particularly preferably 11 to 13.
The number of amino acids constituting the cyclic portion of the cyclic peptide is not limited, but examples thereof include a range identifiable by a combination of any of 4 or more, 6 or more, 7 or more, 8 or more, 9 or more, 10 or more, 11 or more, or 12 or more, and any of 20 or less, 18 or less, 16 or less, 15 or less, 14 or less, 13 or less, 12 or less, or 11 or less. Considering having both membrane permeability and metabolic stability, the number of amino acids constituting the cyclic portion is preferably 5 to 16, more preferably 5 to 15, 5 to 14, 7 to 14, or 8 to 14, further preferably 8 to 13, 9 to 13, 8 to 12, 8 to 11, or 9 to 12, and particularly preferably 9 to 11. In one aspect, examples of the number of amino acids constituting the cyclic portion of the cyclic peptide include 7, 8, 9, 10, 11, 12, 13, 14, 15, and

16. In the present disclosure, the "cyclic portion" of the peptide means a cyclic portion in which 4 or more amino acid residues are linked to form the cyclic portion.

In one non-limiting aspect, the cyclic peptide in the present disclosure may have a linear portion. As used in the present disclosure, a "linear portion" used in referring to a partial structure of the cyclic peptide refers to a portion that is not included in the main chain structure of the cyclic portion and has at least one amide bond and/or ester bond on the chain of the portion. The number of amino acids (number of units) of the linear portion is preferably 0 to 8, more preferably 0 to 5, and more preferably 0 to 3. In one non-limiting aspect, the linear portion in the present disclosure may contain a natural amino acid or a non-natural amino acid (including a chemically modified or backbone-altered amino acid).

In a non-limiting aspect, the number of non-natural amino acids contained in the peptide in the present disclosure is preferably 2 or more, more preferably 4 or more, or 5 or more, further preferably 6 or more, and preferably 20 or less, 15 or less, 14 or less, 13 or less, 12 or less, or 10 or less. Examples of the number of non-natural amino acids contained in the peptide in the present disclosure include 30% or more, 40% or more, 50% or more, 60% or more, or 70% or more of the number of amino acids constituting the cyclic portion. The types of non-natural amino acid contained in the peptide in the present disclosure are preferably 1 or more, more preferably 2 or more, 3 or more, or 4 or more, or further preferably 7 or more, and preferably 20 or less, 15 or less, 14 or less, 13 or less, 12 or less, or 10 or less.

It should be noted that the test molecule in the present disclosure may be already known to bind to either one of the first target molecule or the second target molecule in the present disclosure. The test molecule in the present disclosure may also be a molecule selected in a known screening system. Thus, the screening method according to the present disclosure can further comprise a step of obtaining a test molecule using a known screening system, before step (1). In the screening method according to the present disclosure, the test molecule thus obtained (e.g., a molecule already known to bind to either the first target molecule or the second target molecule) can be used as the test molecule in step (1).

The test molecules in the present disclosure can constitute a library. The library in the present disclosure include a library tagged (i.e., encoded) by nucleic acids (or "polynucleotides") (e.g., DNA encoded library; DNA-encoded library/DEL, mRNA display library, DNA display library; e.g., Molecules. 2019 Apr; 24(8): 1629) or library of tag-free compounds. As the library in the present disclosure, a display library is preferred. Examples of the display library include a display-utilizing library. Among them, an mRNA display library, a DNA display library, and a ribosome display library are preferred, and an mRNA display library are more preferred.

The library tagged (encoded) by nucleic acids is a library in which each compound that constitutes the library is tagged with a specific polynucleotide serving as a barcode. This library can be used to screen candidate molecules capable of specifically binding to a target molecule. For example, candidate molecules that bind to a desired target can be enriched by contacting a library with the target and washing away molecules that do not bind to the target (panning method). It is possible to identify the candidate molecule bound to the target by analyzing the tags corresponded to the candidate molecules selected through such a process (see, e.g., WO2013/100132).

When a peptide is used as a test molecule, the peptide, which is a phenotype, and RNA or DNA encoding the peptide, which is a genotype, are corresponded. For example, methods of utilizing the antibiotic puromycin, an analog of aminoacyl tRNA, which is non-specifically linked to a protein during mRNA translation extension by ribosome are reported as mRNA display (Proc Natl Acad Sci USA. 1997;94:12297-302. RNA-peptide fusions for the in vitro selection of peptides and proteins. Roberts RW, Szostak JW.) or in vitro virus(FEBS Lett. 1997;414:405-8. In vitro virus: bonding of mRNA bearing puromycin at the 3'-terminal end to the C-terminal end of its encoded protein on the ribosome in vitro. Nemoto N, Miyamoto-Sato E, Husimi Y, Yanagawa H.).

When a spacer such as puromycin is linked to the 3' end of the library of mRNA obtained by transcription from a DNA library including a promoter such as a T7 promoter, and the mRNA is translated into a protein in a cell-free translation system, the puromycin is mistakenly incorporated into a protein by the ribosome as an amino acid, and the mRNA and the protein encoded thereby are linked to yield a library in which the mRNA and the product are corresponded. In this process, high efficiency is achieved because it does not include transformation such as with E. coli, and a large display library can be constructed. It is possible to identify the sequence of the bounded peptide by synthesizing cDNA from mRNA which is tagged to molecules enriched and selected in panning and containing genetic information, subjecting the cDNA to PCR amplification, and analyzing the nucleotide sequence.

Examples of the known display library utilizing cell-free translation systems include, in addition to mRNA displays, a cDNA display that is a library of cDNA encoding a peptide to which a complex of a peptide and puromycin is attached (Nucleic Acids Res. 2009;37(16):e108. cDNA display: a novel screening method for functional disulfide-rich peptides by solid-phase synthesis and stabilization of mRNA-protein fusions. Yamaguchi J, Naimuddin M, Biyani M, Sasaki T, Machida M, Kubo T, Funatsu T, Husimi Y, Nemoto N.), a ribosome display utilizing a relatively stable complex of ribosome and a translation product during mRNA translation (Proc Natl Acad Sci USA. 1994;91:9022-6. An in vitro polysome display system for identifying ligands from very large peptide libraries. Mattheakis LC, Bhatt RR, Dower WJ.), a covalent display in which bacteriophage endonuclease P2A forms a covalent bond with DNA (Nucleic AcidsRes. 2005;33:e10. Covalent antibody display--an in vitro antibody-DNA library selection system. Reiersen H, Lobersli I, Loset GA, Hvattum

E, Simonsen B, Stacy JE, McGregor D, Fitzgerald K, Welschof M, Brekke OH, Marvik OJ.) and a CIS display utilizing the replication initiation protein RepA of the plasmid of the microorganism to bind to the replication initiation point ori (Proc Natl Acad Sci USA. 2004;101:2806-10. CIS display: In vitro selection of peptides from libraries of protein-DNA complexes. Odegrip R, Coomber D, Eldridge B, HedererR, Kuhlman PA, Ullman C, FitzGerald K, McGregor D.). Also known is in vitro compartmentalization in which, for each molecule of DNA that constitutes a DNA library, a transcriptional translation system is encapsulated in a water-in-oil emulsion or liposome, and a translation reaction is performed (Nat Biotechnol. 1998;16:652-6. Man-made cell-like compartments for molecular evolution. Tawfik DS, Griffiths AD.). As appropriate, the above methods can be used following a known manner.

Examples of the library of tag-free compounds include a nucleic acid library, a peptide library obtained by translating the nucleic acid library, and a library of compounds obtained by chemical synthesis. The "nucleic acid" here can include a deoxyribonucleic acid (DNA), a ribonucleic acid (RNA), and also a nucleotide derivative having an artificial base. It can also include a peptide nucleic acid (PNA). The nucleic acid of the present disclosure can be any of these nucleic acids or a mixture of them as long as the genetic information of interest is retained. That is, DNA-RNA hybrid nucleotides or chimeric nucleic acids in which different nucleic acids such as DNA and RNA are linked in a single strand are also included in the nucleic acids in the present disclosure.

Examples of the library of nucleic acids that serve as templates for the peptides contained in the peptide library include an mRNA library and a DNA library. A nucleic acid library can be obtained by adding bases to the positions where amino acid residues are not fixed on a peptide sequence and synthesizing them. For example, a nucleic acid library can be synthesized as a mixture of A, T, G, C in the case of DNA library, and A, U, G, C in the case of RNA library, of 3 times the number of 4 bases (N) or a mixture of 2 bases, such as N for the first and second letters of the codon and W, M, K, and S for the third letter. When the types of amino acids introduced are reduced to be 16 or less, there is a method of setting the base at the third letter to be one type of base. In addition, a codon unit corresponding to three letters of the codon is prepared, and the frequency of occurrence of amino acid residues may be freely adjusted by mixing it in any percentage and using it for synthesis.

A cell-free translation system can be used to translate these nucleic acid libraries. When a cell-free translation system is used, it is preferable to include a sequence encoding a spacer downstream of the nucleic acid of interest. Examples of the spacer sequence include, but are not limited to, a sequence including glycine and serine. It is also preferable that a linker formed by polymers (e.g., five polymers) of RNA, DNA, hexaethylene glycol (spc18) or the like is included between a compound incorporated into a peptide and a nucleic acid library during the time of translation by a ribosome such as puromycin or a derivative thereof.

The number of variations (types) of test molecules included in a library herein is referred to as "diversity". The diversity of candidate molecules included in libraries in the present disclosure is not particularly limited, and examples thereof include $1 \times 10^3$ or more, $1 \times 10^4$ or more, $1 \times 10^5$ or more, $1 \times 10^6$ or more, $1 \times 10^7$ or more, $1 \times 10^8$ or more, $1 \times 10^9$ or more, $1 \times 10^{10}$ or more, $1 \times 10^{11}$ or more, and $1 \times 10^{12}$ or more. These diversities are not limited to measured values, and may be theoretical values.

<u>Candidate molecule</u>

The candidate molecule in the present disclosure is a molecule capable of forming a complex with the first target molecule and the second target molecule. The complex may contain other molecules as long as it contains the first target molecule, the second target molecule, and the candidate molecule, and may be a complex of three components, a complex of four components, or a complex of five or more components, but a complex of three components is preferred.

The candidate molecules in the present disclosure are not limited as long as they are capable of forming a complex with the first target molecule and the second target molecule, but preferred examples thereof include peptides, more preferably cyclic peptides.

In one aspect, the candidate molecule in the present disclosure can be a molecule that has a binding activity to the first target molecule and binding activity to the second target molecule, as a molecule alone. In another aspect, the candidate molecule in the present disclosure can be a molecule capable of binding to the first target molecule and the second target molecule simultaneously. In addition to such molecules, the candidate molecule in the present disclosure may also include a molecule that does not have a binding activity to the second (or first) target molecule alone, but acquires binding activity to the second (or first) target molecule due to the binding to the first (or second) target molecule, thereby becoming capable of binding to the first target molecule and the second target molecule. Examples of such molecules include a molecule having 2 times or more, 5 times or more, 10 times or more, or 20 times or more binding affinity for the second (or first) target molecule when bound to the first (or second) target molecule as compared to when alone. Further, the candidate molecule of the present disclosure can also include:

(i) a candidate molecule capable of binding to the first (or second) target molecule, wherein the binding affinity of the candidate molecule for the second (or first) target molecule is higher than before the binding, due to the binding;

(ii) a candidate molecule capable of binding to the first (or second) target molecule, the candidate molecule being capable of binding to the second (or first) target molecule as a complex of the candidate molecule and the first (or second) target molecule, due to the binding; and

(iii) a candidate molecule capable of binding to the first (or second) target molecule, wherein the binding affinity of the first (or second) target molecule for the second target molecule is higher than before binding, due to the binding.

Examples of such candidate molecules may include a molecule capable of altering the structure of the first (or second) target molecule by binding to the first (or second) target molecule, thereby increasing the binding affinity of the first (or second) target molecule for the second (or first) target molecule, and thus directing the formation of a complex containing the first target molecule, the second target molecule, and the candidate molecule.

In the "complex containing the first target molecule, the second target molecule, and a candidate molecule" in the present disclosure, the candidate molecule may bind only to the first target molecule, may bind only to the second target molecule, and may bind to both the first target molecule and the second target molecule.

In a non-limiting aspect, the candidate molecules obtained by the screening method according to the present disclosure may be optimized by chemical modification or the like by known methods. Also, derivatives of the candidate molecules may be chemically synthesized. As used in the present disclosure, "optimization" means chemically modifying the molecule for making a more drug-like molecule, chemically modifying the molecule for making a molecule having a stronger activity to a pharmaceutical target, and/or chemically modifying the molecule for making a molecule having less toxicity. For example, when the candidate molecule is a peptide, it can be "optimized" by altering the structure of each amino acid in the peptide. In the present disclosure, the optimized candidate molecules or the derivatives of the candidate molecules may be subjected to the screening methods in the present disclosure for their evaluation.

Target molecule

In the present disclosure, the first target molecule and the second target molecule are not particularly limited, and examples thereof include proteins, peptides, nucleic acids, sugars, lipids, and the like, but it is preferred that the targeted molecules are proteins. The target molecules in vivo are not particularly limited in their location either. In one aspect, a protein in a cell can be a target molecule. In another aspect, a protein enabling a drug discovery for tough targets that have been difficult so far can be a target molecule.

In one aspect, examples of the first target molecule include a protein belonging to the immunophilin family, ubiquitin E3 ligase, or the like. Examples of the protein belonging to the immunophilin family include FK506 binding protein/FK506 binding protein (FKBP) and cyclophilin. The second target molecule is not particularly limited, but, for example, when a protein belonging to the immunophilin family is used as the first target molecule, examples of the second target molecule include calcineurin, FKBP-rapamycin binding domain of FKBP12-rapamycin-associated protein (FRB), or proteins containing the same.

In another aspect, examples of the first target molecule include a Ras protein such as Kras, Nras, Hras, or the like. In one aspect in this case, examples of the second target molecule include FKBP.

In one aspect, a target molecule may be fixed on a solid phase support. For example, in steps (4) and (5), one target molecule may be fixed to a solid phase support, and the other target molecule may be in a free state. The solid phase support is not particularly limited as long as it is a carrier capable of fixing the target molecule, and a solid phase support commonly used in the art can be used. Examples of the solid phase support include beads (e.g., magnetic beads, glass beads), membranes, microplates, and silicon chips. The target molecules can be fixed to those solid phase supports by known methods.

Affinity-based method

In the screening method according to the present disclosure, a complex containing the first target molecule linked to the first moiety, the second target molecule linked to the second moiety, and the candidate molecule can be recovered by an affinity-based method using the third molecule. Examples of the affinity-based method include a pull-down method. The pull-down method is a method of adding a tag to a protein or protein complex and utilizing the interaction of the tag and a protein that interacts with the tag to detect the protein or protein complex. In an example of the pull-down method, a complex containing the first target molecule linked to the first moiety, the second target molecule linked to the second moiety, the candidate molecule, and the third molecule can be selected by adding a tag to the third molecule and utilizing the interaction between the tag and a protein that interacts with the tag. In one aspect, the third molecule and tag may be linked via a linker of the present disclosure as described above. In another aspect, the tag itself can be used as the third molecule. In this case, the complex can be selected by adding a tag to either or both of the first moiety and the second moiety that are in proximity or association, and selecting based on the interaction between the tag and a protein that interacts with the tag. As described above, in the screening method of the present disclosure,

the third molecule can specifically react with or bind to a complex containing the first target molecule linked to the first moiety, the second target molecule linked to the second moiety, and the candidate molecule, and thus the candidate molecule bound to the first target molecule and the second target molecule can be efficiently obtained by utilizing affinity with the third molecule.

Examples of the combination of a protein available as a tag and a protein that interacts with the tag in the present disclosure can include a combination of biotin or a derivative thereof and a biotin-binding protein (e.g., streptavidin), a combination of a Strep-tag (registered trademark) and Strep-Tactin (registered trademark), a combination of a Streptavidin binding peptide (SBP)-tag and streptavidin, and a combination of a known peptide tag (His-tag, HA-tag, FLAG-tag, or the like) and an antibody thereto.

In the present disclosure, the affinity between an antigen and an antibody can also be utilized to recover a complex containing the first target molecule linked to the first moiety, the second target molecule linked to the second moiety, and the candidate molecule. Specifically, antibodies that specifically recognize a complex containing the first moiety and the second moiety that are in proximity or association can be used to recover a complex containing the first target molecule linked to the first moiety, the second target molecule linked to the second moiety, and the candidate molecule.

In an aspect, in the screening method according to the present disclosure, the candidate molecule or a complex containing the candidate molecule can be isolated by elution. In an aspect, the elution can be performed by an enzymatic reaction or thermal reaction. In a further aspect, the elution may be performed by cleaving the linker contained in the third molecule. In another further aspect, the elution may be performed by cleaving the first linker located between the first moiety and the first target molecule and/or the second linker located between the second moiety of the protein and the second target molecule. In this case, the linker contained in the third molecule or the first linker and/or the second linker can be cleaved, for example, by an enzymatic reaction. Thus, the linker contained in the third molecule in the present disclosure or the first linker and/or the second linker can contain a sequence cleavable by an enzyme. Examples of the combination of an enzyme and a sequence cleavable by the enzyme include TEV protease and ENLYFQG (SEQ ID NO: 2). The elution by a method of cleaving the first linker located between the first moiety and the first target molecule and/or the second linker located between the second moiety of the protein and the second target molecule is useful in that the recovery rate of a target molecule of no interest attached to the first moiety, the second moiety, or the third molecule or the like can be further reduced.

## Specific aspects of first moiety, second moiety, and third molecule

In the screening method according to the present disclosure, examples of the protein that provides the first moiety and the second moiety in the present disclosure include GFP, luciferase, a SNAP tag, and ubiquitin. The terms GFP, luciferase, a SNAP tag, and ubiquitin used herein include their amino acid variants, respectively. Specific examples of the protein include GFP (see, e.g., Cabantous, S. et al. Sci Rep. 2013; 3: 2854. doi: 10.1038/srep02854, Wen-Xue J. et al. Sci Rep. 2016; 6: 20568. doi: 10.1038/srep20568), SNAP tags (see, e.g., Analyst, 2012, 137, 4760-4765; Anal. Chem. 2016, 88, 8166-8171), ubiquitin (see, e.g., PNAS July 3, 2007 104 (27) 11209-11214), and luciferase (see, e.g., luciferase from deep-sea shrimp [Oplophorus gracilirostris], in particular, Nanoluc (registered trademark). See, e.g., US2010/0281552, US2012/0174242, and US2018/0172692, and Andrew, D. et al. Sci Rep. 2017; 7: 8186. doi: 10.1038/s41598-017-07569-y; Anal. Chem. 2018, 90, 3001-3004). Examples of the respective amino acid sequences can include GFP (SEQ ID NO: 71), a SNAP tag (SEQ ID NO: 82), ubiquitin (SEQ ID NO: 75), and Nanoluc (SEQ ID NO: 78), and amino acid variants of these can also be used.

The protein that provides the first moiety and the second moiety in the present disclosure can contain an amino acid sequence selected from the group consisting of (a) to (c):

(a) an amino acid sequence represented by SEQ ID NO: 71, 75, 78, or 82;
(b) the amino acid sequence represented by SEQ ID NO: 71, 75, 78, or 82 in which one or more amino acids are deleted, inserted, substituted, and/or added; and
(c) an amino acid sequence having 80% or more sequence identity to the amino acid sequence represented by SEQ ID NO: 71, 75, 78, or 82.

The protein that provides the first moiety and the second moiety in the present disclosure include wild-type proteins well known to those skilled in the art and proteins in which one or more amino acids are modified.

Other examples of the first moiety and the second moiety in the present disclosure can include moieties obtained by dividing GFP, SNAP tags, ubiquitin, or luciferase (e.g., Nanoluc). The division may produce two or more fragments, such as three or four fragments. When dividing to three fragments, it is preferred that fragments having a small molecular weight among the divided fragments are used as the first moiety and the second moiety in the present disclosure, and examples thereof include GFP10 and GFP11 in GFP and Nanoluc-9 and Nanoluc-10 in Nanoluc. In this case, the

remaining fragment (GFP1-9 in GFP and Nanoluc1-8 in Nanoluc) can be contained in the third molecule in the present disclosure.

It is preferred that ubiquitin in the present disclosure is ubiquitin (I13A) in which the 13th Ile is substituted with Ala.

Specifically, in the case where the protein that provides the first moiety and the second moiety in the present disclosure is, for example, GFP, the moiety consisting of the 192nd to 210th of the amino acid sequence of the full-length GFP (SEQ ID NO: 71) (SEQ ID NO: 73) and the moiety consisting of the 211th to the 231 st of the amino acid sequence of the full-length GFP (SEQ ID NO: 74) can be used as the first moiety and the second moiety, respectively, or the second moiety and the first moiety, respectively. Note that the moiety consisting of the 192nd to 210th of the amino acid sequence of GFP is known as GFP 10, and the moiety consisting of the 211th to the 231st of the amino acid sequence of GFP is known as GFP 11. In one aspect, GFP 10 containing the amino acid sequence of SEQ ID NO: 73 can be used in the screening method according to the present disclosure. In another aspect, GFP 11 containing an amino acid sequence of SEQ ID NO: 74 can be used in the screening method according to the present disclosure. As used herein, fragments of GFP are sometimes referred to as split GFP, and GFP1-9, GFP10, and GFP11 are referred to as splitGFP1-9, splitGFP10, and splitGFP11, respectively.

Also, in the case where the protein that provides the first moiety and the second moiety in the present disclosure is, for example, a SNAP tag, the moiety consisting of the 1st to the 91st of the amino acid sequence of the full-length SNAP tag (SEQ ID NO: 82) (N-terminal fragment of the SNAP tag: SEQ ID NO: 83) and the moiety consisting of the 92nd to the 196th of the amino acid sequence of the full-length SNAP tag (C-terminal fragment of the SNAP tag: SEQ ID NO: 84) can be used as the first moiety and the second moiety, respectively, or the second moiety and the first moiety, respectively. In the present disclosure, the moiety consisting of the 1st to the 91st of the amino acid sequence of the full-length SNAP tag is referred to as SNAP(N), and the moiety consisting of the 92nd to the 196th of the amino acid sequences is also referred to as SNAP(C). In one aspect, SNAP(N) containing the amino acid sequence of SEQ ID NO: 83 can be used in the screening method according to the present disclosure. In another aspect, SNAP(C) containing an amino acid sequence of SEQ ID NO: 84 can be used in the screening method according to the present disclosure. As used herein, fragments of SNAP tags are sometimes referred to as split SNAP.

Also, in the case where the protein that provides the first moiety and the second moiety in the present disclosure is, for example, ubiquitin (I13A), the moiety consisting of the 1st to the 37th of the amino acid sequence of the full-length ubiquitin (SEQ ID NO: 75) (N-terminal fragment of the SNAP tag: SEQ ID NO: 76) and the moiety consisting of the 35th to the 76th of the amino acid sequence of the full-length ubiquitin (C-terminal fragment of the SNAP tag: SEQ ID NO: 77) can be used as the first moiety and the second moiety, respectively, or the second moiety and the first moiety, respectively. In the present disclosure, the moiety consisting of the 1st to the 37th of the amino acid sequence of the full-length ubiquitin is referred to as splitUb(N), and the moiety consisting of the 35th to the 76th of the amino acid sequences is also referred to as splitUb(C). In one aspect, splitUb(N) containing the amino acid sequence of SEQ ID NO: 76 can be used in the screening method according to the present disclosure. In another aspect, splitUb(C) containing the amino acid sequence of SEQ ID NO: 77 can be used in the screening method according to the present disclosure.

In the present disclosure, the protein in which splitUb(N) and splitUb(C) are linked by a linker (GSGS/SEQ ID NO: 90) is sometimes referred to as splitUb(N-C). The ubiquitin in the present disclosure also includes such splitUb(N-C).

As used herein, the fragments of ubiquitin are sometimes referred to as split ubiquitin or splitUb.

Also, in the case where the protein that provides the first moiety and the second moiety in the present disclosure is, for example, Nanoluc that is a type of luciferase, the moiety consisting of the 153rd to the 163rd of the amino acid sequence of the full-length Nanoluc (SEQ ID NO: 78) (SEQ ID NO: 80) and the moiety consisting of the 164th to the 174th of the amino acid sequence of the full-length Nanoluc (SEQ ID NO: 81) can be used as the first moiety and the second moiety, respectively, or the second moiety and the first moiety, respectively. In the present disclosure, the moiety consisting of the 153rd to the 163rd of the amino acid sequence of the full-length Nanoluc is referred to as split Nanoluc-9, and the moiety consisting of the 164th to the 174th of the amino acid sequences is also referred to as split Nanoluc-10. In one aspect, split Nanoluc-9 containing the amino acid sequence of SEQ ID NO: 80 can be used in the screening method according to the present disclosure. In another aspect, split Nanoluc-10 containing the amino acid sequence of SEQ ID NO: 81 can be used in the screening method according to the present disclosure. As used herein, fragments of Nanoluc are sometimes referred to as split Nanoluc, and Nanoluc1-8, Nanoluc-9, and Nanoluc-10 are referred to as splitNanoluc1-8, splitNanoluc-9, and splitNanoluc-10, respectively.

In the case where the protein that provides the first moiety and the second moiety in the present disclosure is, for example, GFP, examples of the third molecule in the present disclosure include a molecule containing GFP1-9 (SEQ ID NO: 72) having the 1st to the 191st amino acid sequence of the full-length GFP, and more specifically a molecule having an amino acid sequence of SEQ ID NO: 48. When the protein is a SNAP tag, examples include SNAP-biotin. Also, when the protein is ubiquitin (I13A), examples include an anti-ubiquitin antibody. SNAP-biotin and the anti-ubiquitin antibody used may be those well known to the person skilled in the art, but preferred examples include anti-ubiquitin antibodies that can bind to full-length ubiquitin but not to its fragment split ubiquitin. The anti-ubiquitin antibody in the present disclosure is more preferably an antibody that does not recognize splitUb(N) or splitUb(C) when they are not in

proximity or association, but specifically recognizes splitUb(N) and splitUb(C) when they are in proximity or association. Examples of the anti-ubiquitin antibody include a Mouse IgG1 monoclonal antibody (clone: FK2, [MEDICAL & BIOLOGICAL LABORATORIES CO., LTD.]) and a Mouse IgG1 monoclonal antibody (clone: 1B3, [MEDICAL & BIOLOGICAL LABORATORIES CO., LTD.]). Also, when the protein is Nanoluc, examples of the third molecule include molecules including split Nanoluc 1-8 (SEQ ID NO: 79) having the 1st to the 152nd amino acid sequence of the full-length Nanoluc, more specifically, molecules having an amino acid sequence of SEQ ID NO: 85.

In the screening method according to the present disclosure, when the protein that provides the first moiety and the second moiety is, for example, GFP, a complex of biotinylated GFP1-9, the first target molecule linked to GFP10, the second target molecule linked to GFP11, and the candidate molecule can be recovered by utilizing interactions between streptavidin and biotin. Also, when the protein is Nanoluc, for example, a complex of biotinylated splitNanoluc1-8, the first target molecule linked to splitNanoluc-9, the second target molecule linked to splitNanoluc-10, and the candidate molecule can be recovered by utilizing interactions between streptavidin and biotin. When a SNAP tag is bound to SNAP-biotin which is a substrate, a biotinylated SNAP tag is generated by an enzymatic reaction. Thus, when the SNAP tag is used as the protein in the present disclosure, a complex of the biotinylated SNAP tag, the first target molecule, the second target molecule, and the candidate molecule can be recovered by utilizing interactions between the biotin bound to the SNAP tag and streptavidin. The third molecule in the present disclosure may contain a tag sequence for biotinylation (e.g., an Avi tag sequence (SEQ ID NO: 1)), a cleavage sequence for elution (e.g., a cleavage sequence by TEV protease), a tag sequence for purification (e.g., a His tag sequence), and/or other sequences.

In one aspect, in the screening method according to the present disclosure, a complex including the first target molecule, the second target molecule, and the candidate molecule can be recovered using the third molecule itself. For example, when ubiquitin is used as the protein in the present disclosure, a complex containing the first target molecule linked to splitUb(N), the second target molecule linked to splitUb(C), and the candidate molecule can also be recovered based on the affinity between an anti-ubiquitin antibody that is the third molecule and ubiquitin.

The protein that provides the first moiety and the second moiety, the first moiety, the second moiety, and the third molecule in the present disclosure includes, in addition to those that have the amino acid sequence described above, those that have amino acid sequences in which modifications are added to the amino acid sequence described above.

Specifically, the protein that provides the first moiety and the second moiety in the present disclosure includes, in addition to the proteins containing an amino acid sequence represented by SEQ ID NO: 71, 75, 78, or 82,

(1) a protein containing the amino acid sequence represented by SEQ ID NO: 71, 75, 78, or 82 in which one or more amino acids are deleted, inserted, substituted, and/or added, wherein the protein is functionally equivalent to a protein containing the amino acid sequence represented by SEQ ID NO: 71, 75, 78, or 82, respectively; and
(2) a protein containing an amino acid sequence having a sequence identity of 80% or more, 85% or more, 90% or more, 95% or more, or 98% or more to the amino acid sequence represented by SEQ ID NO: 71, 75, 78, or 82, where the protein is functionally equivalent to a protein containing an amino acid sequence represented by SEQ ID NO: 71, 75, 78, or 82, respectively.

Also, the first moiety and the second moiety in the present disclosure include, in addition to proteins containing an amino acid sequence represented by SEQ ID NO: 73, 74, 76, 77, 80, 81, 83, or 84,

(1) a protein containing the amino acid sequence represented by SEQ ID NO: 73, 74, 76, 77, 80, 81, 83, or 84 in which one or more amino acids are deleted, inserted, substituted, and/or added, wherein the protein is functionally equivalent to a protein containing the amino acid sequence represented by SEQ ID NO: 73, 74, 76, 77, 80, 81, 83, or 84, respectively; and
(2) a protein containing an amino acid sequence having a sequence identity of 80% or more, 85% or more, 90% or more, 95% or more, or 98% or more to the amino acid sequence represented by SEQ ID NO: 73, 74, 76, 77, 80, 81, 83, or 84, where the protein is functionally equivalent to a protein containing an amino acid sequence represented by SEQ ID NO: 73, 74, 76, 77, 80, 81, 83, or 84, respectively.

Examples of the third molecule in the present disclosure include a protein containing the amino acid sequence represented by the above SEQ ID NO: 72 or 79, but as described above, the protein may contain an Avi tag sequence for biotinylation utilized for pull-down, a cleavage sequence by TEV protease utilized for elution, a His tag for purification, and/or other sequences. That is, in one aspect, as long as the third molecule in the present disclosure contains the amino acid sequence represented by SEQ ID NO: 72 or 79, any sequence may be added to the amino acid sequence as long as the screening method according to the present disclosure can be performed. For example, in one of Examples here, GFP1-9 (SEQ ID NO: 48) containing an Avi tag (SEQ ID NO: 1), a TEV protease cleavage sequence (SEQ ID NO: 2), a His tag sequence (SEQ ID NO: 3), and GFP1-9 (SEQ ID NO: 72) is used as GFP1-9.

The third molecule in the present disclosure also includes:

(1) a protein containing the amino acid sequence represented by SEQ ID NO: 48, 72, or 79 in which one or more amino acids are deleted, inserted, substituted, and/or added, wherein the protein is functionally equivalent to a protein containing the amino acid sequence represented by SEQ ID NO: 48, 72, or 79, respectively; and
(2) a protein containing an amino acid sequence having a sequence identity of 80% or more, 85% or more, 90% or more, 95% or more, or 98% or more to the amino acid sequence represented by SEQ ID NO: 48, 72, or 79, where the protein is functionally equivalent to a protein containing an amino acid sequence represented by SEQ ID NO: 48, 72, or 79, respectively.

The addition, deletion, substitution, and/or insertion of amino acids can be performed by methods known in the art. For example, a nucleic acid encoding an amino acid sequence may be subjected to a site-directed mutagenesis method (Kunkel et al., Proc. Natl. Acad. Sci. USA 82, 488-492 (1985)), overlap extension PCR, or the like. These methods may be performed, as appropriate, singly or in combination.
In general, it is known that modifications (e.g., conservative substitutions, deletions, insertions, and/or additions) of one or more (e.g., 2, 3, 4, 5, 6, 7, 8, 9, 10, or more than 10) amino acids in a protein do not affect the function of the peptide or even enhance the function of the original protein. Amino acids are classified into hydrophobic amino acids (A, I, L, M, F, P, W, Y, V) and hydrophilic amino acids (R, D, N, C, E, Q, G, H, K, S, T) according to the characteristics of their side chains. Side chains of amino acids can also be classified into aliphatic side chains (G, A, V, L, I, P), side chains containing a hydroxyl group (S, T, Y), side chains containing a sulfur atom (C, M), side chains containing a carboxylic acid and an amide (D, N, E, Q), side chains containing a base (R, K, H), and side chains containing an aromatic (H, F, Y, W). Proteins in which amino acids contained in a protein containing an amino acid sequence represented by any one of SEQ ID NOs: 71 to 84 are modified with other amino acids classified into groups having the same characteristics are also included in the protein that provides the first moiety and the second moiety, the first moiety, the second moiety, and the third molecule in the present disclosure. However, the protein that provides the first moiety and the second moiety, the first moiety, the second moiety, and the third molecule in the present disclosure may also have non-conservative modifications as long as they are functionally equivalent to a protein containing an amino acid sequence represented by any one of SEQ ID NOs: 48, 71 to 84.
In one aspect of the present invention, proteins containing an amino acid sequence having a high sequence identity to the amino acid sequence represented by any one of SEQ ID NOs: 48, 71 to 84 are also encompassed in the protein that provides the first moiety and the second moiety, the first moiety, the second moiety, and the third molecule in the present disclosure. In the present disclosure, the "high identity" refers to a sequence identity of at least 50% or more, more preferably 70% or more, 75% or more, 80% or more, 85% or more, and even preferably 90% or more (e.g., 91%, 92%, 93%, 94%, 95%, 96%, 97%, 98%, 99% or more) in the entire amino acid sequence or the entire nucleotide sequence. The sequence identity was determined by the algorithm BLAST by Carlin and Arthur (Proc. Natl. Acad. Sci. USA 87:2264-2268, 1990, Proc Natl Acad Sci USA 90: 5873, 1993). Programs called BLASTN and BLASTX have been developed based on the algorithm BLAST (Altschul SF, et al: J Mol Biol 215: 403, 1990). In the case of analyzing nucleotide sequences using BLASTN, the parameters are set to, for example, score = 100 and wordlength = 12. In the case of analyzing amino acid sequences using BLASTX, the parameters are set to, for example, score = 50 and wordlength = 3. In the case of using BLAST and Gapped BLAST programs, default parameters for each program are used. Specific procedures for these analytical methods are known.
As used herein, the "functionally equivalent" means that a functions of the protein that provides the first moiety and the second moiety, the first moiety, the second moiety, and the third molecule in the present disclosure before the modification of an amino acid are retained. Specifically, as long as the third molecule can detect that the first moiety and the second moiety in the present disclosure are in proximity or association, they are determined to be functionally equivalent before and after the amino acid modification. Meanwhile, when the third molecule cannot be detected that the first moiety and the second moiety in the present disclosure are in proximity or association due to the modification of an amino acid, they are determined to be not functionally equivalent before and after the amino acid modification. For example, GFP can be used in the screening method of the present disclosure as long as the properties of associating with GFP1-9 when GFP 10 and GFP 11 are in proximity are retained, and thus even if the amino acid has been modified, they are functionally equivalent as long as the properties are retained. The same holds true for luciferase (e.g., Nanoluc), SNAP tags, and ubiquitin.
The protein that provides the first moiety and the second moiety, the first moiety, the second moiety, and the third molecule in the present disclosure having modifications in the amino acid sequence can be prepared using nucleic acids encoding the protein. Examples of such methods for preparing nucleic acids include the site-directed mutagenesis method (Kramer, W. and Fritz, H. -J. (1987) Oligonucleotide-directed construction of mutagenesis via gapped duplex DNA. Methods in Enzymology, 154: 350-367). Alternatively, hybridization techniques (Southern, E.M. (1975) Journal of Molecular Biology, 98, 503) can also be used to obtain nucleic acids encoding proteins that are functionally equivalent

to the proteins described in any one of SEQ ID NOs: 48, 71 to 84. That is, the modified nucleic acids encoding the protein that provides the first moiety and the second moiety, the first moiety, the second moiety, and the third moiety in the present disclosure may be those that hybridize under stringent conditions with a nucleic acid encoding an amino acid sequence represented by any one of SEQ ID NOs: 48, 71 to 84. Stringent hybridization conditions can be appropriately set by those skilled in the art. As an example, the pre-hybridization is performed in a hybridization solution containing 25% formamide, or 50% formamide under more stringent conditions, 4 × SSC, 50 mM Hepes pH 7.0, 10 × Denhalt solution, and 20 μg/ml modified salmon sperm DNA, at 42°C overnight, and then the hybridization is performed by adding a labeled probe and incubating at 42°C overnight. Subsequent washes can be performed in the wash solution and temperature conditions, for example, about "2 × SSC, 0.1% SDS, 50°C", "2 × SSC, 0.1% SDS, 42°C", "1 × SSC, 0.1% SDS, 37°C", more stringent conditions at about "2 × SSC, 0.1% SDS, 65°C", "0.5 × SSC, 0.1% SDS, 42°C", or furthermore stringent conditions at "0.2 × SSC, 0.1% SDS, 65°C". As the more stringent the conditions for hybridization are taken in this manner, the isolation of a nucleic acid having a higher homology with a nucleic acid sequence encoding an amino acid sequence of any one of SEQ ID NOs: 48, 71 to 84 can be expected. However, the combination of SSC, SDS, and temperature conditions described above is only an example, and those skilled in the art can achieve the same stringency as described above by appropriately combining the above or other elements (e.g., probe concentration, probe length, or hybridization reaction time) that determine the stringency of hybridization.

It is believed that the nucleic acid isolated thereby has high homology with a protein having an amino acid sequence of any one of SEQ ID NOs: 48, 71 to 84 in the amino acid sequence level. It is also believed that the nucleic acid has high homology with a nucleotide sequence encoding an amino acid sequence of any one of SEQ ID NOs: 48, 71 to 84 in the nucleotide sequence level. The "high homology" refers to the sequence identity of at least 50%, preferably 70%, or 75%, further preferably 80%, or 85%, particularly preferably 90%, (e.g., 91%, 92%, 93%, 94%, 95%, 96%, 97%, 98%, 99% or more) of the entire amino acid sequence or the entire nucleotide sequence, as described above.

The nucleic acids in the present disclosure can be used, for example, in the preparation of the protein that provides the first moiety and the second moiety, the first moiety, the second moiety, and the third molecule in the present disclosure, and amino acid variants of these. When preparing proteins that provide these first and second moieties, the first moiety, the second moiety, and the third molecule, and amino acid variants of these, the nucleic acid encoding these are typically inserted into an appropriate expression vector, the vector is introduced into an appropriate cell, and the transformed cell is cultured, and the protein expressed and modified is isolated, purified, and cultured. Such proteins can also be expressed as fusion proteins with other proteins, for example, to facilitate purification. Examples of the preparation method include a method for preparing a fusion protein with a maltose binding protein using E. coli as the host (vector pMAL series released by New England Biolabs, Inc., USA), a method for preparing a fusion protein with a glutathione-S-transferase (GST) (vector pGEX series released by Amersham Pharmacia Biotech, Inc.), and a method for preparing by adding a histidine tag (e.g., SEQ ID NO: 3) (pET series released by Novagen, USA). As the host cell, there is no particular limitation as long as the cell is suitable for expression of a recombinant protein, and in addition to the E. coli described above, it is possible to use, for example, yeast, various animal and plant cells, insect cells, and the like. For the introduction of vectors into host cells, various methods known to those skilled in the art can be used. For example, an introduction method utilizing calcium ions (Mandel, M., Higa, A. (1970) Journal of Molecular Biology, 53, 158-162, Hanahan, D. (1983) Journal of Molecular Biology, 166, 557-580) can be used for introduction into E. coli. A protein expressed in a host cell can be purified and recovered from the host cell or a cell culture or culture supernatant thereof by a method known to those skilled in the art. When the protein is expressed as a fusion protein with the maltose binding protein or the like described above, affinity purification can be easily performed.

In one aspect of the present disclosure, the nucleic acid may be inserted into a vector. When E. coli is used as the host, examples of the vector include, but are not limited to, a vector having an "ori" for being amplified by E. coli to be amplified in E. coli (e.g., JM109, DH5α, HB101, XL1 Blue) and prepared in large amounts, and further having a drug-resistant gene that can be distinguished by an agent (e.g., ampicillin, tetracycline, kanamycin, chloramphenicol, and the like). Examples of such vectors include M13-based vectors, pUC-based vectors, pBR322, pBluescript, and pCR-Script. Further, when subcloning or excising cDNA is intended, pGEM-T, pDIRECT, pT7, or the like, in addition to the above vectors, can be mentioned. When vectors are used for producing proteins, expression vectors are particularly useful. When used for expression, for example, in Escherichia coli, expression vectors are required to have the above features such that the vector is amplified in Escherichia coli, and when the host is E. coli, such as JM109, DH5α, HB101, XL1-Blue, or the like, to contain a promoter that can be efficiently expressed in E. coli, such as a lacZ promoter (Ward et al., Nature (1989) 341, 544-546; FASEB J. (1992) 6, 2422-2427), an araB promoter (Better et al., Science (1988) 240, 1041-1043), or a T7 promoter. Examples of such vectors include pGEX-SX-1 (manufactured by Pharmacia Corporation), "QIAexpress system" (manufactured by QIAGEN N.V.), pEGFP, or pET.

The vector may further contain a signal sequence for polypeptide secretion. As the signal sequence for polypeptide secretion, when the polypeptide is produced in the periplasm of E. coli, the pelB signal sequence (Lei, S. P. et al J. Bacteriol. (1987) 169, 4379) may be used. The introduction of the vector into the host cell can be performed, for example, by a calcium chloride method and an electroporation method. Examples of the vector expressed in plants include vectors

such as pMH1, pMH2, and pCAMBIA.

In addition to E. coli, examples of the vectors for producing proteins include expression vectors from mammals (e.g., pcDNA3 (manufactured by Invitrogen) and pEGF-BOS (Nucleic Acids. Res.1990, 18(17), p5322), pEF, pCDM8), expression vectors from insect cells (e.g., "Bac-to-BAC baculovairus expression system" (manufactured by Gibco BRL), pBacPAK8), expression vectors from plants (e.g., pMH1, pMH2), expression vectors from animal viruses (e.g., pHSV, pMV, pAdexLcw), expression vectors from retroviruses (e.g., pZIPneo), expression vectors from yeast (e.g., "Pichia Expression Kit" (manufactured by Invitrogen), pNV11, SP-Q01), and expression vectors from Bacillus subtilis (e.g., pPL608, pKTH50).

When intended to be expressed in an animal cell such as a CHO cell, a COS cell, or an NIH3T3 cell, expression vectors are required to have a promoter necessary for expression in the cell, such as an SV40 promoter (Mulligan et al. Nature (1979) 277, 108), an MMLV-LTR promoter, an EF1$\alpha$ promoter (Mizushima et al., Nucleic Acids Res. (1990) 18, 5322), a CMV promoter, and the like, and further preferably to have a gene for selecting the cell transformation (e.g., a drug resistance gene to be identified by a drug (neomycin, G418, etc.). Examples of the vectors having such characteristics include pMAM, pDR2, pBK-RSV, pBK-CMV, pOPRSV, and pOP13.

The transformed cells can be used, for example, as a production system for protein production or expression. The production systems for protein production include in vitro and in vivo production systems.

When eukaryotic cells are used, examples of the host can include animal cells, plant cells, and fungal cells. Examples of animal cells include mammalian cells (e.g., cells such as 3T3, myeloma cells, BHK (baby hamster kidney), HeLa, Vero, in addition to the CHO cells, COS cells, and NIH3T3 cells described above), amphibian cells (e.g., Xenopus oocytes (Valle, et al., Nature (1981) 291, 358-340), and insect cells (e.g., cells such as sf9, sf21, Tn5). As CHO cells, in particular, dhfr-CHO that is a CHO cell deficient in the DHFR gene (Proc. Natl. Acad. Sci. USA (1980) 77, 4216-4220) and CHO K-1 (Proc. Natl. Acad. Sci. USA (1968) 60, 1275) can be suitably used. CHO cells are particularly preferred when expression in large amounts is intended.

As plant cells, in addition to the plant-derived cells described below, cells from Nicotiana tabacum are known as protein production systems and may be callus cultured for production.

Examples of the fungal cell include, but are not limited to, a yeast, such as Saccharomyces, e.g., Saccharomyces cerevisiae, and a filamentous fungus, such as Aspergillus, e.g., Aspergillus niger.

In a non-limiting aspect, the present disclosure relates to a method for producing the candidate molecule obtained by the screening method according to the present disclosure. For example, when the candidate molecule is a peptide, the peptide can be produced based on the amino acid sequence of the peptide.

As used herein, "1E + N" means a 1 $\times$ 10 to the Nth power. For example, 1E + 8 means 1 $\times$ 10 to the eighth power, and 1E + 6 means 1 $\times$ 10 to the sixth power. Likewise, 1.8E + 12 means 1.8 $\times$ 10 to the 12th power, and 3.03E + 05 means 3.03 $\times$ 10 to the 5th power.

Herein, the meaning of the term "and/or" includes any combination in which "and" and "or" are appropriately combined. Specifically, for example, "A, B, and/or C" includes the following seven variations; (i) A, (ii) B, (iii) C, (iv) A and B, (v) A and C, (vi) B and C, (vii) A, B, and C.

In the present specification, the term "to" that indicates a range includes values of both ends thereof. For example, "A to B" means the range of A or more and B or less.

All references cited herein are incorporated herein by reference.

## Examples

The present invention will be further illustrated with reference to Examples given below, but is not limited by Examples below.

In Examples, the following abbreviations were used.

DMSO: dimethyl sulfoxide
DTT: dithiothreitol
FA: formic acid
TFA: trifluoroacetic acid
THF: tetrahydrofuran
TCEP: tris(2-carboxyethyl)phosphine
TEA: triethylamine
HFIP: hexafluoroisopropanol

Example 1: Preparation of biotinylated GFP1-9 protein

Vector Preparation

Expression vectors were prepared by inserting a synthetic DNA of SEQ ID NO: 15 (D-1) into pET-15b vectors using a Nde1/BamH1 restriction enzyme.

Synthetic DNA SEQ ID NO: 15 (D-1)

DNA sequence:

CATATGGGTGGCACCAGCAGCAAGGGCGAGGAACTGTTCACCGGTGTGGTTCCGAT
CCTGGTGGAGCTGGACGGCGATGTTAACGGTCACAAATTTAGCGTGCGTGGTGAGG
GCGAAGGTGACGCGACCATCGGCAAGCTGACCCTGAAATTCATTAGCACCACCGGT
AAACTGCCGGTGCCGTGGCCGACCCTGGTTACCACCCTGACCTACGGTGTGCAGGCG
TTTAGCCGTTATCCGGACCACATGAAGCGTCACGATTTCTTTAAAAGCGCGATGCCG
GAGGGCTACGTTCAAGAACGTACCATTAGCTTCAAGGACGATGGCAAGTATAAAAC
CCGTGCGGTGGTTAAATTTGAGGGCGATACCCTGGTTAACCGTATCGAGCTGAAGG
GTACCGACTTCAAAGAAGATGGCAACATTCTGGGTCACAAGCTGGAATACAACTTT
AACAGCCACAACGTGTATATCACCGCGGACAAGCAGAAAAACGGCATTAAAGCGA
ACTTCACCGTGCGTCACAACGTTGAGGACGGTAGCGTTCAACTGGCGGATCACTACC
AGCAAAACACCCCGATTGGTGATGGTACCCGTGGCAGCGGTAGCATTGAAGGTCGT
CATAGCGGCAGCGGTAGCCCGGTGCTGCTGCCGGACAACCACTATCTGAGCACCCA
GACCGTTCTGAGCAAAGATCCGAACGAGGCGGGCACTCGTGGCAGCGGTAGCATCG
AAGGTCGTCACAGCGGCAGCGGTAGCCGTGACCACATGGTGCTGCACGAATACGTT
AACGCGGCGGGCATCACCCACGGTATGGACGAACTGTATAAAGGCAGCGGCGGCAC
CTAAGGATCC

An insert tag sequence (SEQ ID NO: 16) was inserted at the N-terminus of a protein of interest using a Nco1/Nde1 restriction enzyme to add an Avi tag sequence for biotinylation (SEQ ID NO: 1), a TEV protease recognition sequence for cleavage (SEQ ID NO: 2), and a His tag for purification (SEQ ID NO: 3) to the protein. Using the ligation product, Escherichia coli DH5$\alpha$ competent cells (TOYOBO CO., LTD., #DNA-903) were transformed. After cloning, the insertion sites were subjected to sequence analysis to confirm the completion of a tagged expression vector.

Further, to increase the expression efficiency and the efficiency of His tag purification, a sequence Gly-Gly-Ser-Ser (SEQ ID NO: 88) was inserted in the sequence between the His tag and the ORF sequence by PCR. Specifically, 1 $\times$ Prime STAR Max Premix (Takara Bio Inc.), 2 $\mu$M Fw-primer (SEQ ID NO: 13), 2 $\mu$M Rv-primer (SEQ ID NO: 14), and 1.4 ng of the tagged expression vector were mixed, and cycles of 98°C 10 seconds, 55°C 15 seconds, and 72°C 45 seconds were repeated for 30 cycles. Using the PCR product, Escherichia coli DH5$\alpha$ competent cells (TOYOBO CO., LTD., #DNA-903) were transformed. After cloning, the insertion sites were subjected to sequence analysis to confirm the completion of a GFP1-9 expression vector used in this Example.

Protein Expression and Purification

E. coli BL21 (DE3) competent cells (Novagen) were transformed with the GFP1-9 expression vector and a BirA expression vector, in which an E. coli BirA gene was introduced into a pACYC184 vector, to construct an expression strain.

The constructed E. coli expression strain was inoculated into medium A (10 g/L Tryptone, 5 g/L Yeast Extract, 10 g/L NaCl, 100 $\mu$g/mL ampicillin, 34 $\mu$g/mL chloramphenicol), and the medium was cultured at 37°C, 200 rpm. When

OD600 = 0.7 was reached, the flask was immersed in ice water for 5 minutes to reduce the temperature of the culture solution to 4°C. Isopropyl-β-thiogalactopyranoside (IPTG) was added at 0.5 mM and biotin was added at 50 μM, then the resulted solution was cultured at 19°C, 200 rpm overnight to express a biotinylated modified protein of interest. The cultured bacteria were recovered by centrifugation, frozen in liquid nitrogen, and then stored at -80°C.

The frozen bacteria were suspended using an extraction buffer (50 mM Tris pH 8.0, 150 mM NaCl, 1% CHAPS, 5% Glycerol, Proteinase Inhibitor Cocktail (EDTA-free)), and rLysozyme (Novagen, #71110-3) was added at 5 kUnit/E. coli, and then the resultant solution was left at room temperature for 20 minutes. After resuspension and ultrasonic crushing, 1M $MgCl_2$ was added at 0.2% v/v, and 25 U/μL benzonase (EMD millipore, #70746-2.5KUN) was added at 0.03% v/v, and then the resultant was allowed to stand still again at room temperature for 20 minutes. The sample was centrifuged, and the supernatant was collected and filtered through a 0.22 μm filter.

The filtered sample was purified using HisTrap HP 5 mL (GE Healthcare, #17-5248-02). To cleave the GFP10 and GFP11 moieties, $CaCl_2$ was added to the eluted sample at a final concentration of 2 mM, and Factor Xa Protease (New England Biolabs, #P8010L) was added at the amount of 1/100 (w/w) of the sample. Samples were buffer-exchanged for dialysis buffer (50 mM Tris-HCl (pH 8.0), 150 mM NaCl, 2 mM CaCl2) using a dialysis cassette: Slide-A-Lyzer 10K Dialysis Cassettes G2 30 mL Capacity (Thermo Fisher, #87732).

The dialyzed samples were collected and purified with Streptavidin Mutein Matrix (Roche, #03708152001). Fractions containing proteins of interest were collected, and a total 10 mL of the fractions was concentrated with Amicon Ultra-15 10K (MERCK, #UFC901024) to a volume of 3 mL. An equal amount of 6 M Gdn Buffer (50 mM Tris-HCl, 150 mM NaCl, 10% Glycerol, 3 M Guanidine-Hydrochloride, pH 8.0) was added, and the proteins were allowed to denature on ice for 40 minutes. Changes in the absorption wavelength spectrum before and after denaturation were checked, and denaturation was confirmed, then gel filtration purification was performed with HiLoad 26/600 Superdex 75 pg (GE Healthcare, #28-9893-34). As a running buffer for gel filtration purification, 50 mM Tris-HCl, 150 mM NaCl, 10% Glycerol, 3 M Guanidine Hydrochloride (pH 8.0), 1 mM DTT was used. Finally, the obtained sample was dialyzed with a dialysis cassette: Slide-A-Lyzer 10K Dialysis Cassette 30 mL capacity (Thermo Fisher, #87732) to remove the guanidine denaturant and allow the sample to be refolded.

The results of SDS-PAGE analysis showed a single band at the position corresponding to the theoretical molecular weight of the protein of interest. The biotinylated protein of interest was also confirmed by a gel shift assay utilizing the interaction between the biotinylated protein and streptavidin. The result of SEC analysis confirmed that the main component of the protein of interest had a retention time equivalent to a molecular weight marker of 32 kDa and was present as a monomer. Table 1 shows the lane information of SDS-PAGE, Fig. 1 shows the result of SDS-PAGE, and Fig. 2 shows the result of SEC analysis. AdvanceBio SEC300A 2.7 μm 4.6 × 150 mm (Agilent Technologies, #PL1580-3301) was used as the column for SEC analysis.

[Table 1]

| Lane | Sample name | Additive | Protein amount (μg) |
|---|---|---|---|
| 1 | Marker | - | - |
| 2 | GFP1-9 | Streptavidin | 1 |
| 3 | GFP1-9 | - | 1 |
| 4 | GFP1-9 | - | 0.1 |
| 5 | - | Streptavidin | - |

Example 2: Complementation assay of splitGFP using mRNA display

Screening a library for a candidate molecule capable of forming a complex with a plurality of target molecules requires a system that suppresses the recovery rate of a test molecule that forms a complex with only one target molecule.

Thus, each binding ability of GFP10 (SEQ ID NO: 40), GFP11 (SEQ ID NO: 41), and a GFP10-11 fusion peptide (SEQ ID NO: 39) to GFP1-9 (SEQ ID NO: 48) was evaluated. Nucleic acid-linked Display molecules were prepared for each of GFP10, GFP11, and the GFP10-11 fusion peptide, and they were incubated with GFP1-9, and the nucleic acid moiety of the molecule bound to GFP1-9 was subjected to qPCR. In Examples, the 1st to the 9th strand of GFP to which an Avi tag sequence, a TEV protease recognition sequence, and a His tag sequence are added at the N-terminus are sometimes denoted as GFP1-9; the tenth strand of GFP to which a GS linker is added at the C-terminus is sometimes denoted as GFP10; the eleventh strand of GFP to which a GS linker is added at the C-terminus is sometimes denoted as GFP 11; and a protein in which GFP9 and GFP10 are liked and a GS linker is added at the C-terminus is sometimes denoted as GFP10-11.

(1) Synthesis of mRNA

mRNAs (SEQ ID NO: 29 (mR-1) to SEQ ID NO: 31 (mR-3)) were synthesized from template DNAs (SEQ ID NO: 17 (D-3)-SEQ ID NO: 19 (D-5)) by in vitro transcription reaction using T7 RNA polymerase and purified by RNeasy kit (QIAGEN N.V.).

Template DNA SEQ ID NO: 17 (D-3)

DNA sequence: GFP10-11

GGCGTAATACGACTCACTATAGGGTTAACTTTAAGAAGGAGATATACATATGGACC
TGCCGGACGACCACTACCTGTCTACCCAGACCATCCTGCTGAAAGACCTGAACGAA
AAACGTGACCACATGGTTCTGCTGGAATACGTTACCGCGGCGGGTATCACCGACGC
GTCTGGCTCTGGCTCTGGCTCTGGCTCTAAAAAAA

Template DNA SEQ ID NO: 18 (D-4)

DNA sequence: GFP10

GGCGTAATACGACTCACTATAGGGTTAACTTTAAGAAGGAGATATACATATGGACC
TGCCGGACGACCACTACCTGTCTACCCAGACCATCCTGCTGAAAGACCTGAACGGCT
CTGGCTCTGGCTCTGGCTCTAAAAAAA

Template DNA SEQ ID NO: 19 (D-5)

DNA sequence: GFP11

GGCGTAATACGACTCACTATAGGGTTAACTTTAAGAAGGAGATATACATATGGAAA
AACGTGATCATATGGTGCTGCTGGAATATGTGACCGCGGCGGGCATTACCGATGCGT
CTGGCTCTGGCTCTGGCTCTGGCTCTAAAAAAA

Template mRNA SEQ ID NO: 29 (mR-1)

GFP10-11 RNA sequence:

GGGUUAACUUUAAGAAGGAGAUAUACAUAUGGACCUGCCGGACGACCACUACCU
GUCUACCCAGACCAUCCUGCUGAAAGACCUGAACGAAAAACGUGACCACAUGGUU
CUGCUGGAAUACGUUACCGCGGCGGGUAUCACCGACGCGUCUGGCUCUGGCUCUG
GCUCUGGCUCUAAAAAAA

mRNA SEQ ID NO: 30 (mR-2)

GFP10 RNA sequence:

GGGUUAACUUUAAGAAGGAGAUAUACAUAUGGACCUGCCGGACGACCACUACCUGUCUACCCAGACCAUCCUGCUGAAAGACCUGAACGGCUCUGGCUCUGGCUCUGGCUCUAAAAAAA

mRNA SEQ ID NO: 31 (mR-3)

GFP11 RNA sequence:

GGGUUAACUUUAAGAAGGAGAUAUACAUAUGGAAAAACGUGAUCAUAUGGUGCUGCUGGAAUAUGUGACCGCGGCGGGCAUUACCGAUGCGUCUGGCUCUGGCUCUGGCUCUGGCUCUAAAAAAA

(2) Complementation assay by mRNA Display

Display molecules in which mRNA and a peptide were linked were prepared, and the recovery rate of the Display molecule by biotinylated GFP1-9 was examined. In accordance with the method described in WO2013/100132, puromycin was linked via a linker to the 3' end of the mRNA purified above and then translated to prepare the Display molecule of mRNA-peptide. PUREfrex2.0 (GeneFrontier Corporation), a reconstituted cell-free protein synthesis system from a prokaryotic organism, was used as the translation system, and the translation was performed according to the attached protocol.

To examine the display efficiency of each Display molecule, the RNA portion was digested by RNaseONE (Promega Corporation) and electrophoretically separated with TricineP AGE. When the Display molecule is formed, a fusion molecule of the translated peptide and a puromycin linker is present. When the Display molecule is not formed, an unbound linker alone without binding of the translated peptide is present and detected at a position lower than the former. Thus, a total of two bands are detected. From the band ratio, the display efficiency (%) of the Display molecule was calculated by the following formula.

Display molecule display efficiency (%) = band intensity of translated peptide and linker/total band intensity × 100

For the specific reaction solution with RNaseONE, 1 × Reaction buffer, 0.84 μM fusion molecule, and 0.5 U/μL RNaseONE were mixed, and the mixture was allowed to react at 37°C for 1 hour.

Detection by TricinePAGE resulted in a detection of a fluorescently labeled puromycin linker. The display efficiency obtained from the result of TricinePAGE was as shown in Table 2.

[Table 2]

| SEQ ID NO | Peptide name | Amino acid sequence | Display efficiency |
|---|---|---|---|
| SEQ ID NO: 39 | GFP10-11 | MDLPDDHYLSTQTILLKDLNEKRDHMVLLEYVTAAGITDASGSGSGSGSKK | 56% |
| SEQ ID NO: 40 | GFP 10 | MDLPDDHYLSTQTILLKDLNGSGSGSGSKK | 84% |

(continued)

| SEQ ID NO | Peptide name | Amino acid sequence | Display efficiency |
|---|---|---|---|
| SEQ ID NO: 41 | GFP 11 | MEKRDHMVLLEYVTAAGITDASGSGSGSGSKK | 92% |

For complementation assays, non-fluorescently labeled puromycin linkers were used, and the translation products were reverse transcribed to avoid binding at the mRNA portion. Specifically, 1 × M-MLV RT Reaction buffer (Promega Corporation, #368B), 0.5 mM dNTP, 8 U/μL M-MLV RT (H-) Point Mutant (Promega Corporation, #368B), 3 μM Rv-primer (SEQ ID NO: 5), and 0.5 μM translation product were mixed, and the mixture was allowed to react at 42°C for 1 hour.

In 1 × TBS buffer, 0.42 μM of each Display molecule was mixed with 1 μM of biotinylated GFP1-9, and the mixture was allowed to react at room temperature for 1 hour.

The biotinylated GFP1-9 or a complex containing the same was recovered by pull-down with Streptavidin-coated magnetic beads, washed several times with tTBS (NACALAI TESQUE, INC.), and then cleaved with a TEV protease that recognizes a TEV protease recognition sequence introduced between GFP1-9 and biotin to elute the nucleic acid. Specifically, 1 × TEV buffer and 1 mM DTT, 0.2 U/μL AcTEV protease (Thermo Fisher Scientific, Inc., #12575015) were added as a TEV eluate to the washed beads and reacted. After the reaction, the supernatant was collected and subjected to qPCR with a complementary primer (Fw-primer (SEQ ID NO: 4), Rv-primer (SEQ ID NO: 5)). For the qPCR reaction solution, 1 × Extaq buffer, 0.2 mM dNTP, 0.5 μM Fw-primer, 0.5 μM Rv-primer, 200,000 times dilution SYBR Green I (Lonza K.K., #50513), and 0.02 U/μL Ex Taq polymerase (Takara Bio Inc.) were mixed, and PCR was performed by heating at 95°C for 2 minutes, and then repeating the cycles of 95°C for 10 seconds, 57°C for 20 seconds, and 72°C for 30 seconds for 40 cycles. Each Display molecule before reacting with biotinylated GFP1-9 was also subjected to qPCR in the same way, and the input nucleic acid amount (number of molecules) was calculated. In the Tables, units of nucleic acid amount (number of molecules) were referred to as "molecules".

To draw a calibration curve for each sequence, dilutions were made to be 1E + 8/μL, 1E + 7/μL, 1E + 6/μL, 1E + 5/μL, and 1E + 4/μL in nucleic acid amount (number of molecules), and subjected to qPCR under the same conditions.

The apparent recovery rate obtained by dividing the amount of nucleic acid of each Display molecule recovered by binding to GFP1-9 by the amount of nucleic acid of each Display molecule that was present before mixing with GFP1-9 (input value) was calculated and corrected with the display efficiency to obtain the recovery rate, which was summarized in Table 3.

Apparent recovery rate (%) = recovered nucleic acid amount (number of molecules)/input nucleic acid amount (number of molecules) × 100

Recovery rate (%) = recovered nucleic acid amount (number of molecules)/input nucleic acid amount (number of molecules) × each display efficiency × 0.01) × 100

[Table 3]

| | Sequence | GFP 1-9 | Input nucleic acid amount (molecules) | Recovered nucleic acid amount (molecules) | Apparent recovery rate (%) | Recovery rate (%) |
|---|---|---|---|---|---|---|
| 1 | GFP10-11 | + | 1.00E+12 | 1.31E+11 | 13.08042% | 23.358% |
| 2 | GFP10 | + | 9.79E+11 | 2.32E+08 | 0.02374% | 0.028% |
| 3 | GFP11 | + | 8.22E+11 | 8.95E+07 | 0.01089% | 0.012% |

As a result, the GFP10-11 fusion peptide was recovered by GFP1-9, and it was shown that the recovery rate of GFP10 alone or GFP11 alone was about 1,000 times lower than the recovery rate of the fusion peptide. Thus, it was shown that GFP1-9 does not associate with GFP11 (or GFP10) in the absence of GFP10 (or GFP11) and that GFP10 and GFP11 must be in proximity, association, or interaction so that GFP1-9 associates with GFP10 and GFP11. The results showed that the use of a molecule of GFP10 bound to one target protein, a molecule of GFP11 bound to another target protein, and GFP1-9 allowed for efficient screening for molecules capable of forming a complex with a plurality

of target molecules (e.g., molecules that enhance or inhibit binding between a plurality of target molecules, molecules that link a plurality of target molecules, or molecules that induce complex formation by a plurality of target molecules).

Example 3: Rapamycin recovery verification experiment utilizing GFP complementation

Next, fusion proteins were prepared by attaching different proteins to GFP10 and GFP11, respectively. Then, verification was performed to determine whether it is possible to selectively recover a complex of the proteins with GFP1-9 due to the proximity effect of GFP10 and GFP11 by the formation of the complex with the proteins. FK506-binding protein (FKBP) and FKBP-Rapamycin binding domain of FKBP12-Rapamycin-associated protein (FRB) were used as a model of protein pairs for complex formation. These two proteins are known for forming a complex in the presence of rapamycin.

Rapamycin-nucleic acid was prepared by chemically linking a nucleic acid to rapamycin, and verification was performed to determine whether GFP1-9 recovers a complex of two fusion proteins and rapamycin.

(1) Preparation of rapamycin-click tag

The analysis conditions of LCMS are as described below.

[Table 4]

| Fractionation condition | Apparatus | Column (I.D. × length (mm)) | Mobile phase | Gradient (A/B) | Flow rate (ml/min) | Column temperature (°C) | Wavelength |
|---|---|---|---|---|---|---|---|
| SQD FA05 | Acquity UPLC/SQ D2 | Aldrich Ascentis Express C18 2.7 µm (2.1 × 50) | A) 0.1% FA, in $H_20$ B) 0.1% FA in $CH_3CN$ | 95/5 => 0/100 (1.0 min) => 0/100 (0.4 min) | 0.9 | 35 | 210-400 nm PDA total |
| LTQ TEA/ HFIP 20_01 | Acquity UPLC/LTQ Orbitra p XL | Waters ACQUITY UPLC BEH C18 1.7µm (2.1 × 50) | A) 15 mM TEA, 400 mM HFIP in $H_20$ B) 15 mM TEA, 400 mM HFIP in MeOH | 80/20 => 2/98 (9.0 min) => 2/98 (1.0 min) => 80/20 (0.1 min) => 80/20 (1.9 min) | 0.20 mL/min (10.0 min) => 0.40 mL/min (2.0 min) | 40 | 190-400 nm PDA total |

(1-1) Synthesis of dibenzocyclooctine (DBCO)-modified rapamycin derivative

Synthesis of rapamycin, 42-[22-(11,12-didehydrodibenzo[b,f]azocin-5(6H)-yl)-17,22-dioxo-4,7,10,13-tetraoxa-16-azadcosan-1-oate] (compound nk01)

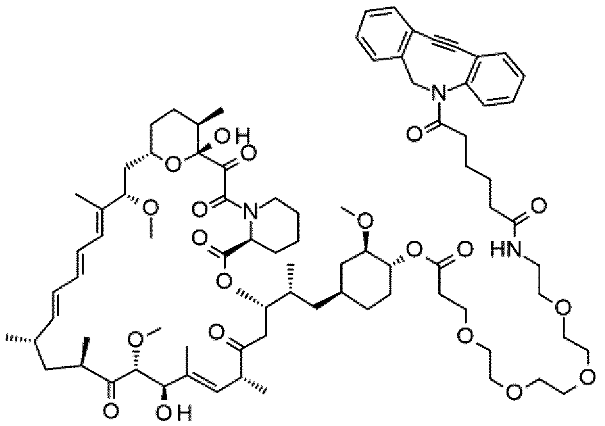

Under nitrogen atmosphere, dibenzocyclooctine-PEG4-acid (7.5 mg, 0.013 mmol) was dissolved in tetrahydrofuran (THF) (64.3 μL), and the mixture was cooled to 0°C, and then triethylamine (2.7 μL, 0.019 mmol) and 2,4,6-trichlorobenzoyl chloride (2.0 μL, 0.013 mmol) were added, and the mixture was stirred at 0°C for 1 hour. To the reaction mixture, rapamycin (11.8 mg, 0.013 mmol) and 4-dimethylaminopyridine (1.6 mg, 0.013 mmol) were added. The reaction mixture was stirred at 0°C for 5 minutes followed by at 15°C for 3 hours, and then was purified by reverse-phase silica gel column chromatography (0.1% aqueous formic acid/0.1% acetonitrile formic acid solution) to obtain rapamycin, 42-[22-(11,12-didehydrodibenzo[b,f]azocin-5(6H)-yl)-17,22-dioxo-4,7,10,13-tetraoxa-16-azadcosan-1-oate] (compound nk01) (3.9 mg, 21%).

LCMS (ESI) m/z = 1477 $(M + H)^+$
Retention time: 1.15 minutes (analytical condition SQDFA05)

(1-2) Preparation of rapamycin-nucleic acid

Synthesis of compound nk02

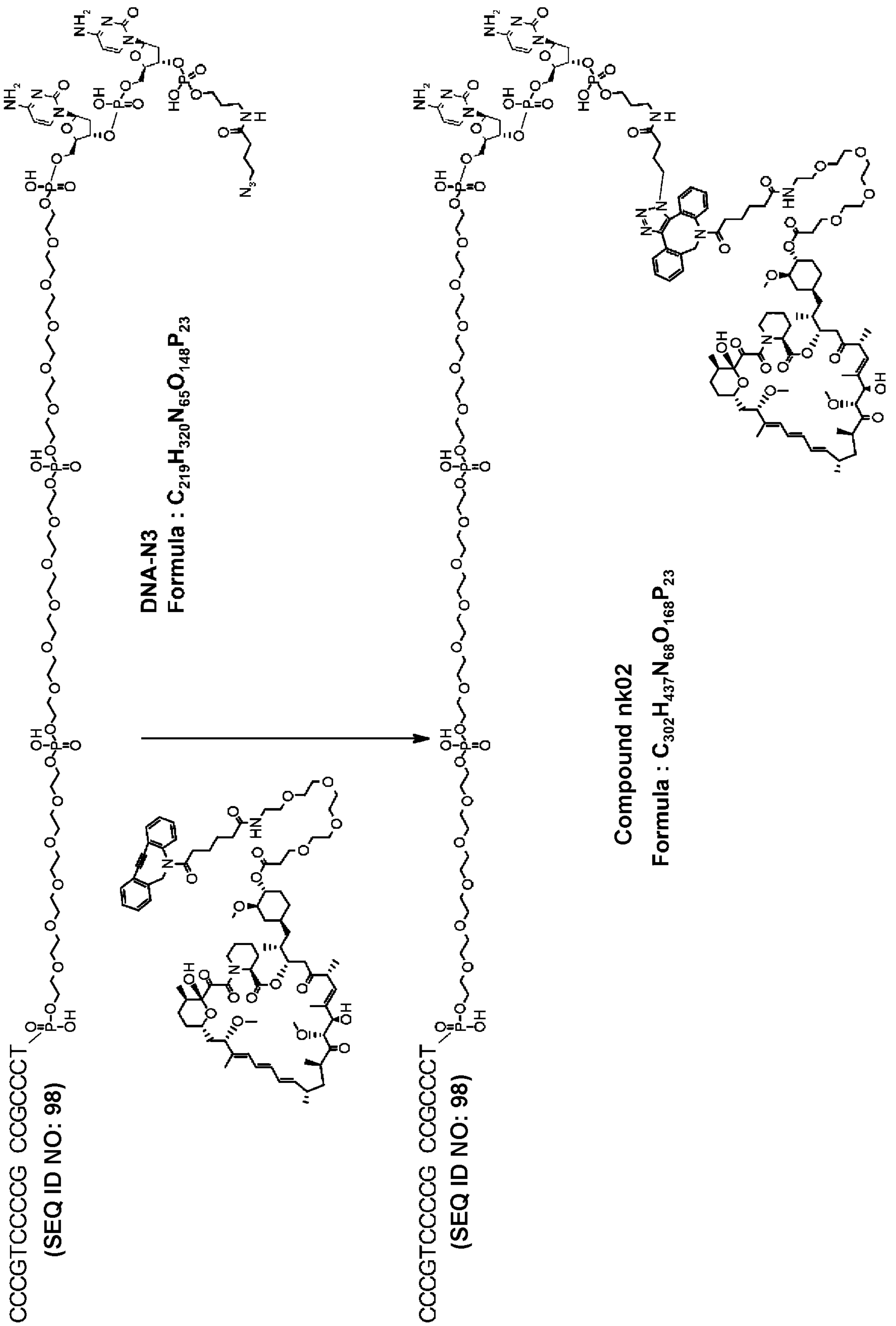

CCCGTCCCCG CCGCCCT (SEQ ID NO: 98)

DNA-N3

Formula : $C_{219}H_{320}N_{65}O_{148}P_{23}$

CCCGTCCCCG CCGCCCT (SEQ ID NO: 98)

Compound nk02

Formula : $C_{302}H_{437}N_{68}O_{168}P_{23}$

To a solution of 10 mM 42-[22-(11,12-didehydrodibenzo[b,f]azocin-5(6H)-yl)-17,22-dioxo-4,7,10,13-tetraoxa-16-azadcosan-1-oate] (compound nk01) in DMSO (20 μL), an aqueous solution of 2 mM DNA-N3 (SEQ ID NO: 98) in 10 mM sodium acetate (20 μL) and DMSO (20 μL) were added, and the mixture was allowed to stand still at 37°C for

16 hours. To the reaction mixture, water (60 μL) and a chloroform-isoamyl alcohol mixture solution (120 μL) were added, and liquid-liquid extraction was performed. The aqueous layer was lyophilized, and the obtained residue was dissolved in DMSO (80 μL) to obtain a solution of the crude product (compound nk02) in DMSO.

LC-HRMS: m/z = 934.5680 $(M-9H)^{9-}$, Calcd 934.5685 $(M-9H)^{9-}$, (Calcd for $C_{302}H_{437}N_{68}O_{168}P_{23}$: Molecular Weight 8420.4)

Retention time: 6.57 minutes (analytical condition LTQ TEA/HFIP20_01)

Synthesis of compound nk03

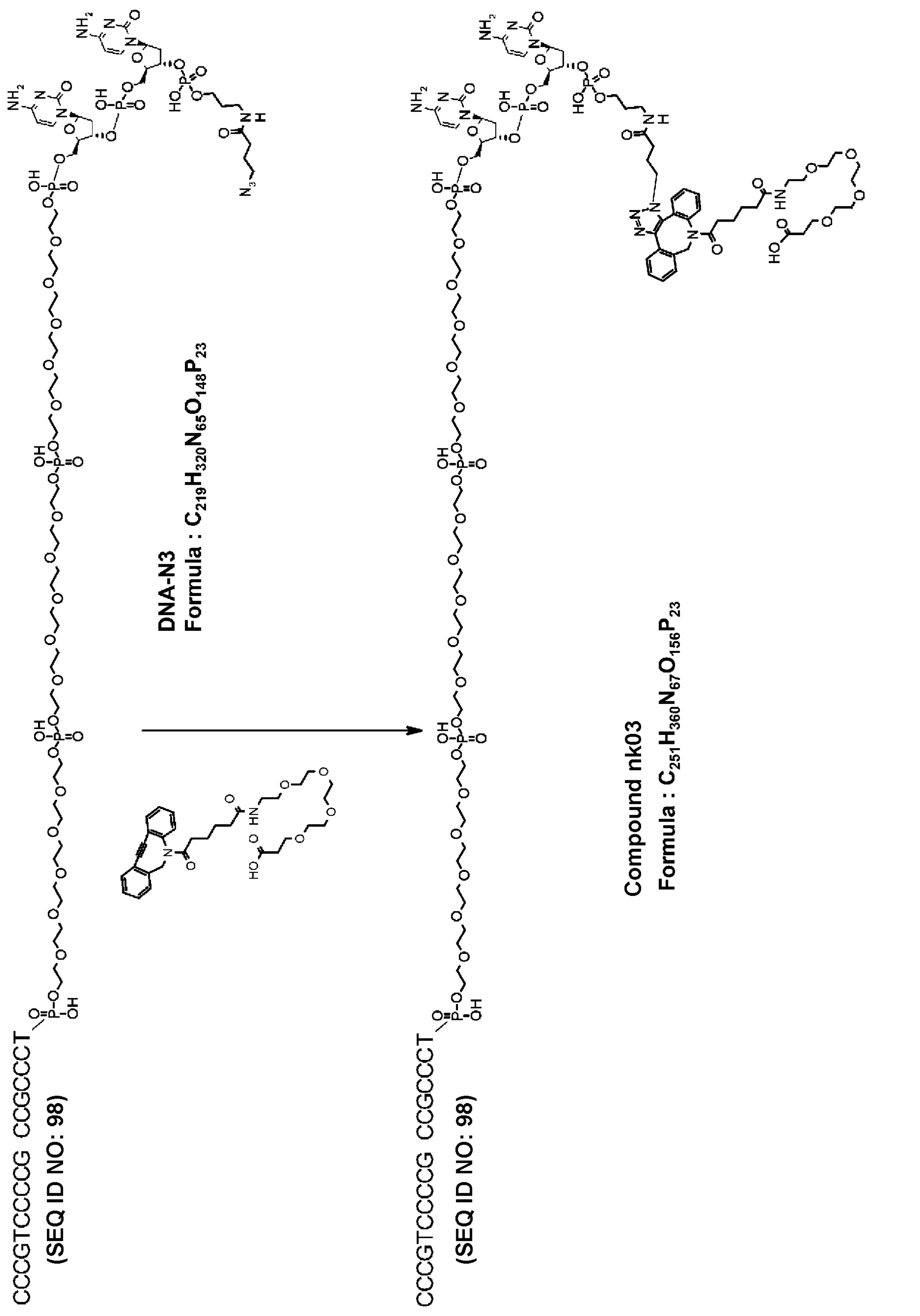

CCCGTCCCCG CCGCCCT (SEQ ID NO: 98)

DNA-N3
Formula : $C_{219}H_{320}N_{65}O_{148}P_{23}$

CCCGTCCCCG CCGCCCT (SEQ ID NO: 98)

Compound nk03
Formula : $C_{251}H_{360}N_{67}O_{156}P_{23}$

To a solution (2.5 μL) of 20 mM dibenzocyclooctine-PEG4-acid in DMSO, an aqueous solution (5 μL) of 2 mM DNA-N3 (SEQ ID NO: 98) and an aqueous solution (2.5 μL) of 20 mM sodium acetate were added, and the mixture was

allowed to stand still at 37°C for 16 hours. The reaction solution was lyophilized, and the obtained residue was dissolved in DMSO (20 μL) to obtain a solution of the crude product (compound nk03) in DMSO.

LC-HRMS: m/z = 1503.7145 $(M-5H)^{5-}$, Calcd 1503.7196 $(M-5H)^{5-}$, (Calcd for $C_{251}H_{360}N_{67}O_{156}P_{23}$: Molecular Weight 7524.3)
Retention time: 3.61 minutes (analytical condition LTQ TEA/HFIP20_01)

(2) Preparation of rapamycin-nucleic acid

Nucleic acids for detection by qPCR were linked to compound nk02 or compound nk03 with T4 RNA ligase to prepare rapamycin-click-tag-nucleic acid molecules (compound nk04, compound nk06) and click-tag-nucleic acid molecules (compound nk05, compound nk07: used as negative control) for use in model experiments.

Template mRNA (SEQ ID NO: 32 (mR-4), SEQ ID NO: 122 (mR-17), SEQ ID NO: 123 (mR-18)) was synthesized from template DNA (SEQ ID NO: 20 (D-6), SEQ ID NO: 120 (D-22), SEQ ID NO: 121 (D-23)) by in vitro transcription reaction using RiboMAX Large Scale RNA Production System T7 (Promega Corporation, P1300), and purified by RNeasy Mini kit (QIAGEN N.V.). 1 × ligation buffer (50 mM HEPES KOH (pH 8.2), 10 mM $MgCl_2$, 2.67 mM DTT, 0.67 mM ATP), 10% PEG8000, 32 μM compound nk02 or compound nk03, 10 μM mR-4, and 2 U/μL T4 RNA ligase (NEB, #M0204L) were mixed, and the mixture was incubated at 37°C for 1 hour, and the ligation reaction was performed. After the ligation reaction, the mixture was purified by RNeasy Mini kit (QIAGEN N.V.) and prepared. A compound of SEQ ID NO: 32 linked to compound nk02 was designated as compound nk04; a compound of SEQ ID NO: 32 linked to compound nk03 was designated as compound nk05; a mixture of SEQ ID NO: 122 or SEQ ID NO: 123 linked to compound nk02 was designated as compound nk06; and a mixture of SEQ ID NO: 122 or SEQ ID NO: 123 linked to compound nk03 was designated as compound nk07.

In (Random) 9 of SEQ ID NO: 120 and SEQ ID NO: 121, 9 residues of any codon of AAC, ACT, AGG, AGT, ATT, CAG, CAT, CCG, CGG, CGT, CTA, GAA, GCT, GGT, GTA, TGC, TGG, or TTT are present randomly.

In (Random) 9 of SEQ ID NO: 122 and SEQ ID NO: 123, 9 residues of any codon of AAC, ACU, AGG, AGU, AUU, CAG, CAU, CCG, CGG, CGU, CUA, GAA, GCU, GGU, GUA, UGC, UGG, or UUU are present randomly.

Template DNA SEQ ID NO: 20 (D-6)

GTAATACGACTCACTATAGGGTTAACTTTAAGAAGGAGATATACATATGCAGTTTAT
GTTTTAGGAGATTCTGAGGCCGACCGGCACCGGCACCGGCGATAGGGCGGCGGGGA
CAAA

mRNA SEQ ID NO: 32 (mR-4)

GGGUUAACUUUAAGAAGGAGAUAUACAUAUGCAGUUUAUGUUUUAGGAGAUUCU
GAGGCCGACCGGCACCGGCACCGGCGAUAGGGCGGCGGGGACAAA

Template DNA SEQ ID NO: 120 (D-22)

GTAATACGACTCACTATAGGGTTAACTTTAAGAAGGAGATATACATATG(Random)9A
TGCCGACCGGCACCGGCACCGGCGATAGGGCGGCGGGGACAAA

Template DNA SEQ ID NO: 121 (D-23)

GTAATACGACTCACTATAGGGTTAACTTTAAGAAGGAGATATACATATG(Random)9TAGCCGACCGGCACCGGCACCGGCGATAGGGCGGCGGGGACAAA

mRNA SEQ ID NO: 122 (mR-17)

GGGUUAACUUUAAGAAGGAGAUAUACAUAUG(Random)9AUGCCGACCGGCACCGGCACCGGCGAUAGGGCGGCGGGGACAAA

mRNA SEQ ID NO: 123 (mR-18)

GGGUUAACUUUAAGAAGGAGAUAUACAUAUG(Random)9UAGCCGACCGGCACCGGCACCGGCGAUAGGGCGGCGGGGACAAA

(3) Preparation of fusion proteins

A fusion protein linking FKBP and GFP11 (Hisx6-FKBP12-Link12-GFP11, SEQ ID NO: 21 of nucleotide sequence, SEQ ID NO: 86 of amino acid sequence) and a fusion protein linking FRB and GFP10 (GFP 10 -Link 12 -FRB -H sx6, SEQ ID NO: 22 of nucleotide sequence, SEQ ID NO: 87 of amino acid sequence) were prepared.

Specifically, pET11a was used as an expression vector, and E. coli BL21 (DE3) was transformed to create an expression strain. The cells were cultured in a TB medium supplemented with ampicillin until OD600 reached 0.5, and induced with 0.5 mM IPTG. Hisx6-FKBP12-Link12-GFP11 was cultured at 37°C for 4 hours, and GFP10-Link12-FRB-Hisx6 was cultured at 15°C overnight. The collected soluble fractions were purified using Profinity IMAC Ni-Charged Resin (BIO-RAD) according to the basic protocol, and then placed in a storage buffer (50 mM Tris-HCl, 150 mM NaCl, 10% glycerol, pH 8.0) and stored at -80°C.

A GS linker of 12 residues was placed between the proteins to be fused. The amino acid sequences of each protein are shown in Table 5.

[Table 5]

| SEQ ID NO | Protein name | Amino acid sequence |
|---|---|---|
| SEQ ID NO: 86 | Hisx6-FKBP12-Link12-GFP11 | MHHHHHHMGVQVETISPGDGRTFPKRGQTCVVHYTGMLEDGKKFDSSRDRNKPFKFMLGKQEVIRGWEEGVAQMSVGQRAKLTISPDYAYGATGHPGIIPPHATLVFDVELLKLEGGGGSGGGSGGSTSEKRDHMVLLEYVTAAGITDAS |
| SEQ ID NO: 87 | GFP10-Link12-FRB-Hisx6 | MDLPDNHYLSTQTILLKDLNDVGGGGSGGGSGGSEMWHEGLEEASRLYFGERNVKGMFEVLEPLHAMMERGPQTLKETSFNQAYGRDLMEAQEWCRKYMKSGNVKDLTQAWDLYYHVFRRISKQHHHHHH |

(4) Rapamycin recovery verification experiment utilizing GFP complementation

Compound nk06, GFP1-9, a fusion protein linking a protein that forms a complex via rapamycin (FKBP or FRB) and GFP11 or 10 (Hisx6-FKBP12-Link12-GFP11 or GFP10-Link12-FRB-Hisx6) were used to verify whether the complex could be recovered by complementation with GFP1-9 due to the proximity effect of GFP10 and 11, using the recovery amount of nucleic acid of compound nk06 as an indicator.

To avoid binding of compound nk06 or compound nk07 at the mRNA portion, reverse transcription of the mRNA site was performed. 1 × M-MLV RT Reaction buffer (Promega Corporation, #368B), 0.5 mM dNTP, 8 U/μL M-MLV RT (H-) Point Mutant (Promega Corporation, #368B), 3 μM Rv-primer (SEQ ID NO: 6), and 0.5 μM compound nk06 or

compound nk07 were mixed, and the mixture was allowed to react at 42°C for 1 hour.

In condition 1, Hisx6-FKBP12-Link12-GFP11, GFP10-Link12-FRB-Hisx6, and biotinylated GFP1-9 were mixed at 1 μM each in a buffer of 1 × TBS, 2 mg/mL BSA (Invitrogen), and the reverse transcript of compound nk06 was added at 0.375 μM, and the mixture was allowed to react at room temperature for 1 hour.

In condition 2, Hisx6-FKBP12-Link12-GFP11 and GFP10-Link12-FRB-Hisx6 were mixed at 1 μM each in a buffer of 1 × TBS, 2 mg/mL BSA (Invitrogen), and the reverse transcript of compound nk06 was added at 0.375 μM, and the mixture was allowed to react at room temperature for 1 hour.

In condition 3, Hisx6-FKBP12-Link12-GFP11 and biotinylated GFP1-9 were mixed at 1 μM each in a buffer of 1 × TBS, 2 mg/mL BSA (Invitrogen), and the reverse transcript of compound nk06 was added at 0.375 μM, and the mixture was allowed to react at room temperature for 1 hour.

In condition 4, GFP10-Link12-FRB-Hisx6 and biotinylated GFP1-9 were mixed at 1 μM each in a buffer of 1 × TBS, 2 mg/mL BSA (Invitrogen), and the reverse transcript of compound nk06 was added at 0.375 μM, and the mixture was allowed to react at room temperature for 1 hour.

In condition 5, Hisx6-FKBP12-Link12-GFP11, GFP10-Link12-FRB-Hisx6, and biotinylated GFP1-9 were mixed at 1 μM each in a buffer of 1 × TBS, 2 mg/mL BSA (Invitrogen), and the reverse transcript of compound nk07 was added at 0.375 μM, and the mixture was allowed to react at room temperature for 1 hour.

The biotinylated GFP1-9 or a complex containing the same was recovered by pull-down with Streptavidin-coated magnetic beads, washed several times with tTBS (NACALAI TESQUE, INC.), and then an elution buffer (1 × Ex taq buffer, 0.2 mM dNTPs, 0.5 μM Fw-primer (SEQ ID NO: 4), 0.5 μM Rv-primer (SEQ ID NO: 6)) was added to suspend, and the suspension was heated at 95°C for 10 minutes to elute nucleic acids. After the reaction, the supernatant was collected and subjected to qPCR with a complementary primer (Fw-primer (SEQ ID NO: 4), Rv-primer (SEQ ID NO: 6)). For the qPCR reaction solution, 1 × Extaq buffer, 0.2 mM dNTP, 0.5 μM Fw-primer, 0.5 μM Rv-primer, 200,000 times dilution SYBR Green I (Lonza K.K., 50513), and 0.02 U/μL Ex Taq polymerase (Takara Bio Inc.) were mixed, and PCR was performed by heating at 95°C for 2 minutes, and then repeating the cycles of 95°C for 10 seconds, 57°C for 20 seconds, and 72°C for 30 seconds for 40 cycles.

To draw a calibration curve for each sequence, dilutions were made to be 1E + 8/μL, 1E + 6/μL, and 1E + 4/μL in nucleic acid amount, and subjected to qPCR under the same conditions.

The recovery rate was calculated from the formula below by dividing the recovery rate of the nucleic acids of compound nk06 or compound nk07 recovered by the binding to GFP1-9 via Hisx6-FKBP12-Link12-GFP11 and GFP10-Link12-FRB-Hisx6 by the input value (the amount of compound nk06 or compound nk07 input into the assay) and summarized in Table 6.

$$\text{Recovery rate (\%)} = \text{recovered nucleic acid amount/input nucleic acid amount} \times 100$$

[Table 6]

| | Sample | | | | Input nucleic acid amount (molecules) | Recovered nucleic acid amount (molecules) | Recovery rate (%) |
|---|---|---|---|---|---|---|---|
| Condition 1 | Compound nk06 | splitGFP1-9 | GFP10-FRB | FKBP12-GFP11 | 4.06E+11 | 3.50E+10 | 8.623% |
| Condition 2 | Compound nk06 | - | GFP10-FRB | FKBP12-GFP11 | 4.06E+11 | 9.54E+06 | 0.002% |
| Condition 3 | Compound nk06 | splitGFP1-9 | - | FKBP12-GFP11 | 4.06E+11 | 8.21E+08 | 0.202% |
| Condition 4 | Compound nk06 | splitGFP1-9 | GFP10-FRB | - | 4.06E+11 | 2.24E+08 | 0.055% |
| Condition 5 | Compound nk07 | splitGFP1-9 | GFP10-FRB | FKBP12-GFP11 | 4.89E+11 | 4.28E+08 | 0.088% |

As a result, the recovery rate was high only under conditions (condition 1) where all compound of nk06, Hisx6-FKBP12-Link12-GFP11, GFP10-Link12-FRB-Hisx6, and biotinylated GFP1-9 were present. From this, it was indicated

that the presence of both Hisx6-FKBP12-Link12-GFP11 and GFP10-Link12-FRB-Hisx6 is required for compound nk06 to be recovered using biotinylated GFP1-9, suggesting that it is possible to selectively obtain molecules capable of promoting the interaction of two proteins, such as rapamycin, by using the GFP system.

Rapamycin is known to exhibit a binding ability of about Kd = 12 nM to FKBP12 protein(J Am Chem Soc. 2005; 127: 4715-21), but rapamycin was not recovered under conditions where GFP10-Link12-FRB-Hisx6 was not present (Condition 2). Taken together with the results of Example 2, it was shown that, when different target proteins were linked to GFP10 and GFP11 respectively, the test compound capable of forming a complex of three components with these target proteins could be selectively recovered, and the test compound capable of forming a complex only with either target protein would not be recovered.

Example 4: Application examples of proteins other than GFP

Next, verification was performed to determine whether molecules other than GFP can recover a target protein by the proximity effect via a test compound. The proteins used were monoubiquitin, Nanoluc (registered trademark) which is luciferase from deep-sea shrimp (Oplophorus gracilirostris), and a SNAP tag that was created by modifying human O6-alkylguanine-DNA alkyltransferase (hAGT) to react specifically with a benzylguanine derivative which is a substrate.

Example 4-1: Complementation assay of splitSNAP-tag using mRNA Display

Split SNAP tags are proteins in which the SNAP-tag is divided between the 91st and 92nd residues, and the proximity of both proteins recovers the enzymatic activity, thus are used in imaging PPI in cells.

Utilizing this property, an experiment was performed to verify whether selective recovery of the full-length body was possible or not due to the reaction in which the N-terminal molecule of split SNAP-tag or the C-terminal molecule of split SNAP-tag alone does not react with the biotin-modified SNAP-tag substrate (SNAP-biotin), and only the full-length body can react with.

Example 4-1-1: Synthesis of template DNA

DNA sequences (SEQ ID NO: 23 (D-9) to SEQ ID NO: 25 (D-11)) encoding a SNAP tag N-terminal fragment to which a GS linker is added at the C-terminus, a SNAP tag C-terminal fragment to which a GS linker is added at the C-terminus, and a full-length SNAP tag to which a GS linker is added at the C-terminus, respectively were prepared by PCR using a pSNAPf vector (New England BioLabs, Inc.) as a template. In this Example, the SNAP tag N-terminal fragment to which a GS linker is added at the C-terminus is referred to as SNAP(N), the SNAP tag C-terminal fragment to which a GS linker is added at the C-terminus is referred to as SNAP(C), and the full-length SNAP tag to which a GS linker is added at the C-terminus is referred to as SNAP (full).

DNA (SEQ ID NO: 23 (D-9)) encoding the SNAP tag N-terminal fragment to which a GS linker is added at the C-terminus was prepared by performing two-step PCR with the following primers.

In the 1st PCR, pSNAPf vector (New England BioLabs, Inc.) was used as a template, and Fw-primer (SEQ ID NO: 7) and Rv-primer (SEQ ID NO: 8) were used.

In the 2nd PCR, a DNA sequence of interest (SEQ ID NO: 23 (D-9)) was obtained by performing PCR with Fw-primer (SEQ ID NO: 9) and Rv-primer (SEQ ID NO: 10) using the 1st PCR product as a template.

DNA SEQ ID NO: 23 (D-9)

DNA sequence: SNAP(N)

GGCGTAATACGACTCACTATAGGGTTAACTTTAATAAGGAGATATAAATATGGACA
AAGACTGCGAAATGAAGCGCACCACCCTGGATAGCCCTCTGGGCAAGCTGGAACTG
TCTGGGTGCGAACAGGGCCTGCACCGTATCATCTTCCTGGGCAAAGGAACATCTGCC
GCCGACGCCGTGGAAGTGCCTGCCCCAGCCGCCGTGCTGGGCGGACCAGAGCCACT
GATGCAGGCCACCGCCTGGCTCAACGCCTACTTTCACCAGCCTGAGGCCATCGAGG
AGTTCCCTGTGCCAGCCCTGCACCACCCAGTGTTCCAGCAGGGCTCTGGCTCTGGCT
CTGGCTCTGGCTCTAAAAAAA

DNA (SEQ ID NO: 24 (D-10)) encoding the SNAP tag C-terminal fragment to which a GS linker is added at the C-terminus was prepared by performing two-step PCR with the following primers.

In the 1st PCR, pSNAPf vector (New England BioLabs, Inc.) was used as a template, and Fw-primer (SEQ ID NO: 11) and Rv-primer (SEQ ID NO: 12) were used.

In the 2nd PCR, a DNA sequence of interest (SEQ ID NO: 24 (D-10)) was obtained by performing PCR with Fw-primer (SEQ ID NO: 9) and Rv-primer (SEQ ID NO: 10) using the 1st PCR product as a template.

DNA SEQ ID NO: 24 (D-10)

DNA sequence: SNAP(C)

GGCGTAATACGACTCACTATAGGGTTAACTTTAATAAGGAGATATAAATATGGTCG
ACGAGAGCTTTACCCGCCAGGTGCTGTGGAAACTGCTGAAAGTGGTGAAGTTCGGA
GAGGTCATCAGCTACAGCCACCTGGCCGCCCTGGCCGGCAATCCCGCCGCCACCGC
CGCCGTGAAAACCGCCCTGAGCGGAAATCCCGTGCCCATTCTGATCCCCTGCCACCG
GGTGGTGCAGGGCGACCTGGACGTGGGGGGCTACGAGGGCGGGCTCGCCGTGAAA
GAGTGGCTGCTGGCCCACGAGGGCCACAGACTGGGCAAGCCTGGGCTGGGTCCTGC

AGGCGGATCCGCGTTTAAACTCGAGGTTAATGGCTCTGGCTCTGGCTCTGGCTCTGG
CTCTAAAAAAA

DNA (SEQ ID NO: 25 (D-11) encoding the SNAP tag N-terminal fragment to which a GS linker is added at the C-terminus was prepared by performing two-step PCR with the following primers.

In the 1st PCR, pSNAPf vector (New England BioLabs, Inc.) was used as a template, and Fw-primer (SEQ ID NO: 7) and Rv-primer (SEQ ID NO: 12) were used.

In the 2nd PCR, a DNA sequence of interest (SEQ ID NO: 25 (D-11)) was obtained by performing PCR with Fw-primer (SEQ ID NO: 9) and Rv-primer (SEQ ID NO: 10) using the 1st PCR product as a template.

DNA SEQ ID NO: 25 (D-11)

DNA sequence: SNAP(full)

GGCGTAATACGACTCACTATAGGGTTAACTTTAATAAGGAGATATAAATATGGACA
AAGACTGCGAAATGAAGCGCACCACCCTGGATAGCCCTCTGGGCAAGCTGGAACTG
TCTGGGTGCGAACAGGGCCTGCACCGTATCATCTTCCTGGGCAAAGGAACATCTGCC
GCCGACGCCGTGGAAGTGCCTGCCCCAGCCGCCGTGCTGGGCGGACCAGAGCCACT
GATGCAGGCCACCGCCTGGCTCAACGCCTACTTTCACCAGCCTGAGGCCATCGAGG
AGTTCCCTGTGCCAGCCCTGCACCACCCAGTGTTCCAGCAGGAGAGCTTTACCCGCC
AGGTGCTGTGGAAACTGCTGAAAGTGGTGAAGTTCGGAGAGGTCATCAGCTACAGC
CACCTGGCCGCCCTGGCCGGCAATCCCGCCGCCACCGCCGCCGTGAAAACCGCCCT
GAGCGGAAATCCCGTGCCCATTCTGATCCCCTGCCACCGGGTGGTGCAGGGCGACCT
GGACGTGGGGGGCTACGAGGGCGGGCTCGCCGTGAAAGAGTGGCTGCTGGCCCACG
AGGGCCACAGACTGGGCAAGCCTGGGCTGGGTCCTGCAGGCGGATCCGCGTTTAAA
CTCGAGGTTAATGGCTCTGGCTCTGGCTCTGGCTCTGGCTCTAAAAAAA

Example 4-1-2: Synthesis of mRNA

mRNAs (SEQ ID NO: 33 (mR-5) to SEQ ID NO: 35 (mR-7)) were synthesized from template DNAs (SEQ ID NO: 23 (D-9) to SEQ ID NO: 25 (D-11)) by in vitro transcription reaction using T7 RNA polymerase and purified by RNeasy kit (QIAGEN N.V.).

mRNA SEQ ID NO: 33 (mR-5)

RNA sequence: SNAP(N)_A7_mRNA

GGGUUAACUUUAAUAAGGAGAUAUAAAUAUGGACAAAGACUGCGAAAUGAAGCG
CACCACCCUGGAUAGCCCUCUGGGCAAGCUGGAACUGUCUGGGUGCGAACAGGGC
CUGCACCGUAUCAUCUUCCUGGGCAAAGGAACAUCUGCCGCCGACGCCGUGGAAG
UGCCUGCCCCAGCCGCCGUGCUGGGCGGACCAGAGCCACUGAUGCAGGCCACCGC
CUGGCUCAACGCCUACUUUCACCAGCCUGAGGCCAUCGAGGAGUUCCCUGUGCCA
GCCCUGCACCACCCAGUGUUCCAGCAGGGCUCUGGCUCUGGCUCUGGCUCUGGCU
CUAAAAAAA

mRNA SEQ ID NO: 34 (mR-6)

RNA sequence: SNAP(C)_A7_mRNA

GGGUUAACUUUAAUAAGGAGAUAUAAAUAUGGUCGACGAGAGCUUUACCCGCCA
GGUGCUGUGGAAACUGCUGAAAGUGGUGAAGUUCGGAGAGGUCAUCAGCUACAG
CCACCUGGCCGCCCUGGCCGGCAAUCCCGCCGCCACCGCCGCCGUGAAAACCGCCC
UGAGCGGAAAUCCCGUGCCCAUUCUGAUCCCCUGCCACCGGGUGGUGCAGGGCGA
CCUGGACGUGGGGGGCUACGAGGGCGGGCUCGCCGUGAAAGAGUGGCUGCUGGCC
CACGAGGGCCACAGACUGGGCAAGCCUGGGCUGGGUCCUGCAGGCGGAUCCGCGU
UUAAACUCGAGGUUAAUGGCUCUGGCUCUGGCUCUGGCUCUGGCUCUAAAAAAA

mRNA SEQ ID NO: 35 (mR-7)

RNA sequence: SNAP(full)_A7_mRNA

GGGUUAACUUUAAUAAGGAGAUAUAAAUAUGGACAAAGACUGCGAAAUGAAGCG
CACCACCCUGGAUAGCCCUCUGGGCAAGCUGGAACUGUCUGGGUGCGAACAGGGC
CUGCACCGUAUCAUCUUCCUGGGCAAAGGAACAUCUGCCGCCGACGCCGUGGAAG
UGCCUGCCCCAGCCGCCGUGCUGGGCGGACCAGAGCCACUGAUGCAGGCCACCGC
CUGGCUCAACGCCUACUUUCACCAGCCUGAGGCCAUCGAGGAGUUCCCUGUGCCA
GCCCUGCACCACCCAGUGUUCCAGCAGGAGAGCUUUACCCGCCAGGUGCUGUGGA
AACUGCUGAAAGUGGUGAAGUUCGGAGAGGUCAUCAGCUACAGCCACCUGGCCGC
CCUGGCCGGCAAUCCCGCCGCCACCGCCGCCGUGAAAACCGCCCUGAGCGGAAAU
CCCGUGCCCAUUCUGAUCCCCUGCCACCGGGUGGUGCAGGGCGACCUGGACGUGG
GGGGCUACGAGGGCGGGCUCGCCGUGAAAGAGUGGCUGCUGGCCCACGAGGGCCA
CAGACUGGGCAAGCCUGGGCUGGGUCCUGCAGGCGGAUCCGCGUUUAAACUCGAG
GUUAAUGGCUCUGGCUCUGGCUCUGGCUCUGGCUCUAAAAAAA

Example 4-1-3: Complementation assay by mRNA Display

In accordance with the method described in WO2013/100132, puromycin was linked via a linker to the 3' end of the mRNA purified above and then translated to prepare the Display molecule of mRNA-peptide. PUREfrex2.0 (GeneFrontier Corporation), a reconstituted cell-free protein synthesis system from a prokaryotic organism, was used as the translation system, and the translation was performed according to the composition of the attached protocol.

To ascertain the display efficiency of each Display molecule, the RNA portion was digested by RNaseONE (Promega Corporation) and electrophoretically separated with TricineP AGE. When the Display molecule is formed, a fusion molecule of the translated peptide and a puromycin linker is present. When the Display molecule is not formed, an unbound linker alone without binding of the translated peptide is present and detected at a position lower than the former. Thus, a total of two bands are detected. From the band ratio, the display efficiency (%) of the Display molecule was calculated according to the formula described above. For the specific reaction solution with RNaseONE, 1 × Reaction buffer, 0.84 μM fusion molecule, and 0.5 U/μL RNaseONE were mixed, and the mixture was allowed to react at 37°C for 1 hour.

Detection by TricinePAGE resulted in a detection of a fluorescently labeled puromycin linker. The display efficiency obtained from the result of TricinePAGE was as shown in Table 7.

[Table 7]

| SEQ ID NO | Peptide name | Amino acid sequence | Display efficiency |
|---|---|---|---|
| SEQ ID NO: 42 | SNAP (N) | MDKDCEMKRTTLDSPLGKLELSGCEQGLHRIIFLGKGTSAADAVEVPAPAAVLGGPEPLMQATAWLNAYFHQPEAIEEFPVPALHHPVFQQGSGSGSGSGSKK | 64% |
| SEQ ID NO: 43 | SNAP (C) | MVDESFTRQVLWKLLKVVKFGEVISYSHLAALAGNPAATAAVKTALSGNPVPILIPCHRVVQGDLDVGGYEGGLAVKEWLLAHEGHRLGKPGLGPAGGSAFKLEVNGSGSGSGSGSKK | 19% |
| SEQ ID NO: 44 | SNAP (full) | MDKDCEMKRTTLDSPLGKLELSGCEQGLHRIIFLGKGTSAADAVEVPAPAAVLGGPEPLMQATAWLNAYFHQPEAIEEFPVPALHHPVFQQESFTRQVLWKLLKVVKFGEVISYSHLAALAGNPAATAAVKTALSGNPVPILIPCHRVVQGDLDVGGYEGGLAVKEWLLAHEGHRLGKPGLGPAGGSAFKLEVNGSGSGSGSGSKK | 33% |

To avoid binding at the mRNA portion, reverse transcription of the mRNA site was performed. Specifically, 1 × M-MLV RT Reaction buffer (Promega Corporation, #368B), 0.5 mM dNTP, 8 U/μL M-MLV RT (H-) Point Mutant (Promega Corporation, #368B), 3 μM Rv-primer (SEQ ID NO: 5), and 0.5 μM translation product were mixed, and the mixture was allowed to react at 42°C for 1 hour.

Each Display molecule alone or a mixture of equally mixed Display molecules of SNAP(N) and SNAP(C) was allowed to stand still at 4°C for 30 minutes in a buffer of 1 × TBS, 2 mg/mL BSA (Invitrogen). To this solution, 1/4 amount of a PBS solution containing 5 mM DTT, 2500 nM SNAP-Biotin (New England BioLabs, Inc.) was added, and the enzymatic reaction was performed at 37°C for 30 minutes.

The Display molecule biotinylated by the enzyme reaction or unreacted SNAP-Biotin was recovered by pull-down with Streptavidin-coated magnetic beads, washed with tTBS (NACALAI TESQUE, INC.) several times, and then purified water was added and incubated at 95°C for 5 minutes to elute the nucleic acid.

After the reaction, the supernatant was collected and subjected to qPCR with a complementary primer (Fw-primer (SEQ ID NO: 99), Rv-primer (SEQ ID NO: 10)). For the qPCR reaction solution, 1 × Extaq buffer, 0.2 mM dNTP, 0.5 μM Fw-primer, 0.5 μM Rv-primer, 200,000 times dilution SYBR Green I (Lonza K.K., #50513), and 0.02 U/μL Ex Taq polymerase (Takara Bio Inc.) were mixed, and PCR was performed by heating at 95°C for 2 minutes, and then repeating the cycles of 95°C for 10 seconds, 55°C for 20 seconds, and 72°C for 60 seconds for 50 cycles.

To draw a calibration curve for each sequence, each Display molecule prepared before the selection (input) was diluted to be 1E + 8/μL, 1E + 6/μL, and 1E + 4/μL in nucleic acid amount, and subjected to qPCR under the same conditions.

The apparent recovery rate (apparent recovery rate (%) = recovered nucleic acid amount/theoretical value of added nucleic acid amount × 100) was obtained by dividing the amount of nucleic acid of each Display molecule recovered by the reaction with SNAP-Biotin and the subsequent pull-down using the Streptavidin-coated magnetic beads by the theoretical amount of nucleic acid added into the reaction system was calculated and corrected with the display efficiency to obtain the "recovery rate" (recovery rate (%) = recovered nucleic acid amount/(theoretical value of added nucleic acid amount × display efficiency × 0.01) × 100), which was summarized in Table 8.

[Table 8]

| | Sequence | Recovered nucleic acid amount (molecules) | Apparent recovery rate (%) | Recovery rate (%) |
|---|---|---|---|---|
| 1 | SNAP(N) | 6.26E+07 | 0.062% | 0.097% |
| 2 | SNAP(C) | 6.76E+06 | 0.007% | 0.035% |
| 3 | SNAP(full) | 4.78E+09 | 4.740% | 14.364% |

(continued)

| | Sequence | Recovered nucleic acid amount (molecules) | Apparent recovery rate (%) | Recovery rate (%) |
|---|---|---|---|---|
| 4 | SNAP(N) + SNAP (C) | 4.59E+07 | 0.046% | 0.240% |

As a result, it was revealed that clones presenting SNAP(full) were efficiently recovered only in the presence of SNAP-Biotin, and the recovery rate was at least 50 times higher than other controls.

This result showed that the use of molecules binding SNAP(N) to one target protein, molecules binding SNAP(C) to another target protein and SNAP-Biotin allows for efficient screening for molecules capable of forming a complex with a plurality of target molecules, similarly to GFP.

Example 4-2: Complementation assay of splitUbiquitin using mRNA Display

split ubiquitin is a tool used for protein-in-protein interaction analysis in cells. It has been reported that by replacing the 13th Ile of ubiquitin with Ala (I13A), the N-terminal molecule and the C-terminal molecule do not naturally associate.

As described above, screening a library for a candidate molecule capable of forming a complex with a plurality of target molecules requires a system that suppresses the recovery rate of a test molecule that forms a complex with only one target molecule. To verify whether split ubiquitin can be used for this screening, a verification experiment was performed that the split ubiquitin N-terminal molecule or the split ubiquitin C-terminal molecule alone does not bind to anti-ubiquitin antibodies, but only the full-length body binds to the antibodies. Ubiquitin I13A variants were used in the experiment. In this Example, the ubiquitin N-terminal fragment to which a GS linker is attached at the C-terminus is referred to as splitUb(N), the ubiquitin C-terminal fragment to which a GS linker is attached at the C-terminus is referred to as splitUb(C), and the protein in which splitUb(N) and splitUb(C) are linked by a GSGS linker and to which a GS linker is attached at the C-terminus is referred to as splitUb(N-C).

Example 4-2-1: Synthesis of mRNA

mRNAs (SEQ ID NO: 36 (mR-8) to SEQ ID NO: 38 (mR-10)) were synthesized from template DNAs (SEQ ID NO: 26 (D-12) to SEQ ID NO: 28 (D-14)) by in vitro transcription reaction using T7 RNA polymerase and purified by RNeasy kit (QIAGEN N.V.).

Template DNA SEQ ID NO: 26 (D-12)

DNA sequence: splitUb(N)

GGCGTAATACGACTCACTATAGGGTTAACTTTAAGAAGGAGATATACATATGATGC
AGATCTTCGTGAAGACCCTGACCGGCAAGACCGCCACTCTGGAGGTGGAGCCCAGT
GACACCATCGAAAATGTGAAGGCCAAGATCCAAGATAAAGAAGGCATCCCCGGCTC
TGGCTCTGGCTCTGGCTCTAAAAAAA

Template DNA SEQ ID NO: 27 (D-13)

DNA sequence: splitUb(C)

GGCGTAATACGACTCACTATAGGGTTAACTTTAAGAAGGAGATATACATATGGGCA
TCCCCCCCGACCAGCAGAGGCTCATCTTTGCAGGCAAGCAGCTGGAAGATGGCCGC
ACTCTTTCTGACTACAACATCCAGAAAGAGTCGACCCTGCACCTGGTCCTGCGCCTG
AGGGGTGGCGGCTCTGGCTCTGGCTCTGGCTCTAAAAAAA

Template DNA SEQ ID NO: 28 (D-14)

DNA sequence: splitUb(N-C)

GGCGTAATACGACTCACTATAGGGTTAACTTTAAGAAGGAGATATACATATGATGC
AGATCTTCGTGAAGACCCTGACCGGCAAGACCGCCACTCTGGAGGTGGAGCCCAGT
GACACCATCGAAAATGTGAAGGCCAAGATCCAAGATAAAGAAGGCATCCCCGGCTC
TGGCTCTGGCATCCCCCCCGACCAGCAGAGGCTCATCTTTGCAGGCAAGCAGCTGGA
AGATGGCCGCACTCTTTCTGACTACAACATCCAGAAAGAGTCGACCCTGCACCTGGT
CCTGCGCCTGAGGGGTGGCGGCTCTGGCTCTGGCTCTGGCTCTAAAAAAA

mRNA SEQ ID NO: 36 (mR-8)

RNA sequence: splitUb(N)

GGGUUAACUUUAAGAAGGAGAUAUACAUAUGAUGCAGAUCUUCGUGAAGACCCU
GACCGGCAAGACCGCCACUCUGGAGGUGGAGCCCAGUGACACCAUCGAAAAUGUG
AAGGCCAAGAUCCAAGAUAAAGAAGGCAUCCCCGGCUCUGGCUCUGGCUCUGGCU
CUAAAAAAA

mRNA SEQ ID NO: 37 (mR-9)

RNA sequence: splitUb(C)

GGGUUAACUUUAAGAAGGAGAUAUACAUAUGGGCAUCCCCCCCGACCAGCAGAG
GCUCAUCUUUGCAGGCAAGCAGCUGGAAGAUGGCCGCACUCUUUCUGACUACAAC
AUCCAGAAAGAGUCGACCCUGCACCUGGUCCUGCGCCUGAGGGGUGGCGGCUCUG
GCUCUGGCUCUGGCUCUAAAAAAA

mRNA SEQ ID NO: 38 (mR-10)

RNA sequence: splitUb(N-C)

GGGUUAACUUUAAGAAGGAGAUAUACAUAUGAUGCAGAUCUUCGUGAAGACCCU
GACCGGCAAGACCGCCACUCUGGAGGUGGAGCCCAGUGACACCAUCGAAAAUGUG
AAGGCCAAGAUCCAAGAUAAAGAAGGCAUCCCCGGCUCUGGCUCUGGCAUCCCCC
CCGACCAGCAGAGGCUCAUCUUUGCAGGCAAGCAGCUGGAAGAUGGCCGCACUCU
UUCUGACUACAACAUCCAGAAAGAGUCGACCCUGCACCUGGUCCUGCGCCUGAGG
GGUGGCGGCUCUGGCUCUGGCUCUGGCUCUAAAAAAA

Example 4-2-2: Complementation assay by mRNA Display

In accordance with the method described in WO2013/100132, puromycin was linked via a linker to the 3' end of the mRNA purified above and then translated to prepare the Display molecules of mRNA-peptide. PUREfrex2.0 (GeneFrontier Corporation), a reconstituted cell-free protein synthesis system from a prokaryotic organism, was used as the translation system, and the translation was performed according to the composition of the attached protocol.

To ascertain the display efficiency of each Display molecule, the RNA portion was digested by RNaseONE (Promega Corporation) and electrophoretically separated with TricineP AGE. When the Display molecule is formed, a fusion molecule of the translated peptide and a puromycin linker is present. When the Display molecule is not formed, an unbound linker alone without binding of the translated peptide is present and detected at a position lower than the former. Thus, a total of two bands are detected. From the band ratio, the display efficiency (%) of the Display molecule was calculated according to the formula described above. For the specific reaction solution with RNaseONE, 1 × Reaction buffer, 0.42 μM fusion molecule, and 0.5 U/μL RNaseONE were mixed, and the mixture was allowed to react at 37°C for 1 hour.

When confirming the display efficiency of each Display molecule, a fluorescently labeled puromycin linker was used, and the fluorescence detection value was evaluated. The display efficiency obtained from the result of Tricine PAGE was as shown in Table 9.

[Table 9]

| SEQ ID NO | Peptide name | Amino acid sequence | Display efficiency |
|---|---|---|---|
| SEQ ID NO: 45 | splitUb (N) | MMQIFVKTLTGKTATLEVEPSDTIENVKAKIQDKEGIPG SGSGSGSKK | 89% |
| SEQ ID NO: 46 | splitUb (C) | MGIPPDQQRLIFAGKQLEDGRTLSDYNIQKESTLHLVLR LRGGGSGSGSGSKK | 56% |
| SEQ ID NO: 47 | splitUb (N-C) | MMQIFVKTLTGKTATLEVEPSDTIENVKAKIQDKEGIPG SGSGIPPDQQRLIFAGKQLEDGRTLSDYNIQKESTLHLVL RLRGGGSGSGSGSKK | 77% |

To avoid binding at the mRNA portion, reverse transcription of the mRNA site was performed. Specifically, 1 × M-MLV RT Reaction buffer (Promega Corporation, #368B), 0.5 mM dNTP, 8 U/μL M-MLV RT (H-) Point Mutant (Promega Corporation, #368B), 3 μM Rv-primer (SEQ ID NO: 5), and 0.5 μM translation product were mixed, and the mixture was allowed to react at 42°C for 1 hour.

Each translated Display molecule was mixed with Anti-Multi Ubiquitin mAb-Magnetic Beads (MEDICAL & BIOLOGICAL LABORATORIES CO., LTD., #D058-11) that are fixed beads of anti-ubiquitin antibodies (Mouse IgG1 monoclonal antibody; clone name FK2), in a buffer of 1 × TBS, 2 mg/mL BSA (Invitrogen), and the mixture was allowed to react at room temperature for 1 hour.

To simultaneously evaluate the interactions of the ubiquitin N-terminal fragment and the C-terminal fragment when they are independent, the conditions of mixing the display molecule of splitUb(N) and the display molecule of splitUb(C) at 1 : 1 were reacted in the same way.

The supernatant was removed, and the residue was washed several times with tTBS (NACALAI TESQUE, INC.), and then an elution buffer (1 × Ex taq buffer, 0.2 mM dNTPs) at the equal amount of the supernatant was added to suspend, and the suspension was heated at 95°C for 10 minutes to elute nucleic acids. The supernatant was collected and subjected to qPCR with a complementary primer (Fw-primer (SEQ ID NO: 4), Rv-primer (SEQ ID NO: 5)). For the qPCR reaction solution, 1 × Extaq buffer, 0.2 mM dNTP, 0.5 μM Fw-primer, 0.5 μM Rv-primer, 200,000 times dilution

SYBR Green I (Lonza K.K., 50513), and 0.02 U/μL Ex Taq polymerase (Takara Bio Inc.) were mixed, and PCR was performed by heating at 95°C for 2 minutes, and then repeating the cycles of 95°C for 10 seconds, 57°C for 20 seconds, and 72°C for 30 seconds for 40 cycles. Each Display molecule before reacting with the anti-ubiquitin antibody fixed beads was also subjected to qPCR in the same way, and the input nucleic acid amount was calculated.

To draw a calibration curve for each sequence, dilutions were made to be 1E + 8/μL, 1E + 6/μL, and 1E + 4/μL in nucleic acid amount, and subjected to qPCR under the same conditions.

The apparent recovery rate obtained by dividing the amount of nucleic acid of each Display molecule recovered by binding to an anti-ubiquitin antibody by the amount of nucleic acid of each Display molecule that was present before mixing in the reaction system (input value) was calculated and corrected with the display efficiency to obtain the recovery rate, which was summarized in Table 10.

$$\text{Apparent recovery rate (\%)} = \text{recovered nucleic acid amount/input nucleic acid amount} \times 100$$

$$\text{Recovery rate (\%)} = \text{recovered nucleic acid amount/input nucleic acid amount} \times \text{each display efficiency} \times 0.01) \times 100$$

[Table 10]

| | Sequence | Recovered nucleic acid amount (molecules) | Apparent recovery rate (%) | Recovery rate (%) |
|---|---|---|---|---|
| 1 | splitUb (N-C) | 6.69E+10 | 12.345% | 16.050% |
| 2 | splitUb (N) | 1.63E+10 | 0.931% | 1.040% |
| 3 | splitUb (C) | 1.16E+10 | 0.571% | 1.012% |
| 4 | splitUb (N)+splitUb (C) | 7.16E+09 | 0.194% | 0.344% |

As a result, it was revealed that the recovery was efficient in the presence of splitUb(N-C), and the recovery rate was at least 16 times higher than other controls.

This result showed that the use of molecules binding the N-terminal molecule of split ubiquitin (I13A) to one target protein, molecules binding the C-terminal molecule of split ubiquitin to another target protein, and an anti-ubiquitin antibody allows for efficient screening for molecules capable of forming a complex with a plurality of target molecules, similarly to GFP.

Example 4-3: Complementation assay of splitUbiquitin using mRNA Display

Next, Display molecules composed of constructs (SEQ ID NO: 55 (P-11) to SEQ ID NO: 57 (P-13)) in which splitUb(N) and splitUb(C) each were added with a charged peptide sequence (K-peptide containing an amino acid having a positive charge) (sometimes referred to as "Kpep") or E-peptide containing an amino acid having a negative charge ("Epep")) were prepared. When the added peptides are attracted to each other by charges, splitUbs become in proximity to each other and recovered by an anti-ubiquitin antibody. Conversely, when the added peptides are repelled to each other by charges, splitUbs are not recovered. Once this is validated, the complexation of molecules added to splitUbs can be evaluated with the recovery rate through ubiquitin and anti-ubiquitin antibodies.

Example 4-3-1: Synthesis of mRNA

mRNAs (SEQ ID NO: 52 (mR-11) to SEQ ID NO: 54 (mR-13)) were synthesized from template DNAs (SEQ ID NO: 49 (D-15) to SEQ ID NO: 51 (D-17)) by in vitro transcription reaction using T7 RNA polymerase and purified by RNeasy kit (QIAGEN N.V.).

Template DNA SEQ ID NO: 49 (D-15)

DNA sequence: Kpep-splitUb(N)

GTAATACGACTCACTATAGGGTTAACTTTAAGAAGGAGATATACATATGAAAGTGA
GCGCGCTGAAAGAAAACGTGAGCGCCCTGAAAGAAAAAGTGAGCGCGCTGACCGA
AAAAGTGAGCGCGCTGAAAGAGAAAGTGAGCGCGCTGAAAGAAGGCTCTGGCTCT
GGCTCTGGCTCTATGCAGATCTTCGTGAAGACCCTGACCGGCAAGACCGCCACTCTG
GAGGTGGAGCCCAGTGACACCATCGAAAATGTGAAGGCCAAGATCCAAGATAAAG
AAGGCATCCCCGGCTCTGGCTCTGGCTCTGGCTCTAAAAAAA

Template DNA SEQ ID NO: 50 (D-16)

DNA sequence: Epep-splitUb(N)

GTAATACGACTCACTATAGGGTTAACTTTAAGAAGGAGATATACATATGAAAGTGA
GCGCGCTGGAAAACGAAGTGAGCGCGCTGGAAAAAGAAGTGAGCGTGCTGGAAAA
AGAAGTGAGCGCGCTGGAAAAAGAAGTGCGCGCGCTGGAAAAAGGCTCTGGCTCTG
GCTCTGGCTCTATGCAGATCTTCGTGAAGACCCTGACCGGCAAGACCGCCACTCTGG
AGGTGGAGCCCAGTGACACCATCGAAAATGTGAAGGCCAAGATCCAAGATAAAGA
AGGCATCCCCGGCTCTGGCTCTGGCTCTGGCTCTAAAAAAA

Template DNA SEQ ID NO: 51 (D-17)

DNA sequence: Epep-splitUb(C)

GGCGTAATACGACTCACTATAGGGTTAACTTTAAGAAGGAGATATACATATGGGCA
TCCCCCCCGACCAGCAGAGGCTCATCTTTGCAGGCAAGCAGCTGGAAGATGGCCGC
ACTCTTTCTGACTACAACATCCAGAAAGAGTCGACCCTGCACCTGGTCCTGCGCCTG
AGGGGTGGCGGCTCTGGCTCTGGCTCTGGCTCTAAAGTGAGCGCGCTGGAAAACGA
AGTGAGCGCGCTGGAAAAAGAAGTGAGCGTGCTGGAAAAAGAAGTGAGCGCGCTG
GAAAAAGAAGTGCGCGCGCTGGAAAAAGGCTCTGGCTCTGGCTCTGGCTCTAAAAA
AA

mRNA SEQ ID NO: 52 (mR-11)

RNA sequence: Kpep-splitUb(N)

GGGUUAACUUUAAGAAGGAGAUAUACAUAUGAAAGUGAGCGCGCUGAAAGAAAA
CGUGAGCGCCCUGAAAGAAAAAGUGAGCGCGCUGACCGAAAAAGUGAGCGCGCU
GAAAGAGAAAGUGAGCGCGCUGAAAGAAGGCUCUGGCUCUGGCUCUGGCUCUAU
GCAGAUCUUCGUGAAGACCCUGACCGGCAAGACCGCCACUCUGGAGGUGGAGCCC
AGUGACACCAUCGAAAAUGUGAAGGCCAAGAUCCAAGAUAAAGAAGGCAUCCCC
GGCUCUGGCUCUGGCUCUGGCUCUAAAAAAA

mRNA SEQ ID NO: 53 (mR-12)

RNA sequence: Epep-splitUb(N)

GGGUUAACUUUAAGAAGGAGAUAUACAUAUGAAAGUGAGCGCGCUGGAAAACGA
AGUGAGCGCGCUGGAAAAAGAAGUGAGCGUGCUGGAAAAAGAAGUGAGCGCGCU
GGAAAAAGAAGUGCGCGCGCUGGAAAAAGGCUCUGGCUCUGGCUCUGGCUCUAU
GCAGAUCUUCGUGAAGACCCUGACCGGCAAGACCGCCACUCUGGAGGUGGAGCCC
AGUGACACCAUCGAAAAUGUGAAGGCCAAGAUCCAAGAUAAAGAAGGCAUCCCC
GGCUCUGGCUCUGGCUCUGGCUCUAAAAAAA

mRNA SEQ ID NO: 54 (mR-13)

RNA sequence: Epep-splitUb(C)

GGGUUAACUUUAAGAAGGAGAUAUACAUAUGGGCAUCCCCCCCGACCAGCAGAG
GCUCAUCUUUGCAGGCAAGCAGCUGGAAGAUGGCCGCACUCUUUCUGACUACAAC
AUCCAGAAAGAGUCGACCCUGCACCUGGUCCUGCGCCUGAGGGGUGGCGGCUCUG
GCUCUGGCUCUGGCUCUAAAGUGAGCGCGCUGGAAAACGAAGUGAGCGCGCUGG

AAAAAGAAGUGAGCGUGCUGGAAAAAGAAGUGAGCGCGCUGGAAAAAGAAGUGC
GCGCGCUGGAAAAAGGCUCUGGCUCUGGCUCUGGCUCUAAAAAAA

Example 4-3-2: Complementation assay by mRNA Display

In accordance with the method described in WO2013/100132, puromycin was linked via a linker to the 3' end of the mRNA purified above and then translated to prepare the Display molecules of mRNA-peptide. PUREfrex2.0 (GeneFrontier Corporation), a reconstituted cell-free protein synthesis system from a prokaryotic organism, was used as the translation system, and the translation was performed according to the composition of the attached protocol.

To ascertain the display efficiency of each Display molecule, the RNA portion was digested by RNaseONE (Promega Corporation) and electrophoretically separated with TricineP AGE. When the Display molecule is formed, a fusion molecule of the translated peptide and a puromycin linker is present. When the Display molecule is not formed, an unbound linker alone without binding of the translated peptide is present and detected at a position lower than the former. Thus, a total of two bands are detected. From the band ratio, the display efficiency (%) of the Display molecule was calculated according to the formula described above. For the specific reaction solution with RNaseONE, 1 × Reaction buffer, 0.42 μM fusion molecule, and 0.5 U/μL RNaseONE were mixed, and the mixture was allowed to react at 37°C

for 1 hour.

When confirming the display efficiency of each Display molecule, a fluorescently labeled puromycin linker was used, and the fluorescence detection value was evaluated. The display efficiency obtained from the result of TricinePAGE was as shown in Table 11.

[Table 11]

| SEQ ID NO | Peptide name | Amino acid sequence | Display efficiency |
|---|---|---|---|
| SEQ ID NO: 55 | Kpep-splitUb (N) | MKVSALKENVSALKEKVSALTEKVSALKEKVSALKEGS GSGSGSMQIFVKTLTGKTATLEVEPSDTIENVKAKIQDK EGIPGSGSGSGSKK | 61% |
| SEQ ID NO: 56 | Epep-splitUb (N) | MKVSALENEVSALEKEVSVLEKEVSALEKEVRALEKGS GSGSGSMQIFVKTLTGKTATLEVEPSDTIENVKAKIQDK EGIPGSGSGSGSKK | 22% |
| SEQ ID NO: 57 | Epep-splitUb (C) | MGIPPDQQRLIFAGKQLEDGRTLSDYNIQKESTLHLVLR LRGGGSGSGSGSKVSALENEVSALEKEVSVLEKEVSALE KEVRALEKGSGSGSGSKK | 32% |

To avoid binding at the mRNA portion, reverse transcription of the mRNA site was performed. Specifically, 1 × M-MLV RT Reaction buffer (Promega Corporation, #368B), 0.5 mM dNTP, 8 U/μL M-MLV RT (H-) Point Mutant (Promega Corporation, #368B), 3 μM Rv-primer (SEQ ID NO: 5), and 0.5 μM translation product were mixed, and the mixture was allowed to react at 42°C for 1 hour.

In condition 1, a Display molecule of Kpep-splitUb(N) and a Display molecule of Epep-splitUb(C) were each added at 0.2 μM in a buffer of 1 × TBS, 2 mg/mL BSA (Invitrogen), and the mixture was allowed to react at room temperature for 1 hour.

Reactions were made under the same conditions as in condition 1, except that, in condition 2, the Display molecule of Epep-splitUb(N) and the Display molecule of Epep-splitUb(C) were added at 0.2 μM; in condition 3, the Display molecule of Kpep-splitUb(N) was added at 0.4 μM; in condition 4, the Display molecule of Epep-splitUb(N) was added at 0.4 μM; and in condition 5, the Display molecule of Epep-splitUb(C) was added at 0.4 μM.

The resultant was mixed with anti-ubiquitin Multi Ubiquitin mAb-Magnetic (MBL, Inc., #D058-11) that is an anti-ubiquitin antibody fixed bead, and the mixture was allowed to react at room temperature for 30 minutes.

The supernatant was removed, and the residue was washed several times with tTBS (NACALAI TESQUE, INC.), and then an elution buffer (1 × Ex taq buffer, 0.2 mM dNTPs) at the equal amount of the supernatant was added to suspend, and the suspension was heated at 95°C for 5 minutes to elute nucleic acids. The supernatant was collected and subjected to qPCR with a complementary primer (Fw-primer (SEQ ID NO: 4), Rv-primer (SEQ ID NO: 5)). For the qPCR reaction solution, 1 × Ex taq buffer, 0.2 mM dNTP, 0.5 μM Fw-primer, 0.5 μM Rv-primer, 200,000 times dilution SYBR Green I (Lonza K.K., 50513), and 0.02 U/μL Ex Taq polymerase (Takara Bio Inc.) were mixed, and PCR was performed by heating at 95°C for 2 minutes, and then repeating the cycles of 95°C for 10 seconds, 57°C for 20 seconds, and 72°C for 30 seconds for 40 cycles. Each Display molecule before reacting with the anti-ubiquitin antibody fixed beads was also subjected to qPCR in the same way, and the input nucleic acid amount was calculated.

To draw a calibration curve for each sequence, dilutions were made to be 1E + 8/μL, 1E + 6/μL, and 1E + 4/μL in nucleic acid amount, and subjected to qPCR under the same conditions.

The recovery rate was calculated from the formula below by dividing the recovery rate of the nucleic acids recovered by the anti-ubiquitin antibody by the input value (the amount of Display molecule input into the assay) and summarized in Table 12.

Apparent recovery rate (%) = recovered nucleic acid amount/input nucleic acid amount × 100

Recovery rate (%) = recovered nucleic acid amount/input nucleic acid amount × each display efficiency × 0.01) × 100

[Table 12]

| | Sample | | Input nucleic acid amount (molecules) | Recovered nucleic acid amount (molecules) | Apparent recovery rate (%) | Recovery rate (%) |
|---|---|---|---|---|---|---|
| Condition 1 | Kpep-splitUb (N) | Epep-splitUb (C) | 9.34E+11 | 2.53E+07 | 0.0027% | 0.0124% |
| Condition 2 | Epep-splitUb (N) | Epep-splitUb (C) | 7.89E+11 | 1.28E+06 | 0.0002% | 0.0007% |
| Condition 3 | Kpep-splitUb (N) | - | 4.59E+11 | 2.28E+06 | 0.0005% | 0.0008% |
| Condition 4 | Epep-splitUb (N) | - | 4.75E+11 | 3.77E+05 | 0.0001% | 0.0004% |
| Condition 5 | Epep-splitUb (C) | - | 3.14E+11 | 3.69E+06 | 0.0012% | 0.0037% |

As a result, the recovery rate was high only under the conditions of both Kpep-splitUb(N) and Epep-splitUb(C) are present (condition 1). From this, it was demonstrated that splitUb(N) and splitUb(C) are in proximity and can be recovered by an anti-ubiquitin antibody, only when the molecules added to splitUb(N) and splitUb(C) interact to form a complex.

Example 4-4: Complementation assay of split Nanoluc using mRNA Display

Nanoluc is a luminescent protein consisting of 10 β strands. There is a report of split Nanoluc system for detecting PPI by luminescence in which Nanoluc is divided into three of the 1-8th β-strand, the 9th β-strand, and the 10th β-strand, and the affinity is adjusted to perform the detection. In this system, similar to the split GFP system described above, the ninth and tenth strands are bound to the two proteins for which PPI is to be detected. Luminescence cannot be observed when the 9th or 10th strand alone and the 1-8th strand are mixed and a substrate is added. In contrast, luminescence can be observed when the two proteins are in proximity and a complex with the remaining 1-8th β strand is formed, and a substrate is added. Thus, the system has been used for wash-free ELISA and the like. Meanwhile, the extent of the binding of the 9th or 10th strand alone and the 1-8th strand has not been clarified, and it was necessary to verify whether they can be used for selective pull-downs.

Thus, verification was performed to ensure that the molecules linking the 9th and 10th strands by a linker can be pulled down with the 1-8th strand, while the 9th or 10th strand alone or a simple mixture of the these two cannot be pulled down with the 1-8th strand. Once this hypothesis is validated, a selective pull-down of the interest is possible. In this example, the 1-8th strand of Nanoluc to which an Avi tag sequence and a His tag sequence are added at the N-terminus is referred to as splitNanoluc1-8; the 9th strand to which a GS linker and a TEV protease recognition sequence is added to the C-terminus is referred to as splitNanoluc-9; the 10th strand in which a GS linker and a TEV protease recognition sequence are added at the C-terminus is referred to as splitNanoluc-10; and the protein in which splitNanoluc-9 and splitNanoluc-10 are linked by a GS linker, and to which a GS linker and a TEV protease recognition sequence are added to the C-terminus is referred to as splitNanoluc9-10.

Example 4-4-1: Preparation of biotinylated splitNanoluc1-8 protein

Vector Preparation

Expression vectors were prepared by inserting a synthetic DNA of SEQ ID NO: 58 (D-18) into pET-15b vectors using a Nco1/BamH1 restriction enzyme.

Synthetic DNA SEQ ID NO: 58 (D-18)

DNA sequence:

CCATGGGCCTGAACGATATTTTTGAAGCGCAGAAGATTGAGTGGCACGAACACCAT
CATCATCATCACGGTGGTAGCAGCGTGTTCACCCTGGAGGACTTTGTTGGCGATTGG
GAACAGACCGCGGCGTACAACCTGGACCAGGTGCTGGAGCAAGGTGGCGTTAGCAG
CCTGCTGCAGAACCTGGCGGTGAGCGTTACCCCGATCCAACGTATTGTTCGTAGCGG
CGAGAACGCGCTGAAGATCGACATTCACGTGATCATTCCGTATGAAGGCCTGAGCG
CGGATCAGATGGCGCAAATCGAGGAAGTGTTCAAGGTGGTTTACCCGGTTGACGAT
CACCACTTTAAAGTGATCCTGCCGTATGGTACCCTGGTGATTGACGGCGTTACCCCG
AACATGCTGAACTACTTCGGTCGTCCGTATGAAGGCATCGCGGTTTTTGATGGTAAG
AAAATTACCGTTACCGGCACCCTGTGGAACGGCAACAAGATTATTGACGAACGCCT
GATTACCCCGGACTAATGAGGATCC

Protein Expression and Purification

E. coli SHuffle T7 Express Competent E.coli (New England BioLabs, Cat.No C3029H) was transformed with a splitNanoluc expression vector and a BirA expression vector, in which the E. coli BirA gene was introduced into the pACYC184 vector, to construct an expression strain.

The constructed E. coli expression strain was cultured in medium A (LB medium (Invitrogen, #12780-052), 100 μg/mL ampicillin, 50 μg/mL spectinomycin dihydrochloride, 34 μg/mL chloramphenicol) until OD600 = 0.5 was reached, then a flask was placed in ice water for 5 minutes to reduce the temperature of the culture solution to 4°C. Isopropyl-β-thiogalactopyranoside (IPTG) was added at 200 μM and biotin was added at 50 μM, and then the resulted solution was cultured at 16°C, 200 rpm overnight to express a biotinylated modified protein of interest. The cultured bacteria were collected by centrifugation, frozen in liquid nitrogen, and then stored at -80°C.

The frozen bacteria were suspended using an extraction buffer (50 mM sodium hydrogen phosphate pH 7.5, 500 mM NaCl, Proteinase Inhibitor Cocktail (EDTA-free)), and rLysozyme (Novagen, #71110-3) was added at 5 kU/E.coli, and then the resultant solution was left at room temperature for 20 minutes. After resuspension and ultrasonic crushing, 1M MgCh was added at 0.2% v/v, and 25 U/μL benzonase (EMD Millipore, #70746-2.5KUN) was added at 0.03% v/v, and then the resultant was allowed to stand still again at room temperature for 20 minutes. The sample was centrifuged, and the collected supernatant was filtered through a 0.22 μm filter.

The filtered sample was purified using HisTrap HP 1 mL (GE Healthcare, #17-5247-01).

The purified samples were collected and purified with Streptavidin Mutein Matrix (Roche, #03708152001). Fractions containing proteins of interest were collected and concentrated with Amicon Ultra-15 10K (MERCK, #UFC801024) to a 1/10 volume.

The concentrated sample was purified by gel filtration. As a running buffer for purification by gel filtration, 50 mM Tris-HCl, 150 mM NaCl, 10% Glycerol, pH 8.0 was used.

The results of SDS-PAGE analysis showed a single band at the position corresponding to the theoretical molecular weight of the protein of interest. The biotinylated protein of interest was also confirmed by a gel shift assay based on the interaction between the biotinylated protein and streptavidin.

[Table 13]

| Lane | Sample name | Additive | Protein amount (μg) |
|---|---|---|---|
| 1 | Marker | - | - |
| 2 | SplitN anoluc 1-8 | Streptavidin | 1 |
| 3 | SplitN anoluc 1-8 | - | 1 |

(continued)

| Lane | Sample name | Additive | Protein amount (μg) |
|---|---|---|---|
| 4 | SplitN anoluc 1-8 | - | 0.1 |
| 5 | - | Streptavidin | - |

Example 4-4-2: Complementation assay of split Nanoluc using mRNA Display

Screening a library for a candidate molecule capable of forming a complex with a plurality of target molecules requires a system that suppresses the recovery rate of a test molecule that forms a complex with only one target molecule.

Thus, each binding ability of splitNanoluc-9 (SEQ ID NO: 66), splitNanoluc-10 (SEQ ID NO: 67), and splitNanoluc9-10 fusion peptide (SEQ ID NO: 65) to splitNanoluc1-8 (SEQ ID NO: 85) was evaluated. Display molecules in which a nucleic acid was linked to each of splitNanoluc-9, splitNanoluc-10, and splitNanoluc9-10 fusion peptide, were prepared, then incubated with splitNanoluc1-8, and the nucleic acid moiety of the molecule bound to splitNanoluc1-8 was subjected to qPCR.

(1) Synthesis of mRNA

mRNAs (SEQ ID NO: 62 (mR-14) to SEQ ID NO: 64 (mR-16)) were synthesized from template DNAs (SEQ ID NO: 59 (D-19) to SEQ ID NO: 61 (D-21)) by in vitro transcription reaction using T7 RNA polymerase and purified by RNeasy kit (QIAGEN N.V.).

Template DNA SEQ ID NO: 59 (D-19)

DNA sequence: splitNanoluc9-10

GGCGTAATACGACTCACTATAGGGTTAACTTTAAGAAGGAGATATACATATGGGCA
GCATGCTGTTTCGTGTGACCATTAACAGCGGCGGCAGCGGCGGCGGCGGCAGC
GGCGTGAGCGGCTGGCGTCTGTTTAAAAAAATTAGCGGTTCTGGTTCTGGTTCTGGT
TCTGAAAACCTGTATTTTCAGGGCAAAAAAA

Template DNA SEQ ID NO: 60 (D-20)

DNA sequence: splitNanoluc-9

GGCGTAATACGACTCACTATAGGGTTAACTTTAAGAAGGAGATATACATATGGGCA
GCATGCTGTTTCGTGTGACCATTAACAGCGGTTCTGGTTCTGGTTCTGGTTCTGAAAA
CCTGTATTTTCAGGGCAAAAAAA

Template DNA SEQ ID NO: 61 (D-21)

DNA sequence: splitNanoluc-10

GGCGTAATACGACTCACTATAGGGTTAACTTTAAGAAGGAGATATACATATGGTGAGCGGCTGGCGTCTGTTTAAAAAAATTAGCGGTTCTGGTTCTGGTTCTGGTTCTGAAAACCTGTATTTTCAGGGCAAAAAAA

mRNA SEQ ID NO: 62 (mR-14)

splitNanoluc9-10 RNA sequence:

GGGUUAACUUUAAGAAGGAGAUAUACAUAUGGGCAGCAUGCUGUUUCGUGUGACCAUUAACAGCGGCGGCGGCAGCGGCGGCGGCGGCAGCGGCGUGAGCGGCUGGCGUCUGUUUAAAAAAAUUAGCGGUUCUGGUUCUGGUUCUGGUUCUGAAAACCUGUAUUUUCAGGGCAAAAAAA

mRNA SEQ ID NO: 63 (mR-15)

splitNanoluc-9 RNA sequence:

GGGUUAACUUUAAGAAGGAGAUAUACAUAUGGGCAGCAUGCUGUUUCGUGUGACCAUUAACAGCGGUUCUGGUUCUGGUUCUGGUUCUGAAAACCUGUAUUUUCAGGGCAAAAAAA

mRNA SEQ ID NO: 64 (mR-16)

splitNanoluc-10 RNA sequence:

GGGUUAACUUUAAGAAGGAGAUAUACAUAUGGUGAGCGGCUGGCGUCUGUUUAAAAAAAUUAGCGGUUCUGGUUCUGGUUCUGGUUCUGAAAACCUGUAUUUUCAGGGCAAAAAAA

(2) Complementation assay by mRNA Display

Display molecules in which mRNA and a peptide are linked were prepared, and the recovery rate with biotinylated splitNanoluc1-8 was examined. In accordance with the method described in WO2013/100132, puromycin was linked via a linker to the 3' end of the mRNA purified above and then translated to prepare the Display molecules of mRNA-peptide. PUREfrex2.0 (GeneFrontier Corporation), a reconstituted cell-free protein synthesis system from a prokaryotic organism, was used as the translation system, and the translation was performed according to the attached protocol.

To ascertain the display efficiency of each Display molecule, the RNA portion was digested by RNaseONE (Promega Corporation) and electrophoretically separated with TricineP AGE. When the Display molecule is formed, a fusion molecule of the translated peptide and a puromycin linker is present. When the Display molecule is not formed, an unbound linker alone without binding of the translated peptide is present and detected at a position lower than the former. Thus, a total of two bands are detected. From the band ratio, the display efficiency (%) of the Display molecule was calculated by the following formula.

Display molecule display efficiency (%) = band intensity of translated peptide and linker/total band intensity × 100

For the specific reaction solution with RNaseONE, 1 × Reaction buffer, 0.84 μM fusion molecule, and 0.5 U/μL RNaseONE were mixed, and the mixture was allowed to react at 37°C for 1 hour.

Detection by TricinePAGE resulted in a detection of a fluorescently labeled puromycin linker. The display efficiency obtained from the result of TricinePAGE was as shown in Table 14.

[Table 14]

| SEQ ID NO | Peptide name | Amino acid sequence | Display efficiency |
|---|---|---|---|
| SEQ ID NO: 65 | SplitNanoluc9-10 | MGSMLFRVTINSGGGSGGGGSGVSGWRLFKKIS GSGSGSGSENLYFQGKK | 38% |
| SEQ ID NO: 66 | SplitN anoluc-9 | MGSMLFRVTINSGSGSGSGSENLYFQGKK | 16% |
| SEQ ID NO: 67 | SplitNanoluc-10 | MVSGWRLFKKISGSGSGSGSENLYFQGKK | 69% |

For complementation assays, non-fluorescently labeled puromycin linkers were used, and the translation products were reverse transcribed to avoid binding at the mRNA portion. Specifically, 1 × M-MLV RT Reaction buffer (Promega Corporation, #368B), 0.5 mM dNTP, 8 U/μL M-MLV RT (H-) Point Mutant (Promega Corporation, #368B), 3 μM Rv-primer (SEQ ID NO: 68), and 0.5 μM translation product were mixed, and the mixture was allowed to react at 42°C for 1 hour.

Each Display molecule was mixed with 1 μM biotinylated splitNanoluc1-8 per 33 nM Display molecule in a buffer of 1 × TBS, 2 mg/mL BSA (Invitrogen), and the mixture was allowed to react at room temperature for 1 hour.

The biotinylated splitNanoluc1-8 or a complex containing the same was recovered by pull-down with streptavidin-coated magnetic beads, washed several times with tTBS (NACALAI TESQUE, INC.), and then cleaved with a TEV protease that recognizes a TEV protease recognition sequence introduced between mRNA moieties encoding themselves linked to splitNanoluc-9 or splitNanoluc-10, to elute the nucleic acid to elute the nucleic acid. Specifically, 1 × TEV buffer and 1 mM DTT, 0.2 U/μL AcTEV protease (Thermo Fisher Scientific, Inc., #12575015) were added as a TEV eluate to the washed beads, and reacted. After the reaction, the supernatant was collected and subjected to qPCR with a complementary primer (Fw-primer (SEQ ID NO: 4), Rv-primer (SEQ ID NO: 68)). For the qPCR reaction solution, 1 × Ex taq buffer, 0.2 mM dNTP, 0.5 μM Fw-primer, 0.5 μM Rv-primer, 200,000 times dilution SYBR Green I (Lonza K.K., #50513), and 0.02 U/μL Ex Taq polymerase (Takara Bio Inc.) were mixed, and PCR was performed by heating at 95°C for 2 minutes, and then repeating the cycles of 95°C for 10 seconds, 57°C for 20 seconds, and 72°C for 30 seconds for 40 cycles. Each Display molecule before reacting with biotinylated splitNanoluc1-8 was also subjected to qPCR in the same way, and the input nucleic acid amount was calculated.

To draw a calibration curve for each sequence, dilutions were made to be 1E + 8/μL, 1E + 6/μL, and 1E + 4/μL in nucleic acid amount, and subjected to qPCR under the same conditions.

The apparent recovery rate obtained by dividing the amount of nucleic acid of each Display molecule recovered by binding to splitNanoluc1-8 by the amount of nucleic acid of each Display molecule that was present before mixing with splitNanoluc1-8 (input value) was calculated and corrected with the display efficiency to obtain the recovery rate, which was summarized in Table 15.

Apparent recovery rate (%) = recovered nucleic acid amount/input nucleic acid amount × 100

Recovery rate (%) = recovered nucleic acid amount/input nucleic acid amount × each display efficiency × 0.01) × 100

[Table 15]

| | Sequence | Input nucleic acid amount (molecules) | Recovered nucleic acid amount (molecules) | Apparent recovery rate (%) | Recovery rate (%) |
|---|---|---|---|---|---|
| 1 | splitNanoluc9-10 | 2.73E+10 | 3.45E+09 | 12.632% | 33.155% |
| 2 | splitNanoluc-9 | 3.23E+10 | 2.53E+06 | 0.008% | 0.049% |
| 3 | splitNanoluc-10 | 4.53E+10 | 5.04E+06 | 0.011% | 0.016% |
| 4 | splitNanoluc-9+splitNanoluc-10 | 5.75E+10 | 9.62E+06 | 0.017% | 0.104% |

As a result, the splitNanoluc9-10 fusion peptide was recovered by splitNanoluc1-8, and the recovery rate of splitNanoluc-9 alone or splitNanoluc-10 alone was 300 times or more lower than the recovery rate of the fusion peptide. Thus, it was shown that Nanoluc1-8 does not associate with Nanoluc-10 (or Nanoluc-9) in the absence of Nanoluc-9 (or Nanoluc-10), and that Nanoluc-9 and Nanoluc-10 must be in proximity, association, or interaction so that Nanoluc1-8 associates with Nanoluc-9 and Nanoluc-10. This result showed that the use of molecules binding splitNanoluc-9 to one target protein, molecules binding splitNanoluc-10 to another target protein, and splitNanoluc1-8 allows for efficient screening for molecules capable of forming a complex with a plurality of target molecules.

Example 5: Validation experiment of rapamycin-nucleic acid enrichment in peptide library

In the above-described rapamycin recovery validation experiment using GFP complementation, it was shown that rapamycin can be efficiently recovered using the three of GFP10, GFP11, and GFP1-9. Thus, a mixed library in which the rapamycin-nucleic acid (compound nk04) was mixed into the peptide display library in a ratio of 1/(1 × 10 to the third power) or 1/(1 × 10 to the sixth power) was prepared, and a round of mRNA Display panning operation was performed to verify whether the rapamycin-nucleic acid of interest could be enriched.

Preparation of peptide display library

Synthesis of acylated tRNA for use in translation

The acylated tRNA used for panning was prepared by the method described in WO2013/100132. An Elongator aminoacylated tRNA mixture was prepared using 15 amino acids including MeHnl7F2, S3F5MePyr, MeSnPr, MeA3Pyr, F3Cl, Pic2, MeG, Nle, MeStBuOH, SNtBuAca, SiPen, nBuG, MeHph, SPh2C, Ala (4-Thz), as described in WO2018/225864. The final concentration of each acylated tRNA in the translation solution was from 10 μM to 20 μM. Pnaz-protected pCpA amino acids were subjected to phenol extraction process or thereafter without deprotection. The Initiator aminoacylated tRNA was the same compound as the compound AAtR-3 (Acbz-MeCys(StBu)-tRNAfMetCAU) described in WO2017/150732, and was added to the translation solution so that the final concentration was 25 μM.

Randomized double-stranded DNA library encoding peptide compound library

A DNA library was constructed by the method described in WO2013/100132. The DNA library prepared included twenty-four triplets, including TTT, TTG, CTT, ATT, ATG, GTT, CCG, Act, GCT, cat, CAG, AAC, GAA, TGG, CGG, AGT, AGG, GGT, CTG, GTG, TCT, TCG, TGC, and CGT, which randomly appeared eight or nine times.

PURE system, a reconstituted cell-free protein synthesis system derived from a prokaryotic organism, was used as the translation system. Specifically, 1 mM GTP, 1 mM ATP, 20 mM creatine phosphate, 50 mM HEPES-KOH pH 7.6, 100 mM potassium acetate, 10 mM magnesium acetate, 2 mM spermidine, 1 mM dithiothreitol, 1 mg/mL E. coli MRE600 (RNase negative)-derived tRNA (F. Hoffmann-La Roche, Ltd.) (some tRNA has been removed in accordance with the method described in a Non Patent Literature, Yokogawa T et al. 2010), 4 μg/mL creatine kinase, 3 μg/mL myokinase, 2 unit/mL inorganic pyrophosphatase, 1.1 μg/mL nucleoside diphosphate kinase, 0.26 μM EF-G, 2.7 μM IF1, 0.4 μM IF2, 1.5 μM IF3, 40 μM EF-Tu, 35 μM EF-Ts, 1.2 μM ribosomes, 2.73 μM AlaRS, 1 μM GlyRS, 0.4 μM IleRS, 0.5 μM PheRS (WO2016/148044), 0.16 μM ProRS, 1 μM SerRS (WO2016/148044), 0.09 μM ThrRS, 1 μM ValRS (WO2016/148044), 0.11 μM LysRS, 0.5 μM HisRS, 3 μM in vitro transcribed E. coli tRNA Ala1B, 250 μM glycine, 100 μM isoleucine, 250 μM proline, 250 μM threonine, 250 μM lysine, 5 mM N-methylvaline, 5 mM N-methylserine, 5 mM N-methylalanine, 5 mM N-methylphenylalanine, 1 mM Ala (4-Thz), Elongator aminoacylated tRNA mixture, 25 μM Initiator aminoacylated tRNA (WO2020/138336A1), 0.5 μM Penicillin G Amidase (PGA), 1 μM EF-P-Lys, and 1 μM

mRNA-puromycin linked compound prepared according to WO2013/100132 from the aforementioned double-stranded DNA library were added to the translation reaction mixture, and allowed to stand still at 37°C for 1 hour to perform translation.

A mixture in which a rapamycin-nucleic acid (compound nk04) was added to the translated library in a ratio of 1/(1 × 10 to the third power) as the molecular number of the nucleic acid was prepared as Mixed Library A, and a mixture in which a rapamycin-nucleic acid (compound nk04) was added to the translated library in a ratio of 1/(1 × 10 to the sixth power) was prepared as Mixed Library B. Panning was performed using Mixed Library A and Mixed Library B for one round in accordance with WO2013/100132.

In condition 1, Mixed Library A was added at 0.3 μM to 2 μM Hisx6-FKBP12-Link12-GFP11, 2 μM GFP10-Link12-FRB-Hisx6, and 1 μM biotinylated GFP1-9 in a buffer of 1 × TBS, 2 mg/mL BSA (Invitrogen), and the mixture was allowed to react at room temperature for 1.5 hours.

In condition 2, Mixed Library B was added at 0.3 μM to 2μM Hisx6-FKBP12-Link12-GFP11, 2μM GFP10-Link12-FRB-Hisx6, and 1 μM biotinylated GFP1-9 in a buffer of 1 × TBS, 2 mg/mL BSA (Invitrogen), and the mixture was allowed to react at room temperature for 1.5 hours.

The biotinylated GFP1-9 or a complex containing the same was recovered by pull-down with Streptavidin-coated magnetic beads, washed several times with tTBS (NACALAI TESQUE, INC.), and then treated with a TEV protease that recognizes and cleaves a TEV protease recognition sequence introduced between GFP1-9 and biotin to elute the nucleic acid. Specifically, 1 × TEV buffer and 1 mM DTT, 0.2 U/μL AcTEV protease (Thermo Fisher Scientific, Inc., #12575015) were added as a TEV eluate to the washed beads, and reacted. After the reaction, the supernatant was recovered and PCR was performed with a primer (Fw-primer (SEQ ID NO: 117), Rv-primer (SEQ ID NO: 118)) having complementarity to the entire sequences of library A (or B) (including compound nk04-added nucleic acid sequence), and then qPCR of the PCR product was performed to evaluate the sequence of the library. However, it has been confirmed that there is no difference in the PCR amplification efficiency when the same primer is used between the compound nk04-added nucleic acid sequence and the other sequences from the library (data are not shown). As the primers for qPCR, the aforementioned primers (Fw-primer (SEQ ID NO: 4), Rv-primer (SEQ ID NO: 6)) or primers having complementarity only to the compound nk04-added nucleic acid sequence ((Fw-primer (SEQ ID NO: 69), Rv-primer (SEQ ID NO: 70)) were used. For the qPCR reaction solution, 1 × Extaq buffer, 0.2 mM dNTP, 0.5 μM Fw-primer, 0.5 μM Rv-primer, 200,000 times dilution SYBR Green I (Lonza K.K., 50513), and 0.02 U/μL Ex Taq polymerase (Takara Bio Inc.) were mixed, and PCR was performed by heating at 95°C for 2 minutes, and then repeating the cycles of 95°C for 10 seconds, 57°C for 20 seconds, and 72°C for 30 seconds for 40 cycles.

To draw a calibration curve for each sequence, dilutions were made to be 1E + 8/μL, 1E + 6/μL, and 1E + 4/μL in input nucleic acid amount, and subjected to qPCR under the same conditions.

By comparing the nucleic acid recovery rate of compound nk04 to the total nucleic acid recovery rate of library A (or B), the molecular concentration (%) of compound nk04 in the library after one round of panning was calculated by the following formula (Table 16).

Molecular concentration of compound nk04 (%) = compound nk04-added nucleic acid sequence nucleic acid concentration (number of molecules/μL)/nucleic acid concentration (number of molecules/μL) of library × 100

[Table 16]

| Condition | Library | One round of panning | Nucleic acid concentration (number of molecules/μL) of library | Compound nk04-added nucleic acid sequence nucleic acid concentration (number of molecules/μL) | Molecular concentration of compound nk04 (%) |
|---|---|---|---|---|---|
| Condition 1 | A | None | 9.02E+10 | 2.48E+08 | 0.2751% |
| Condition 2 | B | None | 9.58E+10 | 7.49E+05 | 0.0008% |
| Condition 1 | A | Done | 5.25E+12 | 6.39E+12 | 121.6000% |

(continued)

| Condition | Library | One round of panning | Nucleic acid concentration (number of molecules/μL) of library | Compound nk04-added nucleic acid sequence nucleic acid concentration (number of molecules/μL) | Molecular concentration of compound nk04 (%) |
|---|---|---|---|---|---|
| Condition 2 | B | Done | 5.53E+12 | 1.43E+11 | 2.6000% |

As a result, it was revealed that the molecular concentration of compound nk04 in the library was 400 to 3,000 times between before and after panning, and that mRNA display panning using splitGFP efficiently enriched molecules recognizing the two targets.

Example 6: Panning of bispecific molecule using splitGFP techniques

As described above, it was shown that the rapamycin-nucleic acid mixed with the peptide display library is enriched by the complementation of GFP. Next, verification was performed by mRNA display panning to determine whether peptides that bind to two target proteins can be obtained from a peptide display library of 1 × 10 to the 12th power using the same method.

In the first round of panning, a selection was performed with a biotinylated version of one target protein being fixed on beads and the other target protein being present in a free state. In the second round, a selection was performed after exchanging the biotinylated target proteins fixed on beads with the free target proteins.

At the third round or thereafter, fusion proteins were prepared by binding respective target proteins to GFP 10 and GFP 11, and selection was performed in the presence of these two fusion proteins and biotinylated GFP1-9.

Example 6-1: Preparation of Panning Target Protein

GTPase KRas (KRAS) that was expressed and purified by Escherichia coli was used as the target protein for panning. KRAS-FLAGSorHisTEV-Bio (SEQ ID NO: 100), in which a sortase recognition sequence (LPXTG sequence (X is any amino acid) (SEQ ID NO: 116)), a His tag, a TEV protease cleavage tag, and a biotinylated enzyme-recognition tag were added to the C-terminus, was prepared.

Specifically, Escherichia coli BL2 (DE3) was transformed with a biotin lyase (BirA) expression vector using pET21a (+) to create an expression strain. The cells were cultured in an LB medium containing ampicillin and chloramphenicol until OD600 reached 0.7 and induced with 1 mM IPTG, and cultured at 30°C for 5 hours in the presence of 0.1 mM biotin. The collected soluble fraction was purified using Ni Sepharose High Performance (GE) followed by purification using Streptavidin Mutein Matrix (F. Hoffmann-La Roche, Ltd.). Purification was performed according to the basic protocol attached to each product. Size fraction chromatography was performed using Superdex 75 Increase 10/300 GL (GE), and the purified final product was placed in a storage buffer (25 mM HEPES, 150 mM NaCl, 1 mM $MsCl_2$, 1 mM DTT, 10% Glycerol, pH 7.6) and stored at -80°C.

The results of SDS-PAGE analysis showed a single band at the position corresponding to the theoretical molecular weight of the protein of interest. The result of SEC analysis confirmed that the protein of interest had a retention time equivalent to a molecular weight marker near the theoretical value and was present as a monomer. TSKgel G2000 SWXL (TOSOH, #08540) was used as the column for SEC analysis.

As a target protein for use in panning, HAT-KRAS-nonbio (SEQ ID NO: 101) in which a purified tag, a thronbin cleavage site were added to the N-terminus was prepared.

Specifically, pETBlue1 was used as an expression vector, and E. coli Tuner(DE3)pLacI (Novagen) was transformed to create an expression strain. The cells were cultured in an LB medium containing ampicillin until OD600 reached 0.6, and induced with 0.1 mM IPTG, and cultured at 30°C overnight. The collected soluble fractions were purified using Ni Sepharose excel (GE Healthcare) according to the basic protocol. Size fraction chromatography was performed using HiLoad 26/600 Superdex 75 pg (GE Healthcare), and the purified final product was placed in a storage buffer (20 mM HEPES, 100 mM NaCl, 1 mM DTT, 5 mM MgCl2, pH 8.0) and stored at -80°C.

The results of SDS-PAGE analysis showed a single band at the position corresponding to the theoretical molecular weight of the protein of interest.

Glutathione S-transferase (GST) which was expressed and purified by Escherichia coli was used as the target protein for panning. GST-HisTEVAvi (SEQ ID NO: 103) in which a purified tag, a TEV protease cleavage tag, and a biotinylated enzyme-recognition tag were added to the C-terminus was prepared.

Specifically, pGEX-4T-1 was used as an expression vector, and E. coli BL21 star (DE3) was transformed to

create an expression strain. The cells were cultured in a TB medium until OD600 reached 1.2, and induced with 0.5 mM IPTG, and cultured at 37°C for 4 hours. The collected soluble fractions were purified using Profinity IMAC Ni-Charged Resin (BIO-RAD) according to the basic protocol attached to the product, and then placed in a storage buffer (50 mM Tris-HCl, 150 mM NaCl, 10% glycerol, pH 8.0) and stored at -80°C.

The results of SDS-PAGE analysis showed a single band at the position corresponding to the theoretical molecular weight of the protein of interest.

The GST-HisTEV-Avi prepared above was treated with biotin ligase (BirA) in the presence of biotin to obtain GST-HisTEV-Bio (SEQ ID NO: 102).

BirA was prepared and purified by E. coli. Specifically, pET30a was used as an expression vector, and E. coli BL21 (DE3) was transformed to create an expression strain. The cells were cultured in a TB medium containing ampicillin until OD600 reached 4, induced with 0.5 mM IPTG, and cultured at 15°C for 16 hours. The collected soluble fractions were purified using Profinity IMAC Ni-Charged Resin (BIO-RAD) according to the basic protocol, and then placed in a storage buffer (50 mM Tris-HCl, 150 mM NaCl, 10% glycerol, pH 8.0) and stored at -80°C.

The biotinylation reaction solution was prepared to 46 mM Tris HCl pH 8.3, 9 mM Mg(OAc)2, 35 μM biotin, 0.825 μM BirA, 30.5 μM GST-HisTev-Avi, and allowed to stand still at room temperature for 1 hour. The biotinylated protein of interest was also confirmed by a gel shift assay based on the interaction between the biotinylated protein and streptavidin.

Glutathione S-transferase (GST) which was expressed and purified by Escherichia coli was used as the target protein for panning. GST-SorAHis (SEQ ID NO: 104) in which a His tag for purification and a sortase recognition sequence were added to the C-terminus was prepared.

Specifically, pGEX-4T-1 was used as an expression vector, and E. coli BL21 Star (DE3) was transformed to create an expression strain. The cells were cultured in an LB medium containing ampicillin until OD600 reached 1.2, induced with 0.5 mM IPTG, and cultured at 15°C overnight. The collected soluble fractions were purified using Profinity IMAC Ni-Charged Resin (BIO-RAD) according to the basic protocol attached to the product, and then placed in a storage buffer (50 mM Tris-HCl, 150 mM NaCl, 10% glycerol, pH 8.0) and stored at -80°C.

The results of SDS-PAGE analysis showed a single band at the position corresponding to the theoretical molecular weight of the protein of interest.

FKBP12 expressed and purified by Escherichia coli was used as the target protein for panning. FKBP-HisTEVAvi (SEQ ID NO: 114) in which a purified tag, a TEV protease cleavage tag, and a biotinylated enzyme-recognition tag were added to the C-terminus was prepared. Specifically, pET11a was used as an expression vector, and E. coli BL21 (DE3) was transformed to create an expression strain. The cells were cultured in an LB medium containing ampicillin until OD600 reached 0.5, induced with 1.0 mM IPTG, and cultured at 37°C for 3 hours. The collected soluble fraction was purified by using His Trap FF (Cytiva) according to the basic protocol attached to the product, then concentrated with Amicon Ultra-15 10k cut(Merck Millipore), treated through 0.22 μm filter and purified by SEC with Superdex 75pg column (Cytiva). The resultant was paced in a storage buffer (50 mM Tris, pH 7.5, 150 mM NaCl, 1 mM TCEP) and stored at -80°C.

The results of SDS-PAGE analysis showed a single band at the position corresponding to the theoretical molecular weight of the protein of interest.

The FKBP-HisTEV-Avi prepared above was treated with biotin ligase (BirA) in the presence of biotin to obtain FKBP-HisTEV-Bio (SEQ ID NO: 115). The biotinylated protein of interest was also confirmed by a gel shift assay based on the interaction between the biotinylated protein and streptavidin.

The sequences of the prepared proteins described above are shown in the table below. "K*" represents biotinylated Lys.

[Table 17]

| SEQ ID NO | Protein name | Amino acid sequence |
|---|---|---|
| SEQ ID NO: 100 | KRAS-FLAGS or HisTEV-Bio | MTEYKLVVVGADGVGKSALTIQLIQNHFVDEYDPTIEDSYRKQVVIDGET CLLDILDTAGQEEYSAMRDQYMRTGEGFLCVFAINNTKSFEDIHHYREQIK RVKDSEDVPMVLVGNKCDLPSRTVDTKQAQDLARSYGIPFIETSAKTRQG VDDAFYTLVREIRKHKEKMSKDGGSSDYKDDDDKGGSSLPMTGGGGSSH HHHHHSSGENLYFQGSGGGLNDIFEAQK*IEWHE |

(continued)

| SEQ ID NO | Protein name | Amino acid sequence |
|---|---|---|
| SEQ ID NO: 101 | HAT-KRAS-nonbio | MKDHLIHNVHKEEHAHAHSSGSLVPRGSSGGSTEYKLVVVGADGVGKSALTIQLIQNHFVDEYDPTIEDSYRKQVVIDGETCLLDILDTAGQEEYSAMRDQYMRTGEGFLCVFAINNTKSFEDIHHYREQIKRVKDSEDVPMVLVGNKCDLPSRTVDTKQAQDLARSYGIPFIETSAKTRQGVDDAFYTLVREIRKHKEKMSKDG |
| SEQ ID NO: 102 | GST-HisTEV-Bio | MSPILGYWKIKGLVQPTRLLLEYLEEKYEEHLYERDEGDKWRNKKFELGLEFPNLPYYIDGDVKLTQSMAIIRYIADKHNMLGGCPKERAEISMLEGAVLDIRYGVSRIAYSKDFETLKVDFLSKLPEMLKMFEDRLCHKTYLNGDHVTHPDFMLYDALDVVLYMDPMCLDAFPKLVCFKKRIEAIPQIDKYLKSSKYIAWPLQGWQATFGGGDHPPKSDGGSSHHHHHHSSGENLYFQGSGGGLNDIFEAQK*IEWHE |
| SEQ ID NO: 103 | GST-HisTEVA vi | MSPILGYWKIKGLVQPTRLLLEYLEEKYEEHLYERDEGDKWRNKKFELGLEFPNLPYYIDGDVKLTQSMAIIRYIADKHNMLGGCPKERAEISMLEGAVLDIRYGVSRIAYSKDFETLKVDFLSKLPEMLKMFEDRLCHKTYLNGDHVTHPDFMLYDALDVVLYMDPMCLDAFPKLVCFKKRIEAIPQIDKYLKSSKYIAWPLQGWQATFGGGDHPPKSDGGSSHHHHHHSSGENLYFQGSGGGLNDIFEAQKIEWHE |
| SEQ ID NO: 104 | GST-SrtAHis | MSPILGYWKIKGLVQPTRLLLEYLEEKYEEHLYERDEGDKWRNKKFELGLEFPNLPYYIDGDVKLTQSMAIIRYIADKHNMLGGCPKERAEISMLEGAVLDIRYGVSRIAYSKDFETLKVDFLSKLPEMLKMFEDRLCHKTYLNGDHVTHPDFMLYDALDVVLYMDPMCLDAFPKLVCFKKRIEAIPQIDKYLKSSKYIAWPLQGWQATFGGGDHPPKSDGGSSDYKDDDDKGGSSLPMTGGGGSSHHHHHHHH |
| SEQ ID NO: 114 | FKBP-HisTEVA vi | MGVQVETISPGDGRTFPKRGQTCVVHYTGMLEDGKKFDSSRDRNKPFKFMLGKQEVIRGWEEGVAQMSVGQRAKLTISPDYAYGATGHPGIIPPHATLVFDVELLKLEGGSSLPMTGGGGSSHHHHHHSSGENLYFQGSGGGLNDIFEAQKIEWHE |
| SEQ ID NO: 115 | FKBP-HisTEV-Bio | MGVQVETISPGDGRTFPKRGQTCVVHYTGMLEDGKKFDSSRDRNKPFKFMLGKQEVIRGWEEGVAQMSVGQRAKLTISPDYAYGATGHPGIIPPHATLVFDVELLKLEGGSSLPMTGGGGSSHHHHHHSSGENLYFQGSGGGLNDIFEAQK*IEWHE |

Example 6-2: Preparation of GFP10 or GFP11-linked target proteins

GFP10 or GFP11 was added to a construct of KRAS-SorHisTEVAvi and GST-SorAHis, which contains a sortase recognition sequence at the C-terminus and prepared by the method described above, by a sortase reaction using eSrtA that is a transpeptidase that recognizes the LPXTG sequence (X is any amino acid) to prepare a fusion protein.

Example 6-2-1: Preparation of Sortase A pentamutant (eSrtA)

eSrtA was expressed in E. coli and purified according to the method of Non Patent Literature: Proc. Natl. Acad. Sci. USA. 2011 Jul 12; 108(28): 11399-11404. Note that the synthesized sortase A here is not a wild-type but a P94R/D160N/D165A/K190E/K196T pentamutant (Sortase A pentamutant (eSrtA)) (SEQ ID NO: 105) having higher activity.

[Table 18]

| SEQ ID NO | Protein name | Amino acid sequence |
|---|---|---|
| SEQ ID NO: 105 | eSrtA | MQAKPQIPKDKSKVAGYIEIPDADIKEPVYPGPATREQLNRGVSFAEENESLDDQNISIAGHTFIDRPNYQFTNLKAAKKGSMVYFKVGNETRKYKMTSIRNVKPTAVEVLDEQKGKDKQLTLITCDDYNEETGVWETRKIFVATEVKLEHHHHHH |

Example 6-2-2: Synthesis of tri-Gly-added GFP peptide

A GFP peptide (Sor-sp11-L12, SEQ ID NO: 106 and Sor-sp10-12, SEQ ID NO: 107) containing Gly-Gly-Gly which is a substrate for sortase at the N-terminus was synthesized in a known manner by a solid-phase synthesis method.

[Table 19]

| SEQ ID NO | Peptide name | Amino acid sequence |
|---|---|---|
| SEQ ID NO: 106 | Sor-sp11-L12 | GGGGSGGGSGGSTSEKRDHMVLLEYVTAAGITDAS |
| SEQ ID NO: 107 | Sor-sp10-12 | GGGGSGGGSGGSMDLPDDHYLSTQTILLKDLN |

Example 6-2-3: Addition of GFP10 or GFP11 to target protein by sortase reaction

A fusion protein linking KRAS and GFP10 (KRAS-sp10, amino acid sequence SEQ ID NO: 109), a fusion protein linking KRAS and GFP11 (KRAS-sp11, amino acid sequence SEQ ID NO: 108), and a fusion protein linking GST and GFP10 (GST-sp10, amino acid sequence SEQ ID NO: 110) were prepared.

Specifically, 40 μM KRAS-SorTEVHisAvi, 1 mM Sor-sp10-12 (or 1 mM Sor-sp11-L12) and 1 μM eSrtA were mixed in the reaction Buffer (0.05 M Tris-HCl (pH 7.5), 0.15 M NaCl, 10 mM CaCl2) on ice and incubated at 4°C overnight. To the reacted solution, 20 mM EDTA was added to stop the reaction.

Similarly, the same reaction solution was prepared for GSTSorAHis, and GFP10 was added. The reaction was incubated at 37°C for 2 hours.

Samples before and after the sortase reaction were analyzed by SDS-PAGE to confirm that the reaction proceeded normally by checking the gel shift corresponding to the theoretical molecular weight of the protein of interest. The full-length sequences of the target proteins obtained in these reactions are shown in the table below.

[Table 20]

| SEQ ID NO | Protein name | Amino acid sequence |
|---|---|---|
| SEQ ID NO: 108 | KRAS-sp11 | MTEYKLVVVGADGVGKSALTIQLIQNHFVDEYDPTIEDSYRKQVVIDGETCLLDILDTAGQEEYSAMRDQYMRTGEGFLCVFAINNTKSFEDIHHYREQIKRVKDSEDVPMVLVGNKCDLPSRTVDTKQAQDLARSYGIPFIETSAKTRQGVDDAFYTLVREIRKHKEKMSKDGGSSDYKDDDDKGGSSLPMTGGGGSGGGSGGSTSEKRDHMVLLEYVTAAGITDAS |
| SEQ ID NO: 109 | KRAS-sp10 | MTEYKLVVVGADGVGKSALTIQLIQNHFVDEYDPTIEDSYRKQVVIDGETCLLDILDTAGQEEYSAMRDQYMRTGEGFLCVFAINNTKSFEDIHHYREQIKRVKDSEDVPMVLVGNKCDLPSRTVDTKQAQDLARSYGIPFIETSAKTRQGVDDAFYTLVREIRKHKEKMSKDGGSSDYKDDDDKGGSSLPMTGGGGSGGGSGGSMDLPDDHYLSTQTILLKDLN |
| SEQ ID NO: 110 | GST-sp10 | MSPILGYWKIKGLVQPTRLLLEYLEEKYEEHLYERDEGDKWRNKKFELGLEFPNLPYYIDGDVKLTQSMAIIRYIADKHNMLGGCPKERAEISMLEGAVLDIRYGVSRIAYSKDFETLKVDFLSKLPEMLKMFEDRLCHKTYLNGDHVTHPDFMLYDALDVVLYMDPMCLDAFPKLVCFKKRIEAIPQIDKYLKSSKYIAWPLQGWQATFGGGDHPPKSDGGSSDYKDDDDKGGSSLPMTGGGGSGGGSGGSMDLPDDHYLSTQTILLKDLN |

Example 6-3: Preparation of peptide display library

Example 6-3-1: Synthesis of acylated tRNA for use in translation

The acylated tRNA used for panning was prepared by the method described in WO2018/143145 and WO2018/225864. Elongator aminoacylated tRNA mixtures were prepared using 19 amino acids including Pro(4-pip-4-F2), Asp(SMe), Hph(3-Cl), cisPro(4-pip-4-F2), MeHnl(7-F2), Ser(3-F-5-Me-Pyr), MeSer(nPr), MeAla(3-Pyr), Phe(3-Cl), Pic(2), MeGly, Nle, MeSer(tBuOH), Ser(NtBu-Aca), Ser(iPen), nBuGly, MeHph, Ser(Ph-2-Cl). The final concentration of each acylated tRNA in the translation solution was from 10 μM to 20 μM. Pnaz-protected pCpA amino acids were subjected to phenol extraction process or thereafter without deprotection. The Initiator aminoacylated tRNA was the same compound as the compound AAtR-18 (BdpFL-Phe-tRNA(fMet)cau) described in WO2020/138336, and was added to the translation solution so that the final concentration was 25 μM.

Example 6-3-2: Randomized double-stranded DNA library encoding peptide library

A DNA library was constructed in accordance with the method described in WO2013/100132. The DNA library prepared included twenty-six triplets, including TTT, TTG, CTT, CTG, ATT, ATG, GTT, GTG, TCT, TCG, CCG, Act, GCT, TAC, CAT CAG, AAC, GAT, GAG, TGC, TGG, CGT, CGG, AGT, AGG, and GGT, which randomly appeared eight or nine times. (SEQ ID NO: 111, 119)

DNA Library (SEQ ID NO: 111)

GTAATACGACTCACTATAGGGTTAACTTTAATAAGGAGATATAAATATG(Random_triplet)$_9$TAGCCGACCGGCACCGGCACCGGCAAAAAAA

DNA Library (SEQ ID NO: 119)

GTAATACGACTCACTATAGGGTTAACTTTAATAAGGAGATATAAATATG(Random_triplet)$_8$TAGCCGACCGGCACCGGCACCGGCAAAAAAA

Example 6-3-3: Translation of peptide display library

PURE system, a reconstituted cell-free protein synthesis system derived from a prokaryotic organism, was used as the translation system. Specifically, 1 mM GTP, 1 mM ATP, 20 mM creatine phosphate, 50 mM HEPES-KOH pH 7.6, 100 mM potassium acetate, 1 mM magnesium acetate, 2 mM spermidine, 1 mM dithiothreitol, 1 mg/mL E. coli MRE600 (RNase negative)-derived tRNA (F. Hoffmann-La Roche, Ltd.) (some tRNA has been removed in accordance with the method described in a Non Patent Literature, Yokogawa T et al. 2010), 4 μg/mL creatine kinase, 3 μg/mL myokinase, 2 unit/mL inorganic pyrophosphatase, 1.1 μg/mL nucleoside diphosphate kinase, 0.26 μM EF-G, 2.7 μM IF1, 0.4 μM IF2, 1.5 μM IF3, 40 μM EF-Tu, 59 μM EF-Ts, 0.4 U/μL RNasein, 1.2 μM ribosomes, 1 μM EF-P-Lys, 2.73 μM AlaRS, 1 μM GlyRS, 0.4 μM IleRS, 0.5 μM PheRS (WO2016/148044), 0.16 μM ProRS, 1 μM SerRS (WO2016/148044), 0.09 μM ThrRS, 1 μM ValRS (WO2016/148044), 0.11 μM LysRS, 3 μM in vitro transcribed E. coli tRNA Ala1B, 250 μM glycine, 100 μM isoleucine, 250 μM proline, 250 μM threonine, 250 μM lysine, 5 mM N-methylvaline, 5 mM N-methylserine, 5 mM N-methylalanine, 5 mM N-methylphenylalanine, Elongator aminoacylated tRNA mixture, 25 μM Initiator aminoacylated tRNA (AAtR-3 (Acbz-MeCys(StBu)-tRNAfMetCAU), a compound described in WO2017/150732), 0.5 μM Penicillin G Amidase (PGA), and 1 μM mRNA-puromycin linked compound prepared according to WO2013/100132 from the aforementioned double-stranded DNA library were added to the translation reaction mixture, and allowed to stand still at 37°C for 1 hour to perform translation.

Example 6-4: Panning

Panning was performed using the translated display library for five rounds in accordance with WO2013/100132.

Example 6-4-1: Panning using GST and KRas as target proteins

In round 1, GST-HisTev-Bio was fixed to the Streptavidin-coated magnetic beads at 0.4 μM, and the display library was added to a buffer of 1 × TBS, 2 mg/mL BSA (Invitrogen) at 0.6 μM, and they were reacted at 4°C for 2 hours in the presence of 4 μM HAT-KRAS-nonbio, 0.4 mM GDP.

After the reaction, the beads collected with a magnet and removed from the supernatant were washed several times with tTBS (NACALAI TESQUE, INC.) added at 2 mM DTT, and then the beads were treated with TEV protease that recognizes and cleaves the TEV protease sequence to elute the nucleic acid.
Specifically, 1 × TEV buffer and 10 mM DTT, 0.1 U/μL AcTEV protease (Thermo Fisher Scientific, Inc., #12575015) were added as a TEV eluate to the washed beads, and reacted. After the reaction, the supernatant was collected and subjected to PCR with the library recognition primer (Fw-primer (SEQ ID NO: 112), Rv-primer (SEQ ID NO: 113)) to obtain an input library for the next round.
In addition, qPCR was performed using a portion of the TEV elution, and the recovery rate of the library for each round was evaluated. The aforementioned primers (Fw-primer (SEQ ID NO: 112), Rv-primer (SEQ ID NO: 113)) were used as the primer for qPCR. For the qPCR reaction solution, 1 × Ex taq buffer, 0.2 mM dNTP, 0.5 μM Fw-primer, 0.5 μM Rv-primer, 200,000 times dilution SYBR Green I (Lonza K.K., 50513), and 0.02 U/μL Ex Taq polymerase (Takara Bio Inc.) were mixed, and PCR was performed by heating at 95°C for 2 minutes, and then repeating the cycles of 95°C for 10 seconds, 57°C for 20 seconds, and 72°C for 30 seconds for 40 cycles.

In round 2, KRAS-FLAG-Sor-His-Tev-Bio was fixed to the Streptavidin-coated magnetic beads at 0.4 μM, and the display library was added to a buffer of 1 × TBS, 2 mg/mL BSA (Invitrogen) at 0.3 μM, and they were reacted at 4°C for 2 hours in the presence of 4 μM GST-HisTevAvi, 0.4 mM GDP.

Thereafter, the same work as in round 1 was performed except that the DTT concentration of the TEV eluate was changed to 1 mM.

In rounds 3 to 5, display Library was added at 0.3 μM to 0.8 μM KRAS-sp11, 0.8 μM GST-sp10, and 1 μM biotinylated GFP1-9 in a buffer of 1 × TBS, 2 mg/mL BSA (Invitrogen), and the mixture was allowed to react at 4°C for 2 hours.

The biotinylated GFP1-9 or a complex containing the same was recovered by pull-down with Streptavidin-coated magnetic beads, washed several times with tTBS (NACALAI TESQUE, INC.), and then treated with a TEV protease that recognizes and cleaves a TEV protease recognition sequence introduced between GFP1-9 and biotin to elute the nucleic acid. Specifically, 1 × TEV buffer and 1 mM DTT, 0.1 U/μL AcTEV protease (Thermo Fisher Scientific, Inc., #12575015) were added as a TEV eluate to the washed beads, and reacted. After the reaction, the supernatant was collected and subjected to PCR with the library recognition primer (Fw-primer (SEQ ID NO: 112), Rv-primer (SEQ ID NO: 113)) to obtain an input library for the next round.

In addition, qPCR was performed using a portion of the TEV elution, and the recovery rate of the library was evaluated. The aforementioned primers (Fw-primer (SEQ ID NO: 112), Rv-primer (SEQ ID NO: 113)) were used as the primer for qPCR. For the qPCR reaction solution, 1 × Extaq buffer, 0.2 mM dNTP, 0.5 μM Fw-primer, 0.5 μM Rv-primer, 200,000 times dilution SYBR Green I (Lonza K.K., 50513), and 0.02 U/μL Ex Taq polymerase (Takara Bio Inc.) were mixed, and PCR was performed by heating at 95°C for 2 minutes, and then repeating the cycles of 95°C for 10 seconds, 57°C for 20 seconds, and 72°C for 30 seconds for 40 cycles.

To draw a calibration curve to estimate the recovery rate of the library, the input library was diluted to be 1E + 8/μL, 1E + 6/μL, and 1E + 4/μL in input nucleic acid amount, and subjected to qPCR under the same conditions.

At the second round or thereafter, the input library was divided into two, and selection was made with one in the presence of the target protein and the other in the absence of the target protein, and the nucleic acid recovery rates of both were evaluated from the results of qPCR. The recovery rate (%) of each round of panning was calculated by the formula below. The results are shown in the table below.

Recovery rate of library (%) = output nucleic acid amount (number of molecules) of library/(input nucleic acid amount (number of molecules) of library) × 100

[Table 21]

| Round | Target protein | | Input nucleic acid amount (molecules) of library | In presence of target protein | | In absence of target protein | |
|---|---|---|---|---|---|---|---|
| | Fixed on beads | Free | | Output nucleic acid amount (molecules) | Recovery rate (%) | Output nucleic acid amount (molecules) | Recovery rate (%) |
| 1 | GST | KRAS | 1.80E+12 | 3.03E+05 | 1.68E-05 | - | - |
| 2 | KRAS | GST | 1.77E+12 | 2.94E+06 | 1.66E-04 | 3.13E+06 | 1.77E-04 |
| 3 | Application of splitGFP | | 3.45E+11 | 4.97E+07 | 1.44E-02 | 1.74E+06 | 5.03E-04 |
| 4 | Application of splitGFP | | 7.28E+11 | 3.06E+08 | 4.21E-02 | 9.76E+06 | 1.34E-03 |
| 5 | Application of splitGFP | | 8.25E+11 | 1.02E+09 | 1.24E-01 | 9.00E+06 | 1.09E-03 |

Panning was performed according to the method of Example 6-4-1 except that, in round 1, KRAS-FLAGSorHisTev-Bio was used instead of GST-HisTev-Bio, and GST-HisTevAvi was used instead of HAT-KRAS-nonbio; and in round 2, GST-HisTev-Bio was used instead of KRAS-FLAGSorHisTev-Bio, and HAT-KRAS-Nonbio was used instead of GST-HisTevAvi. Rounds 3 to 5 were performed according to the method of Example 6-4-1. The recovery rate (%) of each round of panning is shown in the table below.

[Table 22]

| Round | Target protein | | Input nucleic acid amount (molecules) of library | In presence of target protein | | In absence of target protein | |
|---|---|---|---|---|---|---|---|
| | Fixed on beads | Free | | Output nucleic acid amount (molecules) | Recovery rate (%) | Output nucleic acid amount (molecules) | Recovery rate (%) |
| 1 | KRAS | GST | 2.49E+12 | 2.30E+04 | 9.24E-07 | - | - |
| 2 | GST | KRAS | 1.84E+12 | 5.71E+06 | 3.10E-04 | 8.32E+06 | 4.51E-04 |
| 3 | Application of splitGFP | | 3.67E+11 | 4.19E+07 | 1.14E-02 | 1.61E+06 | 4.39E-04 |
| 4 | Application of splitGFP | | 9.23E+11 | 3.96E+08 | 4.29E-02 | 9.31E+06 | 1.01E-03 |
| 5 | Application of splitGFP | | 8.13E+11 | 5.65E+08 | 6.94E-02 | 8.87E+06 | 1.09E-03 |

As a result, in the second half of the rounds, as shown in Tables 21 and 22, there is a difference in the recovery rate between the selections in the presence of the target protein and in the absence of the target protein. It is thus likely that molecules that are only recovered in the presence of the target protein are present in the library of each round, and they are enriched each time they repeat the round.

Example 6-4-2: Panning using FKBP and KRAS as target proteins

Rounds 1 and 2 were performed according to the method of Example 6-4-1, except that FKBP-HisTev-Bio was used instead of GST-HisTev-Bio and FKBP-HisTevAvi was used instead of GST-HisTevAvi.
Rounds 3 to 5 were performed according to the method of Example 6-4-1 except that Hisx6-FKBP12-Link12-GFP11 was used instead of KRAS-sp11, and KRAS-sp10 was used instead of GST-sp10.
The recovery rate (%) of each round of panning is shown in the table below.

[Table 23]

| Round | Target protein | | Input nucleic acid amount (molecules) of library | In presence of target protein | | In absence of target protein | |
|---|---|---|---|---|---|---|---|
| | Fixed on beads | Free | | Output nucleic acid amount (molecules) | Recovery rate (%) | Output nucleic acid amount (molecules) | Recovery rate (%) |
| 1 | FKBP | KRAS | 1.40E+12 | 7.24E+04 | 5.19E-06 | - | - |
| 2 | KRAS | FKBP | 1.34E+12 | 2.34E+06 | 1.75E-04 | 1.73E+06 | 1.29E-04 |
| 3 | Application of splitGFP | | 3.50E+11 | 3.71E+07 | 1.06E-02 | 3.94E+06 | 1.13E-03 |
| 4 | Application of splitGFP | | 7.89E+11 | 3.16E+08 | 4.01E-02 | 7.34E+06 | 9.31E-04 |
| 5 | Application of splitGFP | | 7.41E+11 | 1.60E+09 | 2.16E-01 | 5.57E+06 | 7.51E-04 |

Panning was performed according to the method of Example 6-4-1 except that, in round 1, KRAS-HisTev-Bio was used instead of FKBP-HisTev-Bio, and FKBP-HisTevAvi was used instead of KRAS-FLAG-Sor-His-Tev-Bio; and in round 2, FKBP-HisTev-Bio was used instead of KRAS-FLAGSorHisTev-Bio, and HAT-KRAS-nonbio was used instead of FKBP-HisTevAvi.

Rounds 1 and 2 were performed according to the method of Example 6-4-2, except that KRAS-HisTev-Bio was used instead of FKBP-HisTev-Bio, and FKBP-HisTevAvi was used instead of KRAS-FLAG-Sor-His-Tev-Bio. Rounds 3 to 5 were performed according to the method of Example 6-4-2. The recovery rate (%) of each round of panning is shown in the table below.

[Table 24]

| Round | Target protein | | Input nucleic acid amount (molecules) of library | In presence of target protein | | In absence of target protein | |
|---|---|---|---|---|---|---|---|
| | Fixed on beads | Free | | Output nucleic acid amount (molecules) | Recovery rate (%) | Output nucleic acid amount (molecules) | Recovery rate (%) |
| 1 | KRAS | FKBP | 2.52E+12 | 2.48E+06 | 9.86E-05 | - | - |
| 2 | FKBP | KRAS | 5.29E+11 | 9.26E+05 | 1.75E-04 | 6.90E+05 | 1.31E-04 |
| 3 | Application of splitGFP | | 8.37E+11 | 2.73E+08 | 3.26E-02 | 8.27E+06 | 9.88E-04 |
| 4 | Application of splitGFP | | 9.60E+11 | 5.98E+08 | 6.23E-02 | 7.21E+06 | 7.51E-04 |
| 5 | Application of splitGFP | | 1.00E+12 | 1.08E+09 | 1.08E-01 | 3.26E+07 | 3.26E-03 |

As a result, in the second half of the rounds, as shown in Tables 23 and 24, there is a difference in the recovery rate between the selections in the presence of the target protein and in the absence of the target protein. It is thus likely that molecules that are only recovered in the presence of the target protein are present in the library of each round, and they are enriched each time they repeat the round.

Industrial Applicability

The screening method according to the present disclosure enables a high throughput selection of molecules capable of forming a complex with a plurality of target molecules from a library containing a variety of molecules. According to the screening method of the present disclosure, a candidate molecule of interest can be efficiently obtained while excluding a test molecule that forms a complex with only one target molecule. The screening method according to the

present disclosure can also be used to obtain a candidate molecule capable of producing a variety of binding modes with a plurality of target molecules. The screening method according to the present disclosure is useful in discovering new candidate molecules for a medicament.

## Claims

1. A method for screening for a candidate molecule capable of forming a complex with a first target molecule and a second target molecule, the method comprising steps (1) and (2) below:

   (1) mixing the first target molecule linked to a first moiety, the second target molecule linked to a second moiety, and a library containing a plurality of test molecules, wherein the first moiety and the second moiety constitute a part or all of a protein; and
   (2) recovering, after step (1), a complex containing the first target molecule linked to the first moiety, the second target molecule linked to the second moiety, and the candidate molecule by an affinity-based method using a third molecule capable of detecting that the first moiety and the second moiety are in proximity or association.

2. The method according to claim 1, further comprising, after step (2), a step of identifying the candidate molecule contained in the recovered complex (step (3)).

3. The method according to claim 1 or 2, wherein the third molecule is a molecule that reacts with the first moiety and/or the second moiety when the first moiety and the second moiety are in proximity or association.

4. The method according to any one of claims 1 to 3, wherein the third molecule is a molecule that does not substantially react with the first moiety and/or the second moiety when the first moiety and the second moiety are neither in proximity nor in association.

5. The method according to any one of claims 2 to 4, further comprising, between steps (2) and (3), a step of eluting the candidate molecule from a complex containing the first target molecule linked to the first moiety, the second target molecule linked to the second moiety, and the candidate molecule (step 2A).

6. The method according to claim 5, wherein step (3) comprises determining a sequence of a polynucleotide encoding the candidate molecule eluted in step (2A).

7. The method according to any one of claims 1 to 6, comprising a step of repeating steps (1) and (2) a plurality of times.

8. The method according to any one of claims 1 to 7, wherein the method is performed in vitro.

9. The method according to any one of claims 1 to 8, wherein the library is a display library or a DNA encoding library.

10. The method according to any one of claims 1 to 9, wherein the library is a library having a diversity of $1 \times 10^4$ or more.

11. The method according to any one of claims 1 to 10, wherein the protein is GFP, luciferase, ubiquitin, or a SNAP tag.

12. The method according to any one of claims 1 to 11, wherein the affinity-based method is a pull-down method.

13. The method according to any one of claims 5 to 12, wherein the eluting in step (2A) is performed with an enzyme or by heat.

14. The method according to any one of claims 5 to 13, wherein the eluting in step (2A) is performed by cleaving a first linker located between the first moiety of the protein and the first target molecule and a second linker located between the second moiety of the protein and the second target molecule.

15. The method according to any one of claims 1 to 14, wherein the protein providing the first moiety and the second moiety contains an amino acid sequence selected from the group consisting of (a) to (c) below;

   (a) an amino acid sequence represented by SEQ ID NO: 71, 75, 78, or 82;
   (b) the amino acid sequence represented by SEQ ID NO: 71, 75, 78, or 82 in which one or more amino acids

are deleted, inserted, substituted, and/or added; and
(c) an amino acid sequence having 80% or more sequence identity to the amino acid sequence represented by SEQ ID NO: 71, 75, 78, or 82.

*Fig.1*

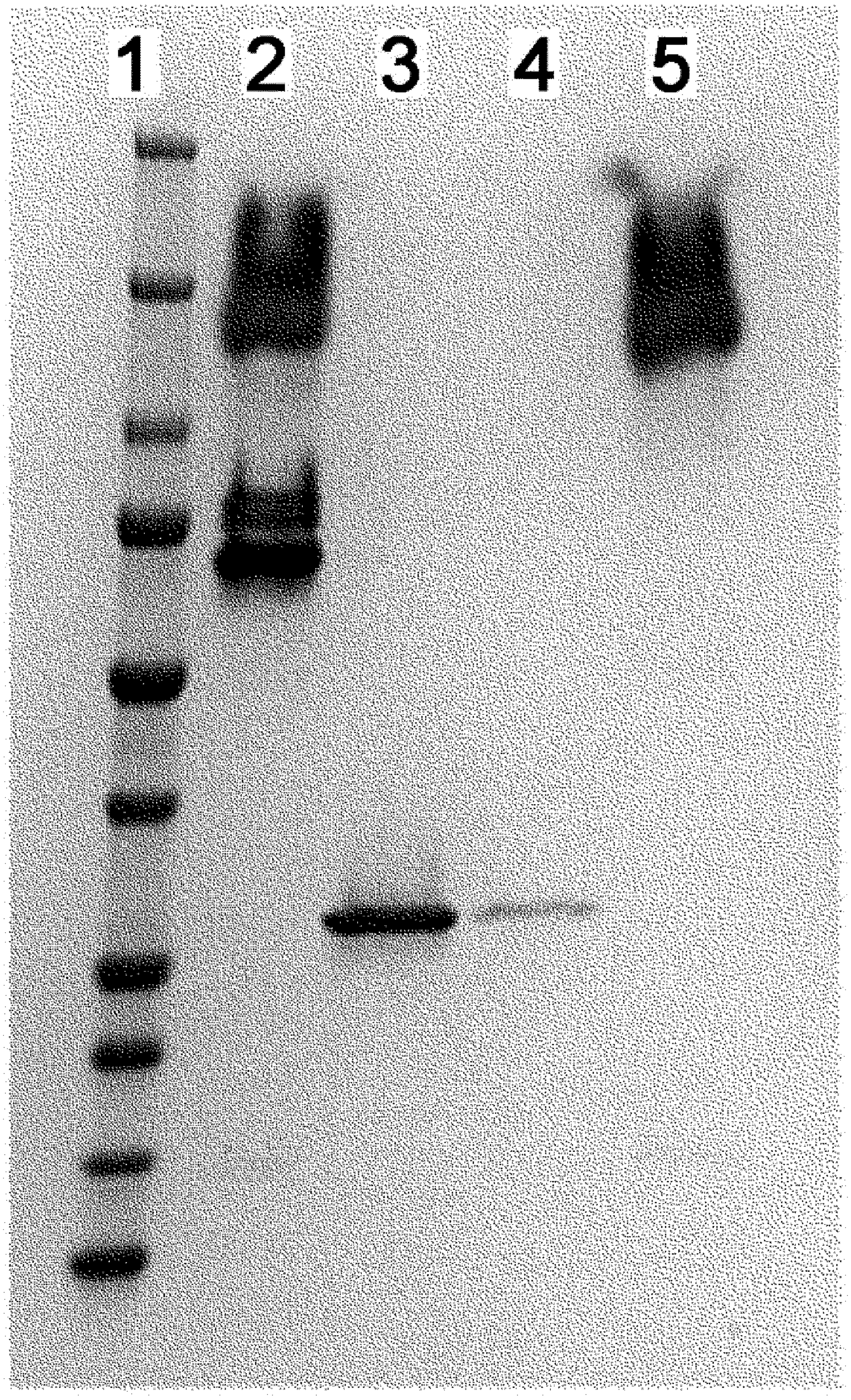

Fig.2

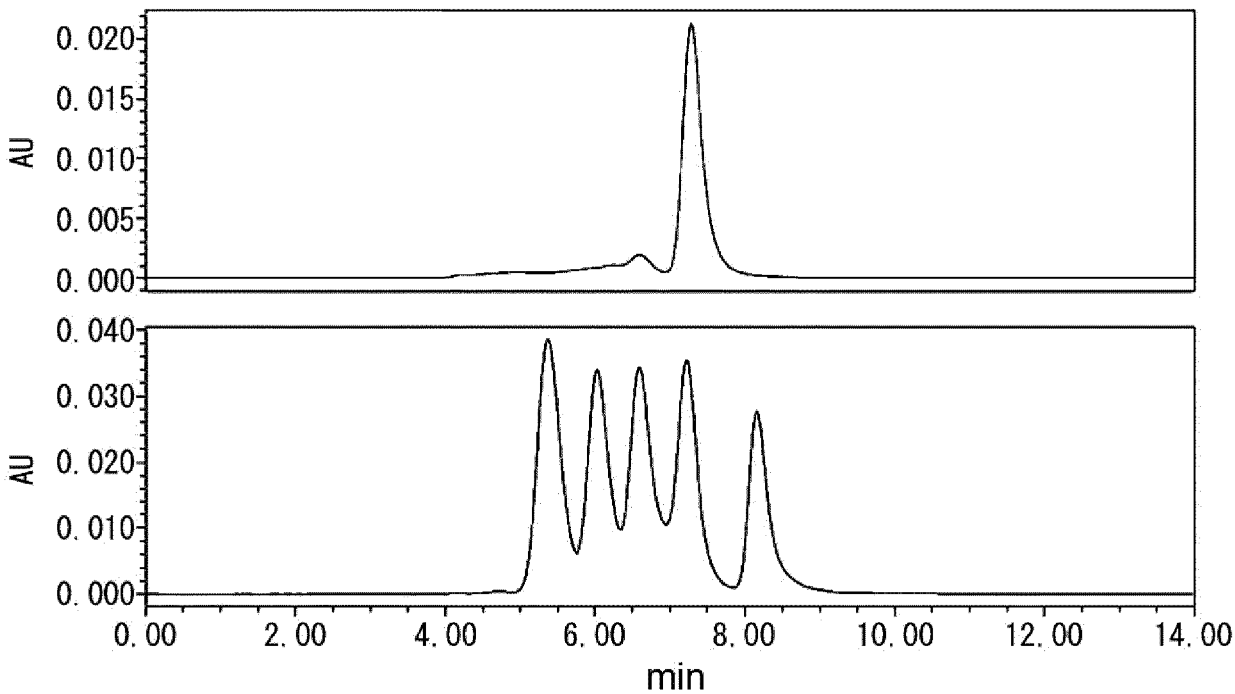
0.020
0.015
0.010
0.005
0.000
AU
0.040
0.030
0.020
0.010
0.000
AU
0.00
2.00
4.00
6.00
8.00
10.00
12.00
14.00
min

*Fig.3*

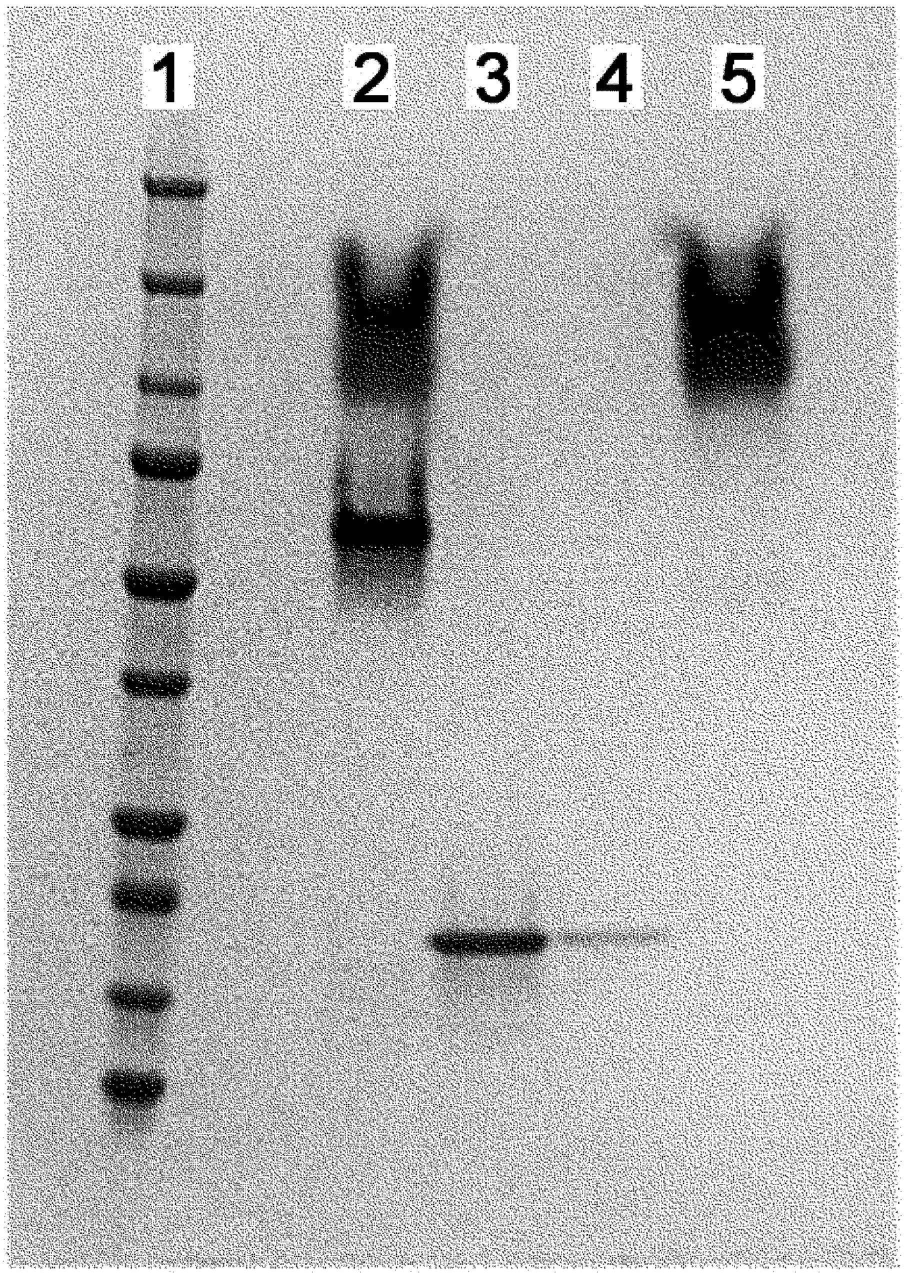

*Fig.4*

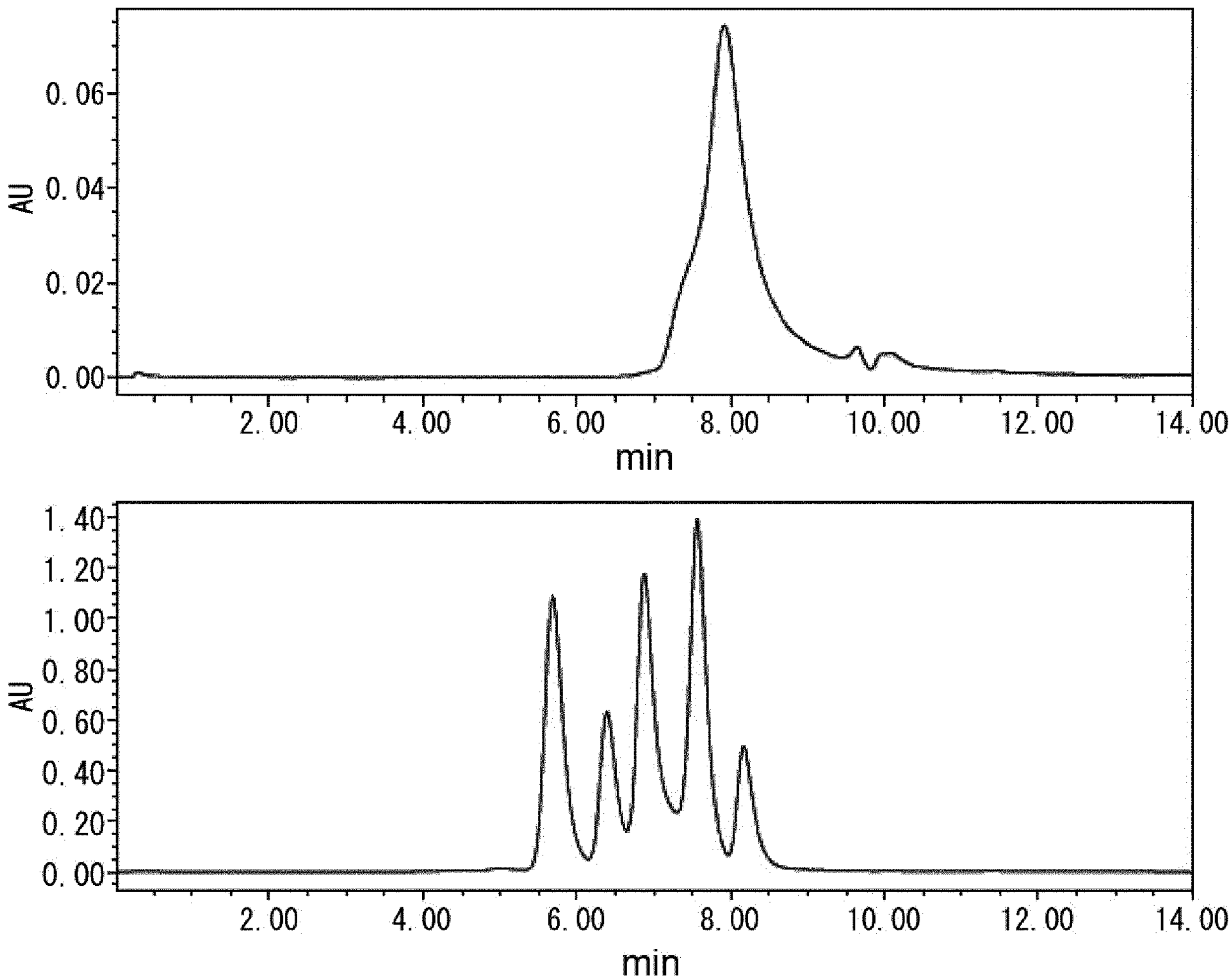
0. 06
0. 04
0. 02
0. 00
AU
2. 00
4. 00
6. 00
8. 00
10. 00
12. 00
14. 00
min
1. 40
1. 20
1. 00
0. 80
0. 60
0. 40
0. 20
0. 00
AU
2. 00
4. 00
6. 00
8. 00
10. 00
12. 00
14. 00
min

## INTERNATIONAL SEARCH REPORT

International application No.
**PCT/JP2021/048097**

**A. CLASSIFICATION OF SUBJECT MATTER**

***G01N 33/537***(2006.01)i; ***C12N 15/12***(2006.01)i; ***C12N 15/53***(2006.01)i; ***C12Q 1/6869***(2018.01)i; ***G01N 33/15***(2006.01)i; ***G01N 33/50***(2006.01)i; ***G01N 33/68***(2006.01)i
FI: G01N33/537; G01N33/50 Z; G01N33/15 Z; C12Q1/6869 Z; C12N15/53; C12N15/12; G01N33/68 ZNA

According to International Patent Classification (IPC) or to both national classification and IPC

**B. FIELDS SEARCHED**

Minimum documentation searched (classification system followed by classification symbols)

G01N33/537; C12N15/12; C12N15/53; C12Q1/6869; G01N33/15; G01N33/50; G01N33/68

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| A | US 2015/0099271 A1 (LOS ALAMOS NATIONAL SECURITY, LLC) 09 April 2015 (2015-04-09)<br>entire text, all drawings | 1-15 |
| A | JP 2008-521447 A (THE REGENTS OF THE UNIVERSITY OF CALIFORNIA LOS ALAMOS NATIONAL LABORATORY) 26 June 2008 (2008-06-26)<br>entire text, all drawings | 1-15 |
| A | CN 110283244 A (CHONGQING MEDICAL UNIVERSITY) 27 September 2019 (2019-09-27)<br>entire text, all drawings | 1-15 |
| A | MIE, M. et al. Tracking a protein following dissociation from a protein-protein complex using a split SNAP-tag system. ANALYTICAL BIOCHEMISTRY. 25 February 2015, vol. 477, pp. 53-55<br>entire text, all drawings | 1-15 |
| A | US 2004/0053388 A1 (ECKERT, Jorg H.) 18 March 2004 (2004-03-18)<br>entire text, all drawings | 1-15 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 February 2022** | **08 March 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

INTERNATIONAL SEARCH REPORT

International application No.
PCT/JP2021/048097

C. DOCUMENTS CONSIDERED TO BE RELEVANT

| Category* | Citation of document, with indication, where appropriate, of the relevant passages | Relevant to claim No. |
|---|---|---|
| A | HORING, C. et al. A Dynamic, Split-Luciferase-Based Mini-G Protein Sensor to Functionally Characterize Ligands at All Four Histamine Receptor Subtypes. INTERNATIONAL JOURNAL OF MOLECULAR SCIENCES. vol. 21/no. 22, 8440, pp. 1-18<br>entire text, all drawings | 1-15 |

Form PCT/ISA/210 (second sheet) (January 2015)

**INTERNATIONAL SEARCH REPORT**

International application No.
**PCT/JP2021/048097**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
**Information on patent family members**

International application No.
**PCT/JP2021/048097**

| Patent document cited in search report | Publication date (day/month/year) | Patent family member(s) | Publication date (day/month/year) |
|---|---|---|---|
| US 2015/0099271 A1 | 09 April 2015 | (Family: none) | |
| JP 2008-521447 A | 26 June 2008 | WO 2006/062882 A2<br>entire text, all drawings<br>US 2006/0257887 A1<br>EP 1828408 A2 | |
| CN 110283244 A | 27 September 2019 | (Family: none) | |
| US 2004/0053388 A1 | 18 March 2004 | WO 2002/066656 A2<br>EP 1349943 A2 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 9428845 B
- US 20180164324 A
- WO 2013100132 A
- US 20100281552 A
- US 20120174242 A
- US 20180172692 A
- WO 2018225864 A
- WO 2017150732 A
- WO 2016148044 A
- WO 2020138336 A1
- WO 2018143145 A
- WO 2020138336 A


### Non-patent literature cited in the description


- **ULRICH, H. W et al.** *CANCER GENOMICS & PROTEOMICS,* 2013, vol. 10, 1-18
- **CABANTOUS, S et al.** *Sci Rep,* 2013, vol. 3, 2854
- **KORAICHI, F. et al.** *Journal of Cell Science,* 2018, vol. 131
- *Protein Engineering,* 1996, vol. 9 (3), 299-305
- *Molecules,* April 2019, vol. 24 (8), 1629
- *Proc Natl Acad Sci USA.,* 1997, vol. 94, 12297-302
- *FEBS Lett.,* 1997, vol. 414, 405-8
- *Nucleic Acids Res,* 2009, vol. 37 (16), e108
- **YAMAGUCHI J ; NAIMUDDIN M ; BIYANI M ; SASAKI T ; MACHIDA M ; KUBO T ; FUNATSU T ; HUSIMI Y ; NEMOTO N.** a ribosome display utilizing a relatively stable complex of ribosome and a translation product during mRNA translation. *Proc Natl Acad Sci USA.,* 1994, vol. 91, 9022-6
- *Nucleic AcidsRes,* 2005, vol. 33, e10
- **REIERSEN H ; LOBERSLI I ; LOSET GA ; HVATTUM E ; SIMONSEN B ; STACY JE ; MCGREGOR D ; FITZGERALD K ; WELSCHOF M ; BREKKE OH.** and a CIS display utilizing the replication initiation protein RepA of the plasmid of the microorganism to bind to the replication initiation point ori. *Proc Natl Acad Sci USA.,* 2004, vol. 101, 2806-10
- *Nat Biotechnol.,* 1998, vol. 16, 652-6
- **WEN-XUE J et al.** *Sci Rep,* 2016, vol. 6, 20568
- *Analyst,* 2012, vol. 137, 4760-4765
- *Anal. Chem.,* 2016, vol. 88, 8166-8171
- *PNAS,* 03 July 2007, vol. 104 (27), 11209-11214
- **ANDREW, D et al.** *Sci Rep.,* 2017, vol. 7, 8186
- *Anal. Chem,* 2018, vol. 90, 3001-3004
- **KUNKEL et al.** *Proc. Natl. Acad. Sci. USA,* 1985, vol. 82, 488-492
- *Proc. Natl. Acad. Sci. USA,* 1990, vol. 87, 2264-2268
- *Proc Natl Acad Sci USA,* 1993, vol. 90, 5873
- **ALTSCHUL SF et al.** *J Mol Biol,* 1990, vol. 215, 403
- **KRAMER, W ; FRITZ, H. -J.** Oligonucleotide-directed construction of mutagenesis via gapped duplex DNA. *Methods in Enzymology,* 1987, vol. 154, 350-367
- **SOUTHERN, E.M.** *Journal of Molecular Biology,* 1975, vol. 98, 503
- **MANDEL, M ; HIGA, A.** *Journal of Molecular Biology,* 1970, vol. 53, 158-162
- **HANAHAN, D.** *Journal of Molecular Biology,* 1983, vol. 166, 557-580
- **WARD et al.** *Nature,* 1989, vol. 341, 544-546
- *FASEB J,* 1992, vol. 6, 2422-2427
- **BETTER et al.** *Science,* 1988, vol. 240, 1041-1043
- **LEI, S. P et al.** *J. Bacteriol.,* 1987, vol. 169, 4379
- **MULLIGAN et al.** *Nature,* 1979, vol. 277, 108
- **MIZUSHIMA et al.** *Nucleic Acids Res,* 1990, vol. 18, 5322
- **VALLE et al.** *Nature,* 1981, vol. 291, 358-340
- *Proc. Natl. Acad. Sci. USA,* 1980, vol. 77, 4216-4220
- *Proc. Natl. Acad. Sci. USA,* 1968, vol. 60, 1275
- *J Am Chem Soc.,* 2005, vol. 127, 4715-21
- *Proc. Natl. Acad. Sci. USA.,* 12 July 2011, vol. 108 (28), 11399-11404